(19) Europäisches Patentamt
European Patent Office
Office européen des brevets

(11) EP 1 558 635 B1

(12) # EUROPEAN PATENT SPECIFICATION

(45) Date of publication and mention of the grant of the patent:
**15.07.2009 Bulletin 2009/29**

(21) Application number: **03778306.5**

(22) Date of filing: **03.11.2003**

(51) Int Cl.:
*C07K 14/16* (2006.01) *A61K 39/21* (2006.01)
*C12N 15/48* (2006.01) *A61K 48/00* (2006.01)
*C12N 15/62* (2006.01)

(86) International application number:
**PCT/EP2003/012429**

(87) International publication number:
**WO 2004/041851 (21.05.2004 Gazette 2004/21)**

(54) **VACCINE**

IMPFSTOFF

VACCIN

(84) Designated Contracting States:
**AT BE BG CH CY CZ DE DK EE ES FI FR GB GR HU IE IT LI LU MC NL PT RO SE SI SK TR**
Designated Extension States:
**LT LV**

(30) Priority: **05.11.2002 GB 0225786**

(43) Date of publication of application:
**03.08.2005 Bulletin 2005/31**

(73) Proprietor: **GLAXO GROUP LIMITED**
**Greenford,**
**Middlesex UB6 0NN (GB)**

(72) Inventor: **ERTL, Peter, Franz**
**Stevenage, Hertfordshire SG1 2NY (GB)**

(74) Representative: **Privett, Kathryn Louise et al**
**GlaxoSmithKline,**
**Corporate Intellectual Property (CN9.25.1),**
**980 Great West Road**
**Brentford, Middlesex TW8 9GS (GB)**

(56) References cited:

| | |
|---|---|
| **EP-A- 0 577 894** | **WO-A-01/27291** |
| **WO-A-02/32943** | **WO-A-02/36792** |
| **WO-A-98/41536** | **WO-A-20/04041852** |
| **CA-A- 2 430 702** | |

- **WOODBERRY T ET AL: "Immunogenicity of a human immunodeficiency virus (HIV) polytope vacine containing multiple HLA A2 HIV CD8+ cytotoxic T-cell epitopes" JOURNAL OF VIROLOGY, THE AMERICAN SOCIETY FOR MICROBIOLOGY, US, vol. 73, no. 7, July 1999 (1999-07), pages 5320-5325, XP002162348 ISSN: 0022-538X**
- **LIU W J ET AL: "Papillomavirus Virus-like Particles for the Delivery of Multiple Cytotoxic T Cell Epitopes" VIROLOGY, ACADEMIC PRESS, ORLANDO, US, vol. 273, no. 2, 1 August 2000 (2000-08-01), pages 374-382, XP004436241 ISSN: 0042-6822**
- **BENKO D M ET AL: "A NOVEL HUMAN IMMUNODEFICIENCY VIRUS TYPE 1 PROTEIN TEV SHARES SEQUENCES WITH TAT ENV AND REV PROTEINS" JOURNAL OF VIROLOGY, vol. 64, no. 6, 1990, pages 2505-2518, XP002285841 ISSN: 0022-538X**
- **SALFELD J ET AL: "A TRIPARTITE HIV-1 TAT-ENV-REV FUSION PROTEIN" EMBO JOURNAL, OXFORD UNIVERSITY PRESS, SURREY, GB, vol. 9, no. 3, 1 March 1990 (1990-03-01), pages 965-970, XP000113784 ISSN: 0261-4189**
- **DOE B ET AL: "INDUCTION OF HIV-1 ENVELOPE (GP120)-SPECIFIC CYTOTOXIC T LYMPHOCYTE RESPONSES IN MICE BY RECOMBINANT CHO CELL-DERIVED GP120 IS ENHANCED BY ENZYMATIC REMOVEL OF N-LINKED GLYCANS" EUROPEAN JOURNAL OF IMMUNOLOGY, WEINHEIM, DE, vol. 24, no. 10, 1994, pages 2369-2376, XP000672290 ISSN: 0014-2980**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

(Cont. next page)

- BOTARELLI P ET AL: "N-GLYCOSYLATION OF HIV-GP120 MAY CONSTRAIN RECOGNITION BY T LYMPHOCYTES" JOURNAL OF IMMUNOLOGY, THE WILLIAMS AND WILKINS CO. BALTIMORE, US, vol. 147, no. 9, 1 November 1991 (1991-11-01), pages 3128-3132, XP000673664 ISSN: 0022-1767
- STEFANIE A ET AL: "Increased Immune Response Elicited by DNA Vaccination with a Synthetic gp120 Sequence with Optimized Codon Usage" JOURNAL OF VIROLOGY, THE AMERICAN SOCIETY FOR MICROBIOLOGY, US, vol. 72, no. 2, February 1998 (1998-02), pages 1497-1503, XP002278034 ISSN: 0022-538X
- KOTSOPOULOU E ET AL: "A Rev-independent human immunodeficiency virus type 1 (HIV-1)-based vector that exploits a codon-optimized HIV-1 gag-pol gene" JOURNAL OF VIROLOGY, THE AMERICAN SOCIETY FOR MICROBIOLOGY, US, vol. 74, no. 10, May 2000 (2000-05), pages 4839-4852, XP002140792 ISSN: 0022-538X
- IGLESIAS, ENRIQUE ET AL: "Chimeric proteins containing HIV-1 T cell epitopes: Expression in E. coli, purification and induction of antibodies in mice" JOURNAL OF BIOCHEMISTRY, MOLECULAR BIOLOGY AND BIOPHYSICS , 5(2), 109-122 CODEN: JBMBF6; ISSN: 1025-8140, 2001, XP009035847

## Description

### Field of the Invention

This invention relates to nucleic acid constructs, vectors comprising such constructs, methods of preparing the vectors and constructs and their use in prophylaxis or therapy, in particular therapeutic vaccines. The invention further relates to host cells comprising the constructs and vectors and to fusion proteins encoded by the constructs as well as to the fusion proteins per se. The invention further relates to pharmaceutical formulations comprising the constructs and vectors and to the use of the constructs and vectors in medicine. The invention relates in particular to DNA vaccines that are useful in the prophylaxis and treatment of HIV infections, more particularly when administered by particle mediated delivery.

### Background to the Invention

HIV-1 is the primary cause of the acquired immune deficiency syndrome (AIDS) which is regarded as one of the world's major health problems. Although extensive research throughout the world has been conducted to produce a vaccine, such efforts thus far have not been successful.

The HIV envelope glycoprotein gp120 is the viral protein that is used for attachment to the host cell. This attachment is mediated by binding to two surface molecules of helper T cells and macrophages, known as CD4 and one of the two chemokine receptors CCR-4 or CXCR-5. The gp120 protein is first expressed as a larger precursor molecule (gp160), which is then cleaved post-translationally to yield gp120 and gp41. The gp 120 protein is retained on the surface of the virion by linkage to the gp41 molecule, which is inserted into the viral membrane.

The gp120 protein is the principal target of neutralizing antibodies, but unfortunately the most immunogenic regions of the proteins (V3 loop) are also the most variable parts of the protein. Therefore, the use of gp 120 (or its precursor gp 160) as a vaccine antigen to elicit neutralizing antibodies is thought to be of limited use for a broadly protective vaccine. The gp120 protein does also contain epitopes that are recognized by cytotoxic T lymphocytes (CTL). These effector cells are able to eliminate virus-infected cells, and therefore constitute a second major antiviral immune mechanism. In contrast to the target regions of neutralizing antibodies some CTL epitopes appear to be relatively conserved among different HIV strains. For this reason gp120 and gp160 may be considered to be useful antigenic components in vaccines that aim at eliciting cell-mediated immune responses (particularly CTL).

Non-envelope proteins of HIV-1 have been described and include for example internal structural proteins such as the products of the gag and pol genes and other non-structural proteins such as Rev, Nef, Vif and Tat (Green et al., New England J. Med, 324, 5, 308 et seq (1991) and Bryant et al. (Ed. Pizzo), Pediatr. Infect. Dis. J., 11, 5, 390 et seq (1992).

HIV Tat and Nef proteins are early proteins, that is they are expressed early in infection and in the absence of structural protein.

The Nef protein is known to cause the removal of CD4, the HIV receptor, from the cell surface, but the biological importance of this function is debated. Additionally Nef interacts with the signal pathway of T cells and induces an active state, which in turn may promote more efficient gene expression. Some HIV isolates have mutations in this region, which cause them not to encode functional protein and are severely compromised in their replication and pathogenesis in vivo.

The Tat gene gives rise to a number of differentially spliced transcripts at different times during infection. The first exon encodes an 86 amino acid protein which dominates early in infection. The second exon encodes an additional 14 amino acids, and this partially spliced form of Tat is found late in infection. Both forms are fully functional transactivators, but the longer form also contains an RGD motif important for binding to $\alpha_v\beta_3$ and $\alpha_5\beta_1$ integrins. Tat binds to a short-stem loop structure, known as the transactivation response element (TAR), that is located at the 5' terminus of HIV RNAs, and up-regulates transcription from the HIV LTR at least 1000-fold. Tat has a role in promoting the elongation phase of HIV infection and stimulates the production of full-length viral transcripts. Tat can affect the expression of a number of cellular genes and can activate the expression of a number of cellular genes including TNF, IL-2 and IL-6, and regulates expression of p53 and Bcl-2. Tat is produced in excess and is secreted from infected cells. This extra-cellular Tat can enter other cells and may prime cells for infection by HIV or accelerate the rate of HIV replication in newly infected cells.

In a conference presentation (C. David Pauza, Immunization with Tat toxoid attenuates SHIV89.6PD infection in rhesus macaques, 12th Cent Gardes meeting, Marnes-La-Coquette, 26.10.1999), experiments were described in which rhesus macaques were immunised with Tat toxoid alone or in combination with an envelope glycoprotein gp160 vaccine combination (one dose recombinant vaccinia virus and one dose recombinant protein). The results observed showed that the presence of the envelope glycoprotein gave no advantage over experiments performed with Tat alone.

The Gag gene is translated from the full-length RNA to yield a precursor polyprotein which is subsequently cleaved into 3-5 capsid proteins; the matrix protein, capsid protein and nucleic acid binding protein and protease. (1. Fundamental Virology, Fields BN, Knipe DM and Howley M 1996 2. Fields Virology vol 2 1996).

The gag gene gives rise to the 55-kilodalton (kD) Gag precursor protein, also called p55, which is expressed from the unspliced viral mRNA. During translation, the N terminus of p55 is myristoylated, triggering its association with the cytoplasmic aspect of cell membranes. The membrane-associated Gag polyprotein recruits two copies of the viral genomic RNA along with other viral and cellular proteins that triggers the budding of the viral particle from the surface of an infected cell. After budding, p55 is cleaved by the virally encoded protease (a product of the pol gene) during the process of viral maturation into four smaller proteins designated MA (matrix [p17]), CA (capsid [p24]), NC (nucleocapsid [p9]), and p6.(4).

In addition to the 3 major Gag proteins, all Gag precursors contain several other regions, which are cleaved out and remain in the virion as peptides of various sizes. These proteins have different roles e.g. the p2 protein has a proposed role in regulating activity of the protease and contributes to the correct timing of proteolytic processing.

The MA polypeptide is derived from the N-terminal, myristoylated end of p55. Most MA molecules remain attached to the inner surface of the virion lipid bilayer, stabilizing the particle. A subset of MA is recruited inside the deeper layers of the virion where it becomes part of the complex which escorts the viral DNA to the nucleus. These MA molecules facilitate the nuclear transport of the viral genome because a karyophilic signal on MA is recognized by the cellular nuclear import machinery. This phenomenon allows HIV to infect non-dividing cells, an unusual property for a retrovirus.

The p24 (CA) protein forms the conical core of viral particles. Cyclophilin A has been demonstrated to interact with the p24 region of p55 leading to its incorporation into HIV particles. The interaction between Gag and cyclophilin A is essential because the disruption of this interaction by cyclosporin A inhibits viral replication.

The NC region of Gag is responsible for specifically recognizing the so-called packaging signal of HIV. The packaging signal consists of four stem loop structures located near the 5' end of the viral RNA, and is sufficient to mediate the incorporation of a heterologous RNA into HIV-1 virions. NC binds to the packaging signal through interactions mediated by two zinc-finger motifs. NC also facilitates reverse transcription.

The p6 polypeptide region mediates interactions between p55 Gag and the accessory protein Vpr, leading to the incorporation of Vpr into assembling virions. The p6 region also contains a so-called late domain which is required for the efficient release of budding virions from an infected cell.

The Pol gene encodes two proteins containing the two activities needed by the virus in early infection, the RT and the integrase protein needed for integration of viral DNA into cell DNA. The primary product of Pol is cleaved by the virion protease to yield the amino terminal RT peptide which contains activities necessary for DNA synthesis (RNA and DNA directed DNA polymerase, ribouclease H) and carboxy terminal integrase protein. HIV RT is a heterodimer of full-length RT (p66) and a cleavage product (p51) lacking the carboxy terminal Rnase integrase domain.

RT is one of the most highly conserved proteins encoded by the retroviral genome. Two major activities of RT are the DNA Pol and Ribonuclease H. The DNA Pol activity of RT uses RNA and DNA as templates interchangeably and like all DNA polymerases known is unable to initiate DNA synthesis de novo, but requires a pre existing molecule to serve as a primer (RNA).

The Rnase H activity inherent in all RT proteins plays the essential role early in replication of removing the RNA genome as DNA synthesis proceeds. It selectively degrades the RNA from all RNA - DNA hybrid molecules. Structurally the polymerase and ribo H occupy separate, non-overlapping domains with the Pol covering the amino two thirds of the Pol.

The p66 catalytic subunit is folded into 5 distinct subdomains. The amino terminal 23 of these have the portion with RT activity. Carboxy terminal to these is the Rnase H Domain.

After infection of the host cell, the retroviral RNA genome is copied into linear ds DNA by the reverse transcriptase that is present in the infecting particle. The integrase (reviewed in Skalka AM '99 Adv in Virus Res 52 271-273) recognises the ends of the viral DNA, trims them and accompanies the viral DNA to a host chromosomal site to catalyse integration. Many sites in the host DNA can be targets for integration. Although the integrase is sufficient to catalyse integration in vitro, it is not the only protein associated with the viral DNA in vivo - the large protein - viral DNA complex isolated from the infected cells has been denoted the pre integration complex. This facilitates the acquisition of the host cell genes by progeny viral genomes.

The integrase is made up of 3 distinct domains, the N terminal domain, the catalytic core and the C terminal domain. The catalytic core domain contains all of the requirements for the chemistry of polynucleotidyl transfer.

DNA vaccines usually consist of a bacterial plasmid vector into which is inserted a strong promoter, the gene of interest which encodes an antigenic peptide and a polyadenylation/transcriptional termination sequence. The gene of interest may encode a full protein or simply an antigenic peptide sequence relating to the pathogen, tumour or other agent which it is intended to protect against. The plasmid can be grown in bacteria, such as for example *E. coli* and then isolated and prepared in an appropriate medium, depending upon the intended route of administration, before being administered to the host. Following administration the plasmid is taken up by cells of the host, or delivered directly into the host cells, where the encoded peptide is produced. The plasmid vector will preferably be made without an origin of replication functional in eukaryotic cells, in order to prevent plasmid replication in the mammalian host and integration within chromosomal DNA of the animal concerned.

There are a number of advantages of DNA vaccination relative to traditional vaccination techniques. First, it is predicted that because the proteins that are encoded by the DNA sequence are synthesised in the host, the structure or conformation of the protein will be similar to the native protein associated with the disease state. It is also likely that DNA vaccination will offer protection against different strains of a virus, by generating a cytotoxic T lymphocyte response that recognises epitopes from conserved proteins. The technology also offers the possibility of combining diverse immunogens into a single preparation to facilitate simultaneous immunisation in relation to a number of disease states.

Helpful background information in relation to DNA vaccination is provided in Donnelly et al "DNA vaccines" Ann. Rev Immunol. 1997 15: 617-648.

Doe et al (1994) Eur J immunol, 24: 2369-2376 investigated how variations in glycosylation affected the CD8+ CTL response to gp120 and found that gp120 produced in mammalian CHO cells had a reduced ability to prime CTL responses when compared with insect or yeast cell-derived envelope proteins unless N-linked oligosaccharides were removed prior to immunization.

It has now been discovered that there are benefits to be gained by employing a polynucleotide encoding a non-glycosylated HIV envelope protein in a vaccine for HIV. Surprisingly, a DNA vector expressing gp120 without a secretion signal and which is thus not glycosylated or secreted from the cell is a more effective stimulator of CTL responses than a DNA vector expressing gp120 with its native secretion signal. Since the secretion signal is responsible for directing the gp120 to the intracellular site where glycosylation takes place, gp120 which lacks its native secretion signal is not glycosylated. Moreover, with the presence of a non-structural HIV protein such as tat in a fusion protein with the non-glycosylated gp120, CTL responses to the gp120 are augmented. In contrast, Tat in a fusion protein with normal gp120 prevents secretion but does not result in an augmented immune response. The non-glycosylated gp120 can also be successfully expressed in a fusion protein with other HIV antigens, both structural and non-structural.

**Summary of the Invention**

The present invention therefore provides novel constructs for use in nucleic acid or polypeptide vaccines for the prophylaxis and treatment of HIV infections and AIDS.

In one aspect the invention provides a polynucleotide which comprises a sequence encoding the HIV envelope protein gp120, wherein the gp-120 is non-glycosylated when expressed in a mammalian target cell and wherein the gp120 lacks a functional secretion signal, linked to a sequence encoding HIV RT, HIV Gag and HIV Nef, operably linked to a heterologous promoter to encode a gp120, RT, Gag and Nef - containing fusion protein.

In the following preferred embodiments the fusion protein is selected from:

gp120-RT-Nef-Gag, and
RT-Nef Gag-gp120

Optionally the Nef sequence for use in the invention is truncated to remove the sequence encoding the N terminal region i.e. removal of 30-85, preferably 60-85, preferably the N terminal 65 amino acids (the latter truncation is referred to herein as trNef).
Advantageously the Nef may be modified to remove one or more myristylation sites. For example the Gly 2 myristylation site may be removed by deletion or substitution.
Alternatively or additionally the Nef may be modified to alter the dileucine motif of Leu 174 and Leu 175 by deletion or substitution of one or both leucines. The importance of the dileucine motif in CD4 downregulation is described e.g. in Bresnahan P.A. et al (1998) Current Biology, 8 (22): 1235-8.

The RT polynucleotide for use in the invention preferably encodes a mutation to substantially inactivate any reverse transcription activity. A preferred inactive mutant involves the substitution of W tryptophan 229 for K lysine. See WO 03/025003.

Preferably the Gag for use in the invention does not encode the Gag P6 polypeptide. Preferred Gag sequences for use in the invention comprise P 17 and/or 24.

Preferably one or more of the HIV sequences included in the polynucleotide according to the invention encoding gp120, Nef, Gag or RT is or are codon optimised for mammalian cells, most preferably such that it/they resemble a highly expressed human gene in their codon use.

The fusion may contain further HIV sequences.

The polynucleotide sequences are preferably codon optimised for mammalian cells, in line with preferred aspects of the invention.

HIV envelope proteins such as gp120 expressed in a mammalian cell will normally be glycosylated. The polynucleotide according to the invention comprises a gp120 encoding sequence which is adapted to prevent glycosylation in a mammalian target cell, particularly a human target cell. Glycosylation may be reduced or prevented in a number of different ways, for example by removal of or mutation of the glycosylation sites or by removing the native secretion signal. In the polynucleotide construct according to the invention the gyp120 lacks a functional secretion signal. The secretion signal may vary in length between HIV isolates, for example it is 30 amino acids long in the W61D isolate described herein, but may be more or less than that for different isolates. Generally the secretion signal is clearly delineated and will be removed in its entirety, although this is not necessarily the case. A sufficient amount of the signal will be removed to prevent its function of taking the envelope protein to the cellular machinery responsible for glycosylation. This can be easily tested for.

The invention preferably relates to HIV-1. It is preferred that the constructs described herein are derived from an HIV clade B or clade C, particularly clade B.

Preferably the promoter is the promoter from HCMV IE gene, more particularly wherein the 5' untranslated region of the HCMV IE gene comprising exon 1 is included as described in WO 02/36792.

In another aspect the invention provides a vector comprising the polynucleotide sequences described herein. The polynucleotide sequence is preferably DNA and is preferably contained within a vector which is a double stranded DNA plasmid. Alternative vectors are described hereinbelow and include in particular adenovirus vectors such as chimp derived adenovirus vectors Pan 9 or Pan 5, 6 and 7, preferably where these are replication defective such that they cannot replicate in the target cells.

In one embodiment the invention provides a fusion protein comprising the HIV envelope protein gp120, HIV RT, HIV Gag and HIV Nef, wherein the gp120 is non-glycosylated when expressed in a mammalian target cell and wherein the gp120 lacks a functional secretion signal.

In another embodiment the invention provides a fusion protein as defined herein, expressed from a polynucleotide which is codon optimised for mammalian cells.

In a further aspect the invention provides pharmaceutical compositions comprising the nucleotide sequences and vectors and fusion proteins described herein, together with a pharmaceutically acceptable excipient, diluent, carrier or adjuvant. In a preferred embodiment the polynucleotide, preferably in the form of a DNA vector and preferably comprising at least one codon optimised HIV sequence, is present in a composition comprising a plurality of particles, preferably beads such as gold beads, onto which the DNA is coated.

Delivery of polynucleotides according to the invention is preferably carried out by particle mediated delivery, particularly via a bombardment approach.

It is envisaged that the vectors according to the invention may be utilised with immunostimulatory agents, preferably but not necessarily administered at the same time as the vectors and preferably formulated together in the compositions according to the invention.

Immunostimulatory agents for use in the invention include, but this list is by no means exhaustive and does not preclude other agents: synthetic imidazoquinolines such as imiquimod [S-26308, R-837], (Harrison, et al. 'Reduction of recurrent HSV disease using imiquimod alone or combined with a glycoprotein vaccine', Vaccine 19: 1820-1826, (2001)); and resiquimod [S-28463, R-848] (Vasilakos, et al.' Adjuvant activites of immune response modifier R-848: Comparison with CpG ODN', Cellular immunology 204: 64-74 (2000).), Schiff bases of carbonyls and amines that are constitutively expressed on antigen presenting cell and T-cell surfaces, such as tucaresol (Rhodes, J. et al. 'Therapeutic potentiation of the immune system by costimulatory Schiff-base-forming drugs', Nature 377: 71-75 (1995)), cytokine, chemokine and co-stimulatory molecules as either protein or peptide or DNA, this would include pro-inflammatory cytokines such as GM-CSF, IL-1 alpha, IL-1 beta, TGF- alpha and TGF - beta, Th1 inducers such as interferon gamma, IL-2, IL-12, IL-15 and IL-18, Th2 inducers such as IL-4, IL-5, IL-6, IL-10 and IL-13 and other chemokine and co-stimulatory genes such as MCP-1, MIP-1 alpha, MIP-1 beta, RANTES, TCA-3, CD80, CD86 and CD40L, other immunostimulatory targeting ligands such as CTLA-4 and L-selectin, apoptosis stimulating proteins and peptides such as Fas, (49), synthetic lipid based adjuvants, such as vaxfectin, (Reyes et al., 'Vaxfectin enhances antigen specific antibody titres and maintains Th1 type immune responses to plasmid DNA immunization', Vaccine 19: 3778-3786) squalene, alpha-tocopherol, polysorbate 80, DOPC and cholesterol, endotoxin, [LPS], Beutler, B., 'Endotoxin, 'Toll-like receptor 4, and the afferent limb of innate immunity', Current Opinion in Microbiology 3: 23-30 (2000)) ; CpG oligo- and di-nucleotides, Sato, Y. et al., 'Immunostimulatory DNA sequences necessary for effective intradermal gene immunization', Science 273 (5273): 352-354 (1996). Hemmi, H. et al., 'A Toll-like receptor recognizes bacterial DNA', Nature 408: 740-745, (2000) and other potential ligands that trigger Toll receptors to produce appropriate Th1-inducing cytokines, such as synthetic Mycobacterial lipoproteins, Mycobacterial protein p19, peptidoglycan, teichoic acid and lipid A.

A preferred immunostimulatory agent for use with the invention is GM-CSF. This may be employed in the form of a polynucleotide expressing GM-CSF which is co-administered with the DNA vaccine of the invention. A DNA plasmid encoding GM-CSF may be present in a pharmaceutical composition comprising the polynucleotide(s) according to the invention.

Certain preferred adjuvants for eliciting a predominantly Th1-type response include, for example, a Lipid A derivative such as monophosphoryl lipid A, or preferably 3-de-O-acylated monophosphoryl lipid A. MPL® adjuvants are available from Corixa Corporation (Seattle, WA; *see,* for example, US Patent Nos. 4,436,727; 4,877,611; 4,866,034 and 4,912,094). CpG-containing oligonucleotides (in which the CpG dinucleotide is unmethylated) also induce a predominantly Th1 response. Such oligonucleotides are well known and are described, for example, in WO 96/02555, WO 99/33488 and U.S. Patent Nos. 6,008,200 and 5,856,462. Immunostimulatory DNA sequences are also described, for example, by Sato et al., Science 273:352, 1996. Another preferred adjuvant comprises a saponin, such as Quil A, or derivatives thereof, including QS21 and QS7 (Aquila Biopharmaceuticals Inc., Framingham, MA); Escin; Digitonin; or *Gypsophila* or *Chenopodium quinoa* saponins.

According to a further aspect of the invention, a host cell comprising a polynucleotide sequence according to the invention, or an expression vector according to the invention, is provided. The host cell may be for example bacterial e.g. *E. coli,* mammalian e.g. human, or may be an insect cell. Mammalian cells comprising a vector according to the present invention may be cultured cells transfected *in vitro* or may be cells *transfected in vivo* by administration of the vector to the mammal.

By codon optimisation is meant that the DNA sequence is optimised to resemble the codon usage of genes in mammalian cells. In particular, the codon usage in the sequence is optimised to resemble that of highly expressed human genes.

The DNA code has 4 letters (A, T, C and G) and uses these to spell three letter "codons" which represent the amino acids the proteins encoded in an organism's genes. The linear sequence of codons along the DNA molecule is translated into the linear sequence of amino acids in the protein(s) encoded by those genes. The code is highly degenerate, with 61 codons coding for the 20 natural amino acids and 3 codons representing "stop" signals. Thus, most amino acids are coded for by more than one codon - in fact several are coded for by four or more different codons.

Where more than one codon is available to code for a given amino acid, it has been observed that the codon usage patterns of organisms are highly non-random. Different species show a different bias in their codon selection and, furthermore, utilisation of codons may be markedly different in a single species between genes which are expressed at high and low levels. This bias is different in viruses, plants, bacteria and mammalian cells, and some species show a stronger bias away from a random codon selection than others. For example, humans and other mammals are less strongly biased than certain bacteria or viruses. For these reasons, there is a significant probability that a mammalian gene expressed in *E. coli* or a foreign or recombinant gene expressed in mammalian cells will have an inappropriate distribution of codons for efficient expression. It is believed that the presence in a heterologous DNA sequence of clusters of codons or an abundance of codons which are rarely observed in the host in which expression is to occur, is predictive of low heterologous expression levels in that host.

In an embodiment of the present invention there is provided a gp120 polynucleotide sequence which encodes a non-glycosylated gp120 amino acid sequence, wherein the codon usage pattern of the polynucleotide sequence resembles that of highly expressed mammalian genes. Preferably the polynucleotide sequence is a DNA sequence. Desirably the codon usage pattern of the polynucleotide sequence is typical of highly expressed human genes.

In the polynucleotides of the present invention, the codon usage pattern is altered from that typical of human immunodeficiency viruses to more closely represent the codon bias of the target organism, e.g. a mammal, especially a human. The "codon usage coefficient" is a measure of how closely the codon pattern of a given polynucleotide sequence resembles that of a target species. Codon frequencies can be derived from literature sources for the highly expressed genes of many species (see e.g. Nakamura et.al. Nucleic Acids Research 1996, 24:214-215). The codon frequencies for each of the 61 codons (expressed as the number of occurrences occurrence per 1000 codons of the selected class of genes) are normalised for each of the twenty natural amino acids, so that the value for the most frequently used codon for each amino acid is set to 1 and the frequencies for the less common codons are scaled to lie between zero and 1. Thus each of the 61 codons is assigned a value of 1 or lower for the highly expressed genes of the target species. In order to calculate a codon usage coefficient for a specific polynucleotide, relative to the highly expressed genes of that species, the scaled value for each codon of the specific polynucleotide are noted and the geometric mean of all these values is taken (by dividing the sum of the natural logs of these values by the total number of codons and take the antilog). The coefficient will have a value between zero and 1 and the higher the coefficient the more codons in the polynucleotide are frequently used codons. If a polynucleotide sequence has a codon usage coefficient of 1, all of the codons are "most frequent" codons for highly expressed genes of the target species.

According to the present invention, the codon usage pattern of the polynucleotide will preferably exclude rare codons. Rare codons can be defined as codons representing <20% or more preferably representing <10% of the codons used for a particular amino acid in highly expressed genes of the target organism. Alternatively rare codons may be defined as codons with a relative synonymous codon usage (RSCU) value of <0.3 or more preferably <0.2 in highly expressed genes of the target organism. An RSCU value is the observed number of codons divided by the number expected if all codons for that amino acid were used equally frequently. An appropriate definition of a rare codon would be apparent to a person skilled in the art.

A polynucleotide of the present invention will generally have a codon usage coefficient for highly expressed human genes of greater than 0.3, preferably greater than 0.4, most preferably greater than 0.5. Preferably also the codon usage coefficient will be less than 1.0, preferably less than 0.9 and more preferably less than 0.8. Thus a codon usage coefficient between 0.5 and 0.9 or between 0.5 and 0.8 is most preferred. Codon usage tables for human can also be found in Genbank.

In comparison, a highly expressed beta actin gene has a codon usage coefficient of 0.747.

The codon usage table for a homo sapiens is set out below:

**Codon usage for human (highly expressed) genes** 1/24/91 (human_high.cod)

| AmAcid | Codon | Number | /1000 | Fraction | . . |
|---|---|---|---|---|---|
| Gly | GGG | 905.00 | 18.76 | 0.24 | |
| Gly | GGA | 525.00 | 10.88 | 0.14 | |
| Gly | GGT | 441.00 | 9.14 | 0.12 | |
| Gly | GGC | 1867.00 | 38.70 | 0.50 | |
| | | | | | |
| Glu | GAG | 2420.00 | 50.16 | 0.75 | |
| Glu | GAA | 792.00 | 16.42 | 0.25 | |
| Asp | GAT | 592.00 | 12.27 | 0.25 | |
| Asp | GAC | 1821.00 | 37.75 | 0.75 | |
| | | | | | |
| Val | GTG | 1866.00 | 38.68 | 0.64 | |
| Val | GTA | 134.00 | 2.78 | 0.05 | |
| Val | GTT | 198.00 | 4.10 | 0.07 | |
| Val | GTC | 728.00 | 15.09 | 0.25 | |
| | | | | | |
| Ala | GCG | 652.00 | 13.51 | 0.17 | |
| Ala | GCA | 488.00 | 10.12 | 0.13 | |
| Ala | GCT | 654.00 | 13.56 | 0.17 | |
| Ala | GCC | 2057.00 | 42.64 | 0.53 | |
| | | | | | |
| Arg | AGG | 512.00 | 10.61 | 0.18 | |
| Arg | AGA | 298.00 | 6.18 | 0.10 | |
| Ser | AGT | 354.00 | 7.34 | 0.10 | |
| Ser | AGC | 1171.00 | 24.27 | 0.34 | |
| | | | | | |
| Lys | AAG | 2117.00 | 43.88 | 0.82 | |
| Lys | AAA | 471.00 | 9.76 | 0.18 | |
| Asn | AAT | 314.00 | 6.51 | 0.22 | |
| Asn | AAC | 1120.00 | 23.22 | 0.78 | |
| | | | | | |
| Met | ATG | 1077.00 | 22.32 | 1.00 | |
| Ile | ATA | 88.00 | 1.82 | 0.05 | |
| Ile | ATT | 315.00 | 6.53 | 0.18 | |
| Ile | ATC | 1369.00 | 28.38 | 0.77 | |
| | | | | | |
| Thr | ACG | 405.00 | 8.40 | 0.15 | |
| Thr | ACA | 373.00 | 7.73 | 0.14 | |
| Thr | ACT | 358.00 | 7.42 | 0.14 | |
| Thr | ACC | 1502.00 | 31.13 | 0.57 | |
| | | | | | |
| Trp | TGG | 652.00 | 13.51 | 1.00 | |
| End | TGA | 109.00 | 2.26 | 0.55 | |
| Cys | TGT | 325.00 | 6.74 | 0.32 | |
| Cys | TGC | 706.00 | 14.63 | 0.68 | |

(continued)

| AmAcid | Codon | Number | /1000 | Fraction | . . |
|---|---|---|---|---|---|
| End | TAG | 42.00 | 0.87 | 0.21 | |
| End | TAA | 46.00 | 0.95 | 0.23 | |
| Tyr | TAT | 360.00 | 7.46 | 0.26 | |
| Tyr | TAC | 1042.00 | 21.60 | 0.74 | |
| Leu | TTG | 313.00 | 6.49 | 0.06 | |
| Leu | TTA | 76.00 | 1.58 | 0.02 | |
| Phe | TTT | 336.00 | 6.96 | 0.20 | |
| Phe | TTC | 1377.00 | 28.54 | 0.80 | |
| Ser | TCG | 325.00 | 6.74 | 0.09 | |
| Ser | TCA | 165.00 | 3.42 | 0.05 | |
| Ser | TCT | 450.00 | 9.33 | 0.13 | |
| Ser | TCC | 958.00 | 19.86 | 0.28 | |
| Arg | CGG | 611.00 | 12.67 | 0.21 | |
| Arg | CGA | 183.00 | 3.79 | 0.06 | |
| Arg | CGT | 210.00 | 4.35 | 0.07 | |
| Arg | CGC | 1086.00 | 22.51 | 0.37 | |
| Gln | CAG | 2020.00 | 41.87 | 0.88 | |
| Gln | CAA | 283.00 | 5.87 | 0.12 | |
| His | CAT | 234.00 | 4.85 | 0.21 | |
| His | CAC | 870.00 | 18.03 | 0.79 | |
| Leu | CTG | 2884.00 | 59.78 | 0.58 | |
| Leu | CTA | 166.00 | 3.44 | 0.03 | |
| Leu | CTT | 238.00 | 4.93 | 0.05 | |
| Leu | CTC | 1276.00 | 26.45 | 0.26 | |
| Pro | CCG | 482.00 | 9.99 | 0.17 | |
| Pro | CCA | 456.00 | 9.45 | 0.16 | |
| Pro | CCT | 568.00 | 11.77 | 0.19 | |
| Pro | CCC | 1410.00 | 29.23 | 0.48 | |

According to a further aspect of the invention, an expression vector is provided which comprises and is capable of directing the expression of a polynucleotide sequence according to the first aspect of the invention, in particular wherein the codon usage pattern of the gyp120 polynucleotide sequence is typical of highly expressed mammalian genes, preferably highly expressed human genes. The vector may be suitable for driving expression of heterologous DNA in bacterial insect or mammalian cells, particularly human cells. In one embodiment, the expression vector is p7313 (see Figure 1).

Administration of the pharmaceutical composition of the invention may take the form of one or of more than one individual doses, for example as repeat doses of the same DNA plasmid, or in a heterologous "prime-boost" vaccination regime, particularly a therapeutic vaccination regime. A heterologous prime-boost regime uses administration of different forms of vaccine in the prime and the boost, each of which may itself include two or more administrations. Preferably but not necessarily the priming and boosting composition comprise the same antigens or different forms of the same antigens. The priming composition and the boosting composition will anyway have at least one antigen in common, although it is not necessarily an identical form of the antigen, it may be a different form of the same antigen. An example of different forms of the same antigen is in the case of a polynucleotide encoding a gp120 which lacks a functional signal sequence and is non-glycosylated in mammalian cells, and a poplypeptide which is gp120 with its signal sequence and which is glycosylated. A full length and a truncated version of the same protein, or a mutated and a non-mutated form of the same protein, may also be considered different forms of the same antigen for the purposes of a prime-boost format according to the invention.

In one example of a prime-boost regime the "prime" vaccination may be via particle mediated DNA delivery of a priming composition which comprises a polynucleotide according to the present invention, preferably incorporated into a plasmid vector, while the "boost" administration may be of a boosting composition comprising a recombinant viral vector comprising the same polynucleotide sequence or a polynucleotide encoding at least one of the same antigens encoded by the priming composition. Alternatively the boosting may be carried out with at least one of the same antigens in the form of the protein in adjuvant. Conversely the priming may be with a priming composition comprising the viral vector or with a protein formulation typically a protein formulated in adjuvant, and the boost a DNA vaccine of the present invention.

A preferred prime-boost format for use with the polynucleotides according to the present invention is selected from:

Protein prime / live vector boost
Live vector prime / protein boost
Protein prime / DNA plasmid boost
DNA plasmid prime / protein boost
Live vector prime / DNA plasmid boost
DNA plasmid prime / live vector boost

Preferred live vectors include live virus vectors in particular adenovirus vectors as described herein.

Preferably the priming and boosting compositions comprise, in addition to the antigen(s) a suitable adjuvant, which may be different according to the particular composition.

Both the priming composition and the boosting composition may be delivered in more than one dose. Furthermore the initial priming and boosting doses may be followed up with further doses which may be alternated to result in e. g. a DNA plasmid prime/ protein boost/ further DNA plasmid dose/ further protein dose.

The invention further provides a process for the production of a polynucleotide as described herein comprising linking a nucleotide sequence encoding HIV envelope protein gp120, wherein the gp120 is non-glycosylated when expressed in a mammalian target cell and wherein gp120 lack a functional secretion signal, and a sequence encoding HIV Nef, NIV RT and HIV Gag.

As discussed above, the present invention includes expression vectors that comprise the nucleotide sequences of the invention. Such expression vectors are routinely constructed in the art of molecular biology and may for example involve the use of plasmid DNA and appropriate initiators, promoters, enhancers and other elements, such as for example polyadenylation signals which may be necessary, and which are positioned in the correct orientation, in order to allow for protein expression. Other suitable vectors and how to construct them would be apparent to persons skilled in the art. By way of further example in this regard we refer to Sambrook et al. Molecular Cloning: a Laboratory Manual. 2nd Edition. CSH Laboratory Press.(1989).

Preferably, a polynucleotide of the invention, or for use in the invention in a vector, is operably linked to a control sequence which is capable of providing for the expression of the coding sequence by the host cell, i.e. the vector is an expression vector. The term "operably linked" refers to a juxtaposition wherein the components described are in a relationship permitting them to function in their intended manner. A regulatory sequence, such as a promoter, "operably linked" to a coding sequence is positioned in such a way that expression of the coding sequence is achieved under conditions compatible with the regulatory sequence.

A nucleic acid sequence of the present invention may be administered by means of specialised delivery vectors useful in gene therapy. Gene therapy approached are discussed for example by Verme et al, Nature 1997, 389:239-242. Both viral and non-viral vector systems can be used. The vectors may be, for example, plasmids, artificial chromosomes (e.g. BAC, PAC, YAC), virus or phage vectors provided with a origin of replication, optionally a promoter for the expression of the polynucleotide and optionally a regulator of the promoter. The vectors may contain one or more selectable marker genes, for example an ampicillin or kanamycin resistance gene in the case of a bacterial plasmid or a resistance gene for a fungal vector. Vectors may be used *in vitro,* for example for the production of DNA or RNA or used to transfect or transform a host cell, for example, a mammalian host cell e.g. for the production of protein encoded by the vector. The vectors may also be adapted to be used *in vivo,* for example in a method of DNA vaccination or of gene therapy.

Examples of suitable viral vectors include retroviral, lentiviral, adenoviral, adeno-associated viral, herpes viral such as herpes simplex viral, alpha-viral, pox viral such as Canarypox and vaccinia-viral based systems. Gene transfer techniques using these viruses are known to those skilled in the art. Retrovirus vectors for example may be used to stably integrate the polynucleotide of the invention into the host genome, although such recombination is not preferred. Replication-defective adenovirus vectors by contrast remain episomal and therefore allow transient expression. Vectors capable of driving expression in insect cells (for example baculovirus vectors), in human cells, yeast or in bacteria may be employed in order to produce quantities of the HIV protein encoded by the polynucleotides of the present invention, for example for use as subunit vaccines or in immunoassays.

In a preferred embodiment the adenovirus used as a live vector is a replication defective simian adenovirus. Typically these viruses contain an E1 deletion and can be grown on cell lines that are transformed with an E1 gene. Preferred Simian adenoviruses are viruses isolated from Chimpanzee. In particular C68 (also known as Pan 9) (See US patent No 6083 716) and Pan 5, 6 and Pan 7 (W0 03/046124) are preferred for use in the present invention. Thus these vectors can be manipulated to insert a heterologous gene of the invention such that the gene product maybe expressed. The use, formulation and manufacture of such recombinant adenoviral vectors is set forth in detail in WO 03/046142.

Promoters and other expression regulation signals may be selected to be compatible with the host cell for which expression is designed. For example, mammalian promoters include the metallothionein promoter which can be induced in response to heavy metals such as cadmium, and the β-actin promoter. Viral promoters such as the SV40 large T antigen promoter, human cytomegalovirus (CMV) immediate early (IE) promoter, rous sarcoma virus LTR promoter, adenovirus promoter, or a HPV promoter, particularly the HPV upstream regulatory region (URR) may also be used. All these promoters are well described and readily available in the art.

A preferred promoter element is the CMV immediate early promoter devoid of intron A, but including exon 1. Accordingly there is provided a vector comprising a polynucleotide of the invention under the control of HCMV IE early promoter. A suitable HCMV IE promoter is described in WO 02/36792.

Non-viral based systems include direct administration of nucleic acids, microsphere encapsulation technology, poly(lactide-co-glycolide) and liposome-based systems.

The polynucleotides according to the invention have utility in the production by expression of the encoded proteins, which expression may take place *in vitro, in vivo* or *ex vivo.* The nucleotides may therefore be involved in recombinant protein synthesis, for example to increase yields, or indeed may find use as therapeutic agents in their own right, utilised in DNA vaccination techniques. Where the polynucleotides of the present invention are used in the production of the encoded proteins *in vitro* or *ex vivo,* cells, for example in cell culture, will be modified to include the polynucleotide to be expressed. Such cells include transient, or preferably stable mammalian cell lines. Particular examples of cells which may be modified by insertion of vectors encoding for a polypeptide according to the invention include mammalian HEK293T, CHO, HeLa, 293 and COS cells. Preferably the cell line selected will be one which is stable. Expression may be achieved in transformed oocytes. A polypeptide may be expressed from a polynucleotide of the present invention, in cells of a transgenic non-human animal, preferably a mouse. A transgenic non-human animal expressing a polypeptide from a polynucleotide of the invention is included within the scope of the invention.

Preferably, expression vectors for use in DNA vaccines, vaccine compositions and immunotherapeutics will be plasmid vectors or live viral vectors.

DNA vaccines may be administered in the form of "nakedDNA", for example in a liquid formulation administered using a syringe or high pressure jet, or DNA formulated with liposomes or an irritant transfection enhancer, or by particle mediated DNA delivery (PMDD or particle mediated imunotherapeutic delivery PMID) as described in more detail herein. All of these delivery systems are well known in the art. The vector may be introduced to a mammal for example by means of a viral vector delivery system.

The compositions of the present invention can be delivered by a number of routes such as intramuscularly, subcutaneously, intraperitonally, intravenously or mucosally.

The invention further provides an intradermal delivery device comprising a pharmaceutical composition described herein.

In a preferred embodiment, the composition is delivered intradermally. In particular, the composition is delivered by means of a gene gun particularly using particle bombardment administration techniques which involve coating the vector on to beads (eg gold beads) which are then administered under high pressure into the epidermis. This is described, for example,in Haynes et al, J Biotechnology 44: 37-42 (1996).

Numerous methods of carrying out a particle bombardment approach are known, see for example WO 91/07487. In one illustrative example, gas-driven particle acceleration can be achieved with devices such as those manufactured by Powderject Pharmaceuticals PLC (Oxford, UK) and Powderject Vaccines Inc. (Madison, WI), some examples of which are described in U.S. Patent Nos. 5,846,796; 6,010,478; 5,865,796; 5,584,807; and EP Patent No. 0500 799. This approach offers a needle-free delivery approach wherein a dry powder formulation of microscopic particles, such as polynucleotide, are accelerated to high speed within a helium gas jet generated by a hand held device, propelling the particles into a target tissue of interest, typically the skin. The particles are preferably gold beads of a 0.4 - 4.0 μm, more preferably 0.6 - 2.0 μm diameter and the DNA conjugate coated onto these and then encased in a cartridge or cassette for placing into the delivery device.

In a related embodiment, other devices and methods that may be useful for gas-driven needle-less injection of compositions of the present invention include those provided by Bioject, Inc. (Portland, OR), some examples of which are described in U.S. Patent Nos. 4,790,824; 5,064,413; 5,312,335; 5,383,851; 5,399,163; 5,520,639 and 5,993,412.

The vectors which comprise the nucleotide sequences encoding antigenic peptides are administered in such amount as will be prophylactically or therapeutically effective. The quantity to be administered, is generally in the range of one picogram to 1 milligram, preferably 1 picogram to 10 micrograms for particle-mediated delivery, and 100 nanograms to 10 milligrams for other routes of nucleotide per dose. The exact quantity may vary considerably depending on the weight of the patient being immunised and the route of administration.

It is possible for the immunogen component comprising the nucleotide sequence encoding the antigenic peptide, to be administered on a one off basis or to be administered repeatedly, for example, between 1 and 7 times, preferably between 1 and 4 times, at intervals between about 1 day and about 18 months. Further administrations may also be given as necessary to maintain immune responses for the lifetime of the patient. However, this treatment regime will be significantly varied depending upon the size of the patient concerned, the amount of nucleotide sequence administered, the route of administration, and other factors which would be apparent to a skilled medical practitioner. The patient may receive one or more other anti HIV retroviral drugs as part of their overall treatment regime. Additionally the nucleic acid immunogen may be administered with an adjuvant.

The adjuvant component specified herein can similarly be administered via a variety of different administration routes, such as for example, via the oral, nasal, pulmonary, intramuscular, subcutaneous, intradermal or topical routes. Preferably, the adjuvant component is administered via the intradermal or topical route, most preferably by a topical route. This administration may take place between about 14 days prior to and about 14 days post administration of the nucleotide sequence, preferably between about 1 day prior to and about 3 days post administration of the nucleotide sequence.

The adjuvant component is, in one embodiment, administered substantially simultaneously with the administration of the nucleotide sequence. By "substantially simultaneous" what is meant is that administration of the adjuvant component is preferably at the same time as administration of the nucleotide sequence, or if not, it is at least within a few hours either side of nucleotide sequence administration. In the most preferred treatment protocol, the adjuvant component will be administered substantially simultaneously with administration of the nucleotide sequence. Obviously, this protocol can be varied as necessary, in accordance with the type of variables referred to above. It is preferred that the adjuvant is a 1H - imidazo [4,5c] quinoline - 4 - amine derivative such as imiquimod. Typically imiquimod will be presented as a topical cream formulation and will be administered according to the above protocol.

Once again, depending upon such variables, the dose of administration of the derivative will also vary, but may, for example, range between about 0.1 mg per kg to about 100 mg per kg, where "per kg" refers to the body weight of the mammal concerned. This administration of the 1H-imidazo[4,5-c]quinolin-4-amine derivative would preferably be repeated with each subsequent or booster administration of the nucleotide sequence. Most preferably, the administration dose will be between about 1 mg per kg to about 50 mg per kg. In the case of a "prime-boost" scheme as described herein, the imiquimod or other 1H-imidazo[4,5-c]quinolin-4-amine derivative may be administered with either the prime or the boost or with both the prime and the boost.

While it is possible for the adjuvant component to comprise only 1H-imidazo[4,5-c]quinolin-4-amine derivatives to be administered in the raw chemical state, it is preferable for administration to be in the form of a pharmaceutical formulation. That is, the adjuvant component will preferably comprise the 1H-imidazo[4,5-c]quinolin-4-amine combined with one or more pharmaceutically acceptable carriers, and optionally other therapeutic ingredients. The carrier(s) must be "acceptable" in the sense of being compatible with other ingredients within the formulation, and not deleterious to the recipient thereof. The nature of the formulations will naturally vary according to the intended administration route, and may be prepared by methods well known in the pharmaceutical art. All methods include the step of bringing into association a 1H-imidazo[4,5-c]quinolin-4-amine derivative with an appropriate carrier or carriers. In general, the formulations are prepared by uniformly and intimately bringing into association the derivative with liquid carriers or finely divided solid carriers, or both, and then, if necessary, shaping the product into the desired formulation. Formulations of the present invention suitable for oral administration may be presented as discrete units such as capsules, cachets or tablets each containing a pre-determined amount of the active ingredient; as a powder or granules; as a solution or a suspension in an aqueous liquid or a non-aqueous liquid; or as an oil-in-water liquid emulsion or a water-in-oil emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent.

The tablets may optionally be coated or scored and may be formulated so as to provide slow or controlled release of the active ingredient.

Formulations for injection via, for example, the intramuscular, intraperitoneal, intradermal,or subcutaneous administration routes include aqueous and non-aqueous sterile injection solutions which may contain antioxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampoules and vials, and may be stored in a freeze-dried (lyophilised) condition requiring only the addition of the sterile liquid carrier, for example, water for injections, immediately prior to use. Extemporaneous injection solutions and suspensions maybe prepared from sterile powders, granules and tablets of the kind previously described. Formulations suitable for pulmonary administration via the buccal or nasal cavity are presented such that particles containing the active ingredient, desirably having a diameter in the range of 0.5 to 7 microns, are delivered into the bronchial tree of the recipient. Possibilities for such formulations are that they are in the form of finely comminuted powders which may conveniently be presented either in a piercable capsule, suitably of, for example, gelatine, for use in an inhalation device, or alternatively, as a self-propelling formulation comprising active ingredient, a suitable liquid propellant and optionally, other ingredients such as surfactant and/or a solid diluent. Self-propelling formulations may also be employed wherein the active ingredient is dispensed in the form of droplets of a solution or suspension. Such self-propelling formulations are analogous to those known in the art and may be prepared by established procedures. They are suitably provided with either a manually-operable or automatically functioning valve having the desired spray characteristics; advantageously the valve is of a metered type delivering a fixed volume, for example, 50 to 100 μL, upon each operation thereof.

In a further possibility, the adjuvant component may be in the form of a solution for use in an atomiser or nebuliser whereby an accelerated airstream or ultrasonic agitation is employed to produce a find droplet mist for inhalation.

Formulations suitable for intranasal administration generally include presentations similar to those described above for pulmonary administration, although it is preferred for such formulations to have a particle diameter in the range of about 10 to about 200 microns, to enable retention within the nasal cavity. This may be achieved by, as appropriate, use of a powder of a suitable particle size, or choice of an appropriate valve. Other suitable formulations include coarse powders having a particle diameter in the range of about 20 to about 500 microns, for administration by rapid inhalation through the nasal passage from a container held close up to the nose, and nasal drops comprising about 0.2 to 5% w/w of the active ingredient in aqueous or oily solutions. In one embodiment of the invention, it is possible for the vector which comprises the nucleotide sequence encoding the antigenic peptide to be administered within the same formulation as the 1H-imidazo[4,5-c]quinolin-4-amine derivative. Hence in this embodiment, the immunogenic and the adjuvant component are found within the same formulation.

In one embodiment the adjuvant component is prepared in a form suitable for biolistic administration, and is administered via that route substantially simultaneously with administration of the nucleotide sequence. For preparation of formulations suitable for use in this manner, it may be necessary for the 1H-imidazo[4,5-c]quinolin-4-amine derivative to be lyophilised and adhered onto, for example, particles such as gold beads which are suited for biolistic administration.

In an alternative embodiment, the adjuvant component may be administered as a dry powder, via high pressure gas propulsion.

Even if not formulated together, it may be appropriate for the adjuvant component to be administered at or about the same administration site as the nucleotide sequence.

Other details of pharmaceutical preparations can be found in Remington's Pharmaceutical Sciences, Mack Publishing Company, Easton, Pennysylvania (1985).

Suitable techniques for introducing the naked polynucleotide or vector into a patient also include topical application with an appropriate vehicle. The nucleic acid may be administered topically to the skin, or to mucosal surfaces for example by intranasal, oral, intravaginal or intrarectal administration. The naked polynucleotide or vector may be present together with a pharmaceutically acceptable excipient, such as phosphate buffered saline (PBS). DNA uptake may be further facilitated by use of facilitating agents such as bupivacaine, either separately or included in the DNA formulation. Other methods of administering the nucleic acid directly to a recipient include ultrasound, electrical stimulation, electroporation and microseeding which is described in US 5,697,901.

Uptake of nucleic acid constructs may be enhanced by several known transfection techniques, for example those including the use of transfection agents. Examples of these agents include cationic agents, for example calcium phosphate and DEAE-Dextran and lipofectants, for example lipofectam and transfectam. The dosage of the nucleic acid to be administered can be altered.

A nucleic acid sequence of the present invention may also be administered by means of specialised delivery vectors useful in gene therapy. Gene therapy approaches are discussed for example by Verme et al, Nature 1997, 389: 239-242. Both viral and non-viral vector systems can be used and are described above. Viral and non-viral delivery systems may be combined where it is desirable to provide booster injections after an initial vaccination, for example an initial "prime" DNA vaccination using a non-viral vector such as a plasmid followed by one or more "boost" vaccinations using a viral vector or non-viral based system. Similarly the invention contemplates prime boost systems with the polynucleotide of the invention, followed by boosting with protein in adjuvant or vice versa.

A nucleic acid sequence of the present invention may also be administered by means of transformed cells. Such cells include cells harvested from a subj ect. The naked polynucleotide or vector of the present invention can be introduced into such cells *in vitro* and the transformed cells can later be returned to the subject. The polynucleotide of the invention may integrate into nucleic acid already present in a cell by homologous recombination events. A transformed cell may, if desired, be grown up *in vitro* and one or more of the resultant cells may be used in the present invention. Cells can be provided at an appropriate site in a patient by known surgical or microsurgical techniques (e.g. grafting, micro-injection, etc.)

The pharmaceutical compositions of the present invention may include adjuvant compounds as detailed above, or other substances which may serve to increase the immune response induced by the protein which is encoded by the DNA. These may be encoded by the DNA, either separately from or as a fusion with the antigen, or may be included as non-DNA elements of the formulation. Examples of adjuvant-type substances which may be included in the formulations of the present invention include ubiquitin, lysosomal associated membrane protein (LAMP), hepatitis B virus core antigen, FLT3-ligand (a cytokine important in the generation of professional antigen presenting cells, particularly dentritic cells) and other cytokines such as IFN-γ and GMCSF. Other preferred adjuvants include imiquimod and resimquimod and tucarasol, imiquimod being particularly preferred.

In a particular embodiment of the invention there is provided the use of a nucleic acid molecule as herein described for the treatment or prophylaxis of HIV infection, administered with imiquimod. The imiquimod is preferably administered topically, whereas the nucleic acid molecule is preferably administered by means of particle mediated delivery.

The present invention will now be described by reference to the following examples, references to Tat being provided by way of comparison and do not form part of the claimed invention.

EXAMPLES

**Example 1: <u>Plasmid Construction</u>**

### 1.1 Construction of gp120 containing plasmid

Recombinant gp120 glycoprotein described in the following examples is a synthetic form of the gp120 envelope protein of HIV-1 isolate W61D.

<u>Codon Optimised (pgp120c):</u>

The gene sequence was based on the gp120 sequence from the HIV-1 isolate W61D. This has a Codon Usage Coefficient of 0.297. Optimisation was performed using SynGene 2d, resulting in a CUC of 0.749 (Ertl, PF., Thomsen, LL. Technical issues in construction of nucleic acid vaccines. (2003) Methods 31(3); 199-206. SynGene uses a mathematical method for codon optimisation based on the relative frequencies of use. Briefly, codons are assigned value ranges according to their frequencies, so that more frequent codons have wider ranges, and placed in ascending frequency order. The value ranges are expressed as >=0.000, >=0.0??, >=0.??? And so on. A random number is generated between 0 and 0.99999. This is then used to select a codon, which will be the codon allocated the range within which the random number falls. To exclude rare codons the value 0.1 is added to the random number, so that it falls in the range 0.1-1.09999.

The gp120 sequence was split into 40 overlapping oligonucleotides, PCR assembled and recovered using the end primers. The gene was cloned into vector p7313-ie (shown in Figure 1) as aNotI-BamHI fragment and sequenced. Restriction fragments from three initial clones were combined to generate a single correct clone. The amino acid sequence and codon optimised DNA sequence are given in Figure 2.

### 1.2 Generation of Nef/Tat containing plasmids

<u>**Nef/Tat** (pNTm and ptrNTm)</u>

The gene for the Nef/Tat fusion protein was provided in plasmid pRIT15244 (Figure 3). The plasmid pRIT 15244 is identical to pRIT 14913 described below except that the His tail has been deleted.

<u>General</u>

The Nef gene from the Bru/Lai isolate (Cell 40: 9-17, 1985) was selected for the constructs since this gene is among those that are most closely related to the consensus Nef.

The starting material for the Bru/Lai Nef gene was a 1170bp DNA fragment cloned on the mammalian expression vector pcDNA3 (pcDNA3/Nef).

The Tat gene originates from the BH10 molecular clone. This gene was received as an HTLV III cDNA clone named pCV and described in Science, 229, p69-73, 1985. This *tat* gene bears mutations in the active site region (Lys41→Ala) and in RGD motif (Arg78→Lys and Asp80→Glu) (Virology 235: 48-64, 1997).
The mutant *tat* gene was received as a cDNA fragment subcloned between the EcoRI and HindIII sites within a CMV expression plasmid (pCMVLys41/KGE)

Construction of vector pRIT14597 (encoding Nef-His protein).

The *nef* gene was amplified by PCR from the pcDNA3/Nef plasmid with primers 01 and 02.

NcoI

PRIMER 01: 5'ATCGTCCATG.GGT.GGC.AAG.TGG.T 3' [SEQ ID NO: 1]

SpeI

PRIMER 02: 5' CGGCTACTAGTGCAGTTCTTGAA 3' [SEQ ID NO:2]

The integrative vector PHIL-D2 (INVITROGEN) was used. This vector was modified in such a way that expression of heterologous protein starts immediately after the native ATG codon of the AOX1 gene and will produce recombinant protein with a tail of one glycine and six histidines residues. This PHIL-D2-MOD vector was constructed by cloning an oligonucleotide linker between the adjacent AsuII and EcoRI sites of PHIL-D2 vector. In addition to the His tail, this linker carries NcoI, SpeI and XbaI restriction sites between which *nef, tat* and *nef-tat* fusion were inserted.
The nef PCR fragment obtained and the integrative PHIL-D2-MOD vector were both restricted by NcoI and SpeI, purified on agarose gel and ligated to create the integrative plasmid pRIT14597.

Construction of vector pRIT14913 (encoding fusion Nef-Tat mutant-His).

To construct pRIT14913, the *tat* mutant gene was amplified by PCR from the pCMVLys41/KGE plasmid with primers 03 and 04.

SpeI

PRIMER 03: 5' ATCGTACTAGT.GAG.CCA.GTA.GAT.C 3' [SEQ ID NO: 3]

SpeI

PRIMER 04: 5' CGGCTACTAGTTTCCTTCGGGCCT 3' [SEQ ID NO: 4]

The PCR fragment obtained and the plasmid pRIT14597 (expressing Nef-His protein) were both digested by SpeI restriction enzyme, purified on agarose gel and ligated to create the integrative plasmid pRIT14913.

**1.3 Generation of PMID vectors for gp120 and Nef/Tat:**

**gp120**: Codon-optimised gp120 was provided as described above.

**Nef/Tat** (pNTm and ptrNTm):

The gene for the Nef/Tat fusion protein was provided in plasmid pRIT15244 described above. The Tat in this plasmid contains three mutations to inactivate the transactivation function. The fusion contains full length Nef which has an immune modulatory function (Collins and Baltimore (1999)) that may be abrogated by N-terminal truncation. Therefore constructs were generated for both full length Nef/mutant Tat(pNTm) and truncated Nef/mutant Tat(ptrNTm), in which the first 65 amino acids of Nef were removed. These sequences were PCR amplified from pRIT15244 using primers:

5'Nef GAATTCGCGGCCGCCATGGGTGGCAAGTGGTCAAAAAG

5'trNef GAATTCGCGGCCGCCATGGTGGGTTTTCCAGTCACACC

3'Tat GAATTCGGATCCTTATTCCTTCGGGCCTGTCGGG

[SEQ ID NOS: 5,6,7]

The genes were cloned into vector p7313-ie as NotI-BamHI fragments and sequenced. PNTm and ptrNTm and the Nef/Tat and truncated Nef/Tat sequences are shown in Figures 4 and 5.

**Dual expression vectors:** (pRIX1 and pRIX2)

The Nef/Tat and trNef/Tat expression cassettes were excised as ClaI-XmnI restriction fragments, and ligated into the ClaI and blunted Sse8387 I sites of the vector containing the codon optimised gp120 (pgp120c) to provide single plasmids for expression of both proteins (pRIX1 and pRIX2 respectively).

**Composition of plasmid p7313-ie (Figure 1)**

The plasmid was constructed by replacing the beta-lactamase gene containing Eam1105I - Pst1 fragment of pUC19 (available from Amersham Pharmacia Biotech UK Ltd., Amersham Place, Little Chalfont, Bucks, HP7 9NA) with an EcoRI fragment of pUC4K (Amersham-Pharmacia) containing the Kanamycin resistance gene, following blunt ending of both fragments using T4 DNA polymerase. The human Cytomegalovirus IE1 promoter /enhancer, Intron A, was derived from plasmid JW4303 obtained from Dr Harriet Robinson, University of Massachusetts, and inserted into the Sal1 site of pUC19 as a XhoI -Sal1 fragment, incorporating the bovine growth hormone polyadenylation signal. Deletion of the 5'SalI-BanI fragment from the promoter generated the minimal promoter used in the vector (WO00/23592 - Powderject Vaccines Inc.). HBV Surface antigen 3'UTR was derived from Hepatitis B Virus, serotype adw, in the vector pAM6 (Moriarty et al., Proc.Natl.Acad.Sci. USA, 78, 2606-10,1981). pAM6 (pBR322 based vector) was obtained from the American Type Culture Collection, catalogue number ATCC 45020. The 3'UTR was inserted 5' to the polyadenylation signal as a 1.4kb BamHI fragment, blunt ended for insertion to remove the BamHI sites. In a series of steps (including digestion with *Bgl* II, Klenow polymerase treatment, digestion with *Bst*X I, digestion with *Nco* I, treatment with mung bean nuclease to remove overhang and further digestion with *BstX* I), modifications were made to the region between the 3'untranslated enhancer region of the HBV S gene and bGHpA signal to remove all open reading frames of greater than 5 codons between the X gene promoter and the bGHpA signal. This resulted in deletion of sequence encoding the translatable portion of the X protein (9 amino acids) and the X gene start codon. The bovine growth hormone polyadenylation signal was substituted with the rabbit beta globin polyadenylation signal. The 5'non-coding and coding sequences of the S antigen were excised and replaced with an oligonucleotide linker to provide multiple cloning sites as shown to produce plasmid p7313-PL.

```
Hind---NotI--     EcoRV-       -NdeI-     -BamHI
AGCTTGCGGCCGCTAGCGATATCGGTACCATATGTCGACGGATCC....
....ACGCCGGCGATCGCTATAGCCATGGTCTACAGCTGCCTAGGCCGG
           -NheI-        -KpnI-     -SalI-     ΔNotI
```

[SEQ ID NO: 8]

This polylinker was further extended by insertion of an additional oligonucleotide linker between the KpnI and

Sall sites:

```
AspI-    -MunI-  NaeI-      NdeI--     BglII-
GTACCGGTCAATTGGCGCCGGCGCGCCATATGACGTCAGATCTG----
----GCCAGTTAACCGCGGCCGCGCGGTATACTGCAGTCTAGACAGCT
--AgeI-      -NarI--              AatII-        SalI
```

[SEQ ID NO: 9]

The ColE1 cer sequence was obtained from a subclone from plasmid pDAH212 from David Hodgeson (Warwick University) and amplified by PCR using primers to place EcoRI restriction sites at the ends of the sequence. The cer sequence was then inserted into the EcoRI site of p7313-PL to produce plasmid p7313-PLc. The sequence of the amplified cer was verified against the Genbank entry M11411.
The HBV 3'UTR sequence between the promoter and polyadenylation signal was removed by PCR amplification of the polyadenylation signal using primers:

sense: CCATGGATCCGATCTTTTTCCCTCTGCC [SEQ ID NO: 10]
antisense: GTTAGGGTGAAAAGCTTCCGAGTGAGAGACAC [SEQ ID NO: 11]

The resulting product was cut with BamHI and XmnI and used to replace the corresponding fragment containing both the polyadenylation signal and the 3'UTR. The Intron A sequence was removed from the plasmid by PCR amplification of the CMV promoter/enhancer using primers:

sense: GCTAGCCTGCAGGCTGACCGCCCAACGAC [SEQ ID NO: 12]
antisense: GTTCTCCATCGCGGCCGCACTCTTGGCACGGGG [SEQ ID NO: 13]

The resulting product was cut with Sse8387 I and NotI, and inserted back into the Sse8387 I and NotI sites of the parental vector.

**Example 2: Modification of pg120 and Nef/Tat(mut) expression vectors**

gp120 constructs were modified to reduce secretion of the protein.

**Generation of constructs:**

gp120 without a secretion signal (dsgp120, pRix 12 - see Figure 2 and 6)

The gp120 gene was PCR amplified from pgp120c using the following primers:

5' 120ds: 5'GAATTCGCGGCCGCCATGGCCGAGCAGCTGTGGGTCACC [SEQ ID NO:14]

L01:

5'GAATTCGGATCCTCATCTCTGCACGACGCGGCGCTTGGCCCGGT GGGGGCCACG [SEQ ID NO: 15]

Fragments were amplified using PWO DNA polymerase (Roche) and the cycle:

95°C (30s) 95°C(30s) 50°C(30s) 72°C(90s) 72°C(120s) 4°C(hold)

___ repeat x20 ___/

The products were cut with NotI and BamHI and cloned into p7313-ie to give pRix12 (Figure 6).

**Results**

In 293T cells the vector pRIX12, which lacks the secretion signal, makes a good amount of a 60kDa non-glycosylated protein that is not secreted.

**Example 3: Construction of vectors for expression of gp120 and Nef/Tat(mut) from a single plasmid**

**Vector construction:**

The gp120 Nef/Tat(m) constructs were generated by PCR stitching the gp120 and Nef/Tat(m) or trNef/Tat(m) orfs.

5' and 3' Gp120, 5' and 3' Nef/Tat(m) and 5'trNef/Tat were amplified from pRix1. 3'trNef/Tat(m) was amplified from pNTm. The following primers were used:

3'120: (antisense to): GCCAAGCGCCGCGTCGTGCAGAGA [SEQ ID NO: 16] 5'120/NT: GCCAAGCGCCGCGTCGTGCAGAGAATGGGTGGCAAGTGGTCAAAAAGT [SEQ ID NO:17]
3'NT (antisense to): GGGGAGCCGACAGGCCCGAAGGAA [SEQ ID NO: 18]

5'NT/120: GGGGAGCCGACAGGCCCGAAGGAAATGAAGGTCAAGGAGACCAGAAAG [SEQ ID NO: 19]
5'120/trN: GCCAAGCGCCGCGTCGTGCAGAGAATGGTGGGTTTTCCAGTCAC [SEQ ID NO: 20]
5'trNef: GAATTCGCGGCCGCCATGGTGGGTTTTCCAGTCACACC [SEQID NO: 21]
L01:

GAATTCGGATCCTCATCTCTGCACGACGCGGCGCTTGGCCCGGGTG GGGGCCACG [SEQ ID NO: 22]

L02:

ACCACCTTGTACTTGTACAGCTCGCTCCGCCAGTTATCCCTCATGTC GCCGCCGCCGGGC [SEQ ID NO: 23]

Fragments were amplified using PWO DNA polymerase (Roche) and the cycle:

95°C (60s) 95°C(30s) 55°C(30s) 72°C(120s) 72°C(120s) 4°C(hold)

___ repeat x20 ___/

Primer L1 was used as the 3' primer for 3'gp120. However there were problems using this primer when stitching Nef/Tat or trNef/T to the 5' end of gp120 so primer L2 was used instead.

The stitched gp120-N/Tm and gp120-trN/Tm fragments were cut with Not1 and BamHI and cloned into similarly cut p7313-ie. Due to the use of primer L2 rather than L1 the N/Tm-gp120 and trN/Tm-gp120 fragments lacked a BamHI site, so these were cut with NotI and AccI, and cloned into similarly cut pgp120c. All inserts were fully verified by sequencing. The plasmids were designated pRix6 (gp120c NefTat$^m$), pRix11 (gp120c trNefTat$^m$), pRix7 (NefTatm gp120c) and pRix8 (trNefTat$^m$gp120) (Figures 23-26).

**Example 4: Construction of vectors to invesigate the effects of glycosylation and secretion, inclusion of Tat and inclusion of Gag (p17/24) and Nef and RT on gp120 and gp120 fusions**

Vectors were constructed as shown in Figures 33 and 34 (schematic).

pRix 28 and pRix29 (Figures 7 and 8)

pRix28 and 29 containing ds gp120c NefTat$^m$ and ds gp120c trNefTat were generated by transferring the AccI-BamHI fragments from pRix6 (2315bp) and pRix11 (2123bp) into similarly cut pRix12 (ds gp120c).

pRix30 and pRix31 (Figure 27 and Figure 9)

To generate glycosylated and non-glycosylated fusion vectors of gp120c Nef without Tat, the NotI-KpnI fragment was transferred from pRix11 (1580bp) or pRix29 (1496bp) into similarly cut pRix15, a vector containing Tat/trNef.

(pRix15) - Tat(mut)trNef

The genes for Tat and trNefwere PCR amplified from pNTm using the following primers:

5'Tat: 5'GAATTCGCGGCCGCCATGGAGCCAGTAGATCCTAGAC [SEQ ID NO: 24]
3'Tat: 5'TTCCTTCGGGCCTGTCGGC [SEQ ID NO: 25]
5'trTN: GCCGACAGGCCCGAAGGAAATGGTGGGTTTTCCAGTCACAC [SEQ ID NO: 26]
3'Nef: GAATTCGGATCCTTAGCAGTTCTTGAAGTACTCCGG [SEQ ID NO: 27]

The individual genes were gel purified and then PCR stitched to give TmtrN using the end primers. The fusion was then digested with NotI and BamHI and cloned into p7313-ie.

pRix32 (Figure 28)

To generate the fusion containing p 17/24, gp 120 was PCR amplified from pgp 1 20c using primers U1 and 3'120, p17/24-Nef was amplified from p73i-GN2 using primers 5'120G and 3'Nef, and the two were PCR stitched using U1 and 3'Nef. p73I-GN2 contained a synthetic codon optimised sequence of p17/p24 based on the sequence of HXB2 (GenBank entry K03455) and designed using SynGene and assembled from overlapping oligonucleotides as described for codon optimised gp120 above, fused to HXB2 Nef, which had been obtained from plasmid pHXBΔPr (B.Maschera, E Furfine and E.D. Blair 1995 J.Virol 69 5431-5436) by PCR. Since the HXB2 nef gene in this plasmid contains a premature termination codon two overlapping PCRs were used to repair the codon (TGA [stop] to TGG [Trp]). The position of the repaired codon is underlined in the sequence. The p17/p24/Nef gene was inserted into the NotI and BamHI sites of plasmid p7313ie. The coding sequence and map is given in Figure 10. A * marks the p24/trNef junction.

Primers:

U1:
GAATTCGCGGCCGCAATGAAGGTCAAGGAGACCAGAAAGAACTACCAGC
ATCTGTG [SEQ ID NO: 28]

3'120: TCTCTGCACGACGCGGCGCTTGGC [SEQ ID NO: 29]5'120G: GCCAAGCGCCGCGTCGTGGAGAGAATGGGTGCCCGAGCTTCGGTAC [SEQ ID NO: 30]
3'Nef: GAATTCGGATCCTTAGCAGTTCTTGAAGTACTCCGG [SEQ ID NO: 31]

Initial cycle:

94°C(30s) 20x[94°C (30s) 50°C (30s) 68°C (180s)] 68°C (120s) 4°C (0s)

Using pfx polymerase with 1x enhancer.

Stitch:

94°C(30s) 20x[94°C (30s) 50°C (30s) 72°C (180s)] 72°C (120s) 4°C (0s)

Using Vent polymerase in standard conditions + 2mM $MgCl_2$.

The product was cut with NotI and BamHI and cloned into p7313-ie.

On sequencing the construct was found to have a error in the signal peptide, which was corrected by transferring the 2560bp BstEII - KpnI fragment containing the back of gp120 to the front of Nef into pRix30.

<u>pRix 33 (Figure 11), pRix34 (Figure 29), and pRix35 (Figure 12)</u>

The 2560bp BstEII - KpnI fragment containing the back of gp120 to the front of Nef was transferred to pRix31, pRix11 and pRix29 to make vectors pRix 33 (Figure 11), 34 and 35 (Figure 12) respectively.

<u>pRix39 (gp120 codon optimised minus secretion signal - p17/24 gag - Nef-Tat - Figure 13) and pRix40-47</u> (Constructs pRix 40-47 contain non-glycosylated gp120, gag-p 17/24, Nef and Tat(m) fusions with mutations in the miristoylation site and/or dileucine motif of Nef)

A fragment containing gag p17/24 was PCR amplified from vector pRix35 using primers:

5'120G: GCCAAGCGCCGCGTCGTGGAGAGAATGGGTGCCCGAGCTTCGGTAC [SEQ ID NO: 32]
and
p24AS: CAACACTCTGGCTTTGTGTCC [SEQ ID NO: 33]

Full length Nef was PCR amplified from pNTm using primers:

5'p24-N: GGACACAAAGCCAGAGTGTTGATGGGCAAGTGGTCAAAAAGTAG [SEQ ID NO: 34]
and
3'Nef: GAATTCGGATCCTTAGCAGTTCTTGAAGTACTCCGG [SEQ ID NO: 35]

The two fragments were PCR stitched together using the end primers (5'120G and 3'Nef). The product was cut with SalI and KpnI, and the 423bp fragment containing part of p24 and Nef was used to replace the corresponding fragment in pRix35 to make pRix39 (Figure 13).

pRix40 (Figure 14) was similarly constructed except primer 5'p24-N was replaced with primer:

5'p24-Ndm:

GGACACAAAGCCAGAGTGTTGATGGGCAAGTGGTCAAAAAGTAG [SEQ ID NO: 36]
G H K A R V L| M G K W S K S

This primer deletes the one G to destroy the miristoylation site at the start of Nef.

<u>pRix41-44, 46 and 47 (Figures 15 to 20)</u>

Mutations to the dileucine motif in Nef (L174L175) were made by PCR:

To insert the mutations, the portion of Nef 5' to the LL motif was PCR amplified using the 5'Nef primer and asNefLL
5'Nef GAATTCGCGGCCGCCATGGGTGGCAAGTGGTCAAAAAG [SEQ ID NO: 37]
asNefLL (Antisense to)

GCCAATAAAGGAGAGAACACCAGC [SEQ ID NO: 38]
A N K G E N T S

Mutations to L174, L175 or both 174 and 175 were generated using forward primers

sNefL1 (L174A)

GCCAATAAAGGAGAGAACACCAGCGCCTTACACCCTGTGAGCCTGCATG
A N K G E N T S| A L H P V S L H
[SEQ ID NO: 39]

sNefL2 (L175A)

GCCAATAAAGGAGAGAACACCAGCTTGGCACACCCTGTGAGCCTGCATG
A N K G E N T S| L a H P V S L H
[SEQ ID NO: 40]

SnefLL (LL174/5AA)

GCCAATAAAGGAGAGAACACCAGCGCCGCACACCCTGTGAGCCTGCATG
A N K G E N T S| A A H P V S L H
[SEQ ID NO: 41]

and the 3'NT primer:

3'NT (antisense to):GGGGAGCCGACAGGCCCGAAGGAA [SEQ ID NO: 42]

to amplify the 3' portion of Nef. The 5' and each of the 3' products were PCR stitched using the 5'Nef and 3'NT primers. These were cut with KpnI and SpeI and inserted into similarly cut pRix39 to generate pRix41 (L174A), pRix42 (L175A), and pRix43 (LL174/175A) in the absence of the myristoylation site mutation, or into pRix40 to generate pRix44 (mLL174/175AA) pRix46 (mL174A) and pRix47 (mL175A) with the myristoylation site mutation.

<u>pRix58 (Figure 21)</u>

The gp 120 fragment without signal sequence was PCR amplified from pgp120c using the primers:

5' ds120 : GAATTCGCGGCCGCCATGGCCGAGCAGCTGTGGGTCACC [SEQ ID NO: 43]
3'120: (antisense to): GCCAAGCGCCGCGTCGTGCAGAGA [SEQ ID NO: 44]

the 5' end of RT (codon optimised and containing the W229K inactivating mutation) was PCR amplified from pt-rng (Figure 32 - see also WO 03/025003) using a 5' primer to insert a sequence homologous to 3'120, and a primer within RT

120RTf: GCCAAGCGCCGCGTCGTGCAGAGAATGGGCCCCATCAGTCCCATC [SEQ ID NO: 45]
RT3SR1: CGTCACGATGTTCACCTCCAGGCC [SEQ ID NO: 46]

The two products were PCR stitched using the end primers, and cut with NotI and NheI. The fragment was gel purified and used to replace the NotI -NheI fragment from pT-rng.

<u>pRix59 (Figure 22)</u>: the 3'Gag fragment was PCR amplified from pT-rng using a primer 5' to the MunI site in p24 and a 3' primer encoding the start of dsgp120, covering the position of the BstEII site near the 5' end:

<u>GagMunf</u>
GTGGCCCGAGAGCTGCATCCG [SEQ ID NO: 47]

GAG120R (Antisense to:)

Gag------------------ds120

GGACACAAAGCCAGAGTGTTGATGGCCGAGCAGCTGTGGGTCACCGTC

[SEQ ID NO: 48]

The product was cut with MunI and BstEII, and inserted into the 7113bp fragment from MunI-BstEII cut pRix54.

pRix48 and 49 (Figures 30 and 31)

The plasmids pRix 48 and 49 are equivalent to pRix39 and 41 except that they contain glycosylated gp120. To generate pRix48 and pRix49, the NotI - SalI fragments of pRix39 and pRix41 were replaced with the equivalent fragment from pRix32.

**Results**

Expression data is shown in Figures 33 and 35.

293T cell monolayers in 24 well plates were transfected with 1μg of each DNA indicated using Lipofectamine 2000 following the manufacturer's supplied protocol. After 24 hours the cells were detached and separated from the culture medium by centrifugation. Samples equivalent to $1x10^4$ cells or 12μl of medium were examined by PAGE and Western blot.

The gp 120c construct gave a highly glycosylated well secreted protein. Addition of c-terminal Nef/Tat fusions (pRix 6 and pRix11) resulted in a reduction of the intracellular protein levels and loss of secretion. Removal of the secretion signal from gp120c (pRix12) gave a non-glycosylated non-secreted form of the protein.

As expected, fusion constructs with no secretion signal pRix28, 29, 31, 33 and 35, made non-glycosylated intracellular proteins in similar amounts, though expression from pRix35 was somewhat reduced. Surprisingly, when the secretion signal was present in constructs pRix30, 32 and 34, only pRix34 failed to be secreted. It appears that the presence of Tat in the fusion inhibits secretion of the protein. The initial pRix32.1 construct had a point mutation resulting in poor expression. This was corrected in pRix32.7, which showed greatly improved expression.

For pRix40-47 the western blot in Figure 35 shows the expression of the dsgp120/Gag/Nef/Tat fusions with mutations in Nef in 293T cells 24hours post transfection with the plasmids indicated. Total cell extracts equivalent to $\sim 1x10^4$ cells were loaded onto the gel. The blot was probed with an anti-nef antiserum.

For pRix48-49 the western blot in Figure 35 shows the expression of the gp120/Gag/Nef/Tat fusions with glycosylated gp120 in 293T cells 24hours post transfection with the plasmids shown. Total cell extracts equivalent to $\sim 1x10^4$ cells were loaded onto the gel. The blot was probed with an anti-nef antiserum.

Similarly, expression data (anti-Nef) for the quadrivalent fusion proteins containing RT, Nef, Gag and dsgp120, compared to expression of the RT,Nef,Gag fusion alone, is shown in Figure 35.

**Refs:**

Andre S. Seed B. Eberle J. Schraut W. Bultmann A. Haas J. (1998) Increased immune response elicited by DNA vaccination with a synthetic gp120 sequence with optimized codon usage. Journal of Virology. 72(2):1497-503.

Vinner L. Nielsen HV. Bryder K. Corbet S. Nielsen C. Fomsgaard A. (1999) Gene gun DNA vaccination with Rev-independent synthetic HIV-1 gp160 envelope gene using mammalian codons. Vaccine. 17(17):2166-75

Collins KL. Baltimore D.(1999) HIV's evasion of the cellular immune response. Immunological Reviews. 168: 65-74,

**Example 5: <u>Preparation of plasmid-coated 'gold slurry' for 'gene gun' DNA cartridges</u>**

Plasmid DNA (approximately 1μg/μl), eg. 100 ug, and 2μm gold particles, eg. 50 mg, (PowderJect), were suspended in 0.05M spermidine, eg. 100 ul, (Sigma). The DNA was precipitated on to the gold particles by addition of 1M $CaCl_2$, eg. 100ul (American Pharmaceutical Partners, Inc., USA). The DNA/gold complex was incubated for 10 minutes at room temperature, washed 3 times in absolute ethanol, eg. 3 x 1 ml, (previously dried on molecular sieve 3A (BDH)). Samples were resuspended in absolute ethanol containing 0.05mg/ml of polyvinylpyrrolidone ( PVP, Sigma), and split into three equal aliquots in 1.5 ml microfuge tubes, (Eppendorf). The aliquots were for analysis of (a) 'gold slurry', (b) eluate- plasmid eluted from (a) and (c) for preparation of gold/ plasmid coated Tefzel cartridges for the 'gene gun', (see Example 3 below). For preparation of samples (a) and (b), the tubes containing plasmid DNA / 'gold slurry' in ethanol / PVP were spun for 2 minutes at top speed in an Eppendorf 5418 microfuge, the supernatant was removed and the 'gold slurry' dried for 10 minutes at room temperature. Sample (a) was resuspended to 0.5 - 1.0 ug / ul of plasmid DNA in TE pH 8.0, assuming approx. 50 % coating. For elution, sample (b) was resuspended to 0.5 -1.0 ug / ul of plasmid DNA in TE pH 8.0 and incubated at $37^0$C for 30 minutes, shaking vigorously, and then spun for 2 minutes at top speed in an Eppendorf 5418 microfuge and the supernatant, eluate, was removed and stored at - $20^0$C. The exact DNA concentration eluted was determined by spectrophotometric quantitation using a Genequant II (Pharmacia Biotech).

**Example 6: Preparation of Cartridges for DNA immunisation**

Preparation of cartridges for the Accell gene transfer device was as previously described (Eisenbraun et al DNA and Cell Biology, 1993 Vol 12 No 9 pp 791-797; Pertner et al). Briefly, plasmid DNA was coated onto 2 μm gold particles (DeGussa Corp., South Plainfield, N.J., USA) and loaded into Tefzel tubing, which was subsequently cut into 1.27 cm lengths to serve as cartridges and stored desiccated at 4°C until use. In a typical vaccination, each cartridge contained 0.5 mg gold coated with a total of 0.5 μg DNA/cartridge.

**Example 7: PMID immunisations including using gp120-Nef-Tat triple fusion lacking gp120 secretion signal**

**Protocol:** For PMID immunisations (DNA) cartridges were prepared for using standard methods as described in Examples 5 and 6. A DNA loading rate of 2, which will give approximately 0.5 μg DNA/cartridge was used and each immunisation consisted of two shots. Balb/c mice were given a primary immunisation of DNA (using PMID). The mice were boosted 28 days later with DNA (using PMID). Mice were culled 7 days later and serum and spleens were collected. The splenocytes were harvested by teasing out the spleen cells and erythrocytes were lysed. The splenocytes were washed and counted. Specialised ELIspot plates (coated with interferon-gamma capture antibody and blocked) were used. Splenocytes were transferred to these plates and incubated overnight at 37°C/5% $CO_2$ in the presence of a gp120 peptide, RT peptide or Gag peptide. The splenocytes were lysed and the plate developed using standard procedures to demonstrate the number of interferon-gamma secreting cells present. Serum was analysed by ELISA assay to detect for specific antibodies. Results are shown in Figures 36-38.

**Conclusion**

Unexpectedly, the cellular immune response of mice immunised with dsgp120 (gp 120 lacking secretion signal) expressing constructs was approximately double that of mice immunised with gp120 constructs (see Figure 36 and 37). This was consistent with the observation that in *in vitro* transfection studies the expression of dsgp120 had remained largely cell associated, whereas gp120 had been excreted.

Inclusion of Tat (mutated Tat) in the dsgp120 constructs increased the cellular immune response to twice that of the dsgp120 constucts without Tat (Figures 36 and 37). Tat on its own did not affect the immune response to gp120, but acted synergistically with dsgp120 to optimise the cellular response.

The inclusion of other HIV antigens in the constructs produced a balanced cellular response to all the different antigens included and thus broadened the immune response compared to the gp 120 only vectors (Figure 38).

## Claims

1. A polynucleotide which comprises a sequence encoding the HIV envelope protein gp120, wherein the gp120 is non-glycosylated when expressed in a mammalian target cell and wherein the gp120 lacks a functional secretion signal, linked to a sequence encoding HIV RT, HIV Gag, and HIV Nef, operably linked to a heterologous promoter, to encode a gp120, RT, Gag and Nef containing fusion protein.

2. The polynucleotide according to claim 1 wherein the fusion is selected from gp120-RT-Nef-Gag and RT-Nef-Gag-gp120.

3. The polynucleotide according to any one of claims 1 or 2 wherein the Gag comprises p17 and/or 24.

4. The polynucleotide according to any one of claims 1 to 3 wherein one or more of the sequences encoding gp120, Nef, Gag or RT is or are codon optimised to resemble the codon usage in a highly expressed human gene.

5. The polynucleotide according to any one of claims 1 to 4 wherein the promoter is the promoter from HCMV IE gene.

6. The polynucleotide according to claim 5 wherein the 5' untranslated region between the promoter and coding polynucleotide comprises exon 1 of the HCMV IE gene.

7. A vector comprising a polynucleotide as claimed in any one of claims 1 to 6.

8. The vector according to claim 7 which is a double-stranded DNA plasmid.

9. The vector according to claim 7 which is a replication defective adenovirus vector.

10. The vector according to claim 9 which is derived from Pan 9, 5, 6 or 7.

11. A fusion protein comprising the HIV envelope protein gp120, HIV RT, HIV Gag and HIV Nef, wherein the gp120 is non-glycosylated when expressed in a mammalian target cell and wherein the gp120 lacks a functional secretion signal.

12. A fusion protein according to claim 11 wherein the fusion is selected from gp120-RT-Nef-Gag and RT-Nef-Gag-gp120.

13. A pharmaceutical composition comprising a nucleotide sequence according to any one of claims 1 to 6, a vector of any one of claims 7 to 10, or a fusion protein or claim 11 or 12, and a pharmaceutically acceptable excipient, diluent, carrier or adjuvant.

14. The pharmaceutical composition according to claim 13 wherein the carrier is a plurality of particles such as gold beads.

15. The pharmaceutical composition according to claim 13 or 14 for delivery in a prime boost format.

16. An intradermal delivery device comprising a pharmaceutical composition according to any one of claims 13 to 15.

17. A polynucleotide or a vector or fusion protein according to any one of claims 1 to 12 for use in medicine.

18. Use of a polynucleotide or a vector or fusion protein according to any one of claims 1 to 12 in the manufacture of a medicament for the prophylaxis and treatment of HIV infection and AIDS.

19. A process for the production of a polynucleotide according to any one of claims 1 to 6 comprising linking a nucleotide sequence encoding HIV envelope protein gp120 sequence, wherein the gp120 is non-glycosylated when expressed in a mammalian target cell and wherein the gp120 lacks a functional secretion signal, and a sequence encoding HIV RT, HIV Gag, and HIV Nef, to a heterologous promoter sequence.

**Patentansprüche**

1. Polynukleotid, umfassend eine Sequenz, codierend für das HIV-Hüllprotein gp120, worin das gp120, wenn in einer Säuger-Zielzelle exprimiert, nicht-glycosyliert ist und worin dem gp120 ein funktionelles Sekretionssignal fehlt, verknüpft an eine Sequenz, codierend für HIV-RT, HIV-Gag und HIV-Nef, funktionell verknüpft an einen heterologen Promotor zum Codieren eines gp120, RT, Gag und Nef enthaltenden Fusionsproteins.

2. Polynukleotid gemäss Anspruch 1, worin die Fusion ausgewählt ist aus gp120-RT-Nef-Gag und RT-Nef-Gag-gp120.

3. Polynukleotid gemäss irgendeinem der Ansprüche 1 oder 2, worin das Gag p17 und/oder 24 umfasst.

4. Polynukleotid gemäss irgendeinem der Ansprüche 1 bis 3, worin eine oder mehrere der für gp120, Nef, Gag oder RT codierenden Sequenzen Kodon-optimiert ist oder sind, um der Kodonverwendung in einem stark exprimierten Human-Gen ähnlich zu sein.

5. Polynukleotid gemäss irgendeinem der Ansprüche 1 bis 4, worin der Promotor der Promotor des HCMV-IE-Gens ist.

6. Polynukleotid gemäss Anspruch 5, worin die 5'-untranslatierte Region zwischen dem Promotor und dem codierenden Polynukleotid exon 1 des HCMV-IE-Gens umfasst.

7. Vektor, umfassend ein Polynukleotid gemäss irgendeinem der Ansprüche 1 bis 6.

8. Vektor gemäss Anspruch 7, der ein doppelsträngiges DNA-Plasmid ist.

9. Vektor gemäss Anspruch 7, der ein Replikationsdefekter Adenovirus-Vektor ist.

10. Vektor gemäss Anspruch 9, der von Pan 9, 5, 6 oder 7 stammt.

11. Fusionsprotein, umfassend das HIV-Hüllprotein gp120, HIV-RT, HIV-Gag und HIV-Nef, worin das gp120, wenn in einer Säuger-Zielzelle exprimiert, nicht-glycosyliert ist und worin dem gp120 ein funktionelles Sekretionssignal fehlt.

12. Fusionsprotein gemäss Anspruch 11, worin die Fusion ausgewählt ist aus gp120-RT-Nef-Gag und RT-Nef-Gag-gp120.

13. Pharmazeutische Zusammensetzung, umfassend eine Nukleotidsequenz gemäss irgendeinem der Ansprüche 1 bis 6, einen Vektor gemäss irgendeinem der Ansprüche 7 bis 10 oder ein Fusionsprotein gemäss Anspruch 11 oder 12, und einen/ein pharmazeutisch annehmbaren/annehmbares Exzipienten, Verdünnungsstoff, Träger oder Adjuvans.

14. Pharmazeutische Zusammensetzung gemäss Anspruch 13, worin der Träger eine Vielzahl von Partikeln, wie Goldkügelchen, ist.

15. Pharmazeutische Zusammensetzung gemäss Anspruch 13 oder 14 zur Übertragung in einem Prime-Boost-Format.

16. Intradermale Übertragungsvorrichtung, umfassend eine pharmazeutische Zusammensetzung gemäss irgendeinem der Ansprüche 13 bis 15.

17. Polynukleotid oder Vektor oder Fusionsprotein gemäss irgendeinem der Ansprüche 1 bis 12 zur Verwendung in der Medizin.

18. Verwendung eines Polynukleotids oder eines Vektors oder Fusionsproteins gemäss irgendeinem der Ansprüche 1 bis 12 in der Herstellung eines Medikaments zur Prophylaxe oder Behandlung von HIV-Infektion und AIDS.

19. Verfahren zur Herstellung eines Polynukleotids gemäss irgendeinem der Ansprüche 1 bis 6, umfassend das Verknüpfen einer Nukleotidsequenz, codierend für die Sequenz des HIV-Hüllproteins gp120, worin das gp120, wenn in einer Säuger-Zielzelle exprimiert, nicht-glycosyliert ist und worin dem gp120 ein funktionelles Sekretionssignal fehlt, und einer Sequenz, codierend für HIV-RT, HIV-Gag und HIV-Nef, an eine heterologe Promotorsequenz.

## Revendications

1. Polynucléotide qui comprend une séquence codant la protéine d'enveloppe de VIH gp120, dans lequel la gp120 est non glycosylée quand elle est exprimée dans une cellule cible de mammifère et dans lequel la gp120 est dépourvue d'un signal de sécrétion fonctionnel, liée à une séquence codant RT de VIH, Gag de VIH, et Nef de VIH, liée de façon opérable à un promoteur hétérologue, pour coder une protéine de fusion contenant gp120, RT, Gag et Nef.

2. Polynucléotide selon la revendication 1, dans lequel la fusion est choisie parmi gp120-RT-Nef-Gag et RT-Nef-Gag-gp120.

3. Polynucléotide selon l'une quelconque des revendications 1 et 2, dans lequel Gag comprend p17 et/ou 24.

4. Polynucléotide selon l'une quelconque des revendications 1 à 3, dans lequel une ou plusieurs des séquences codant gp120, Nef, Gag ou RT est ou sont optimisées du point de vue des codons pour ressembler à l'usage de codons dans un gène humain fortement exprimé.

5. Polynucléotide selon l'une quelconque des revendications 1 à 4, dans lequel le promoteur est le promoteur provenant du gène HCMV IE.

6. Polynucléotide selon la revendication 5, dans lequel la région non traduite en 5' entre le promoteur et le polynucléotide codant comprend l'exon 1 du gène HCMV IE.

7. Vecteur comprenant un polynucléotide tel que revendiqué dans l'une quelconque des revendications 1 à 6.

8. Vecteur selon la revendication 7, qui est un ADN plasmidique double brin.

9. Vecteur selon la revendication 7, qui est un vecteur adénoviral à réplication défectueuse.

10. Vecteur selon la revendication 9, qui dérive de Pan 9, 5, 6 ou 7.

11. Protéine de fusion comprenant la protéine d'enveloppe de VIH gp120, RT de VIH, Gag de VIH et Nef de VIH, dans laquelle la gp120 est non glycosylée quand elle est exprimée dans une cellule cible de mammifère et dans laquelle la gp120 est dépourvue de signal de sécrétion fonctionnel.

12. Protéine de fusion selon la revendication 11, dans laquelle la fusion est choisie parmi gp120-RT-Nef-Gag et RT-Nef-Gag-gp120.

13. Composition pharmaceutique comprenant une séquence de nucléotides selon l'une quelconque des revendications 1 à 6, un vecteur selon l'une quelconque des revendications 7 à 10, ou une protéine de fusion selon la revendication 11 ou 12, et un excipient, diluant, véhicule ou adjuvant acceptable en pharmacie.

14. Composition pharmaceutique selon la revendication 13, dans laquelle le véhicule est constitué d'une pluralité de particules telles que des perles d'or.

15. Composition pharmaceutique selon la revendication 13 ou 14, pour une délivrance dans un format de sensibilisation-rappel.

16. Dispositif de délivrance intradermique comprenant une composition pharmaceutique selon l'une quelconque des revendications 13 à 15.

17. Polynucléotide ou vecteur ou protéine de fusion selon l'une quelconque des revendications 1 à 12, pour une utilisation en médecine.

18. Utilisation d'un polynucléotide ou d'un vecteur ou d'une protéine de fusion selon l'une quelconque des revendications 1 à 12, dans la fabrication d'un médicament destiné à la prophylaxie et au traitement d'une infection par VIH et du SIDA.

19. Procédé pour la production d'un polynucléotide selon l'une quelconque des revendications 1 à 6, comprenant la liaison d'une séquence de nucléotides codant la séquence de la protéine d'enveloppe de VIH gp120, dans laquelle la gp120 est non glycosylée quand elle est exprimée dans une cellule cible de mammifère et dans laquelle la gp120 est dépourvue de signal de sécrétion fonctionnel, et d'une séquence codant RT de VIH, Gag de VIH, et Nef de VIH, à une séquence de promoteur hétérologue.

# Fig.1.

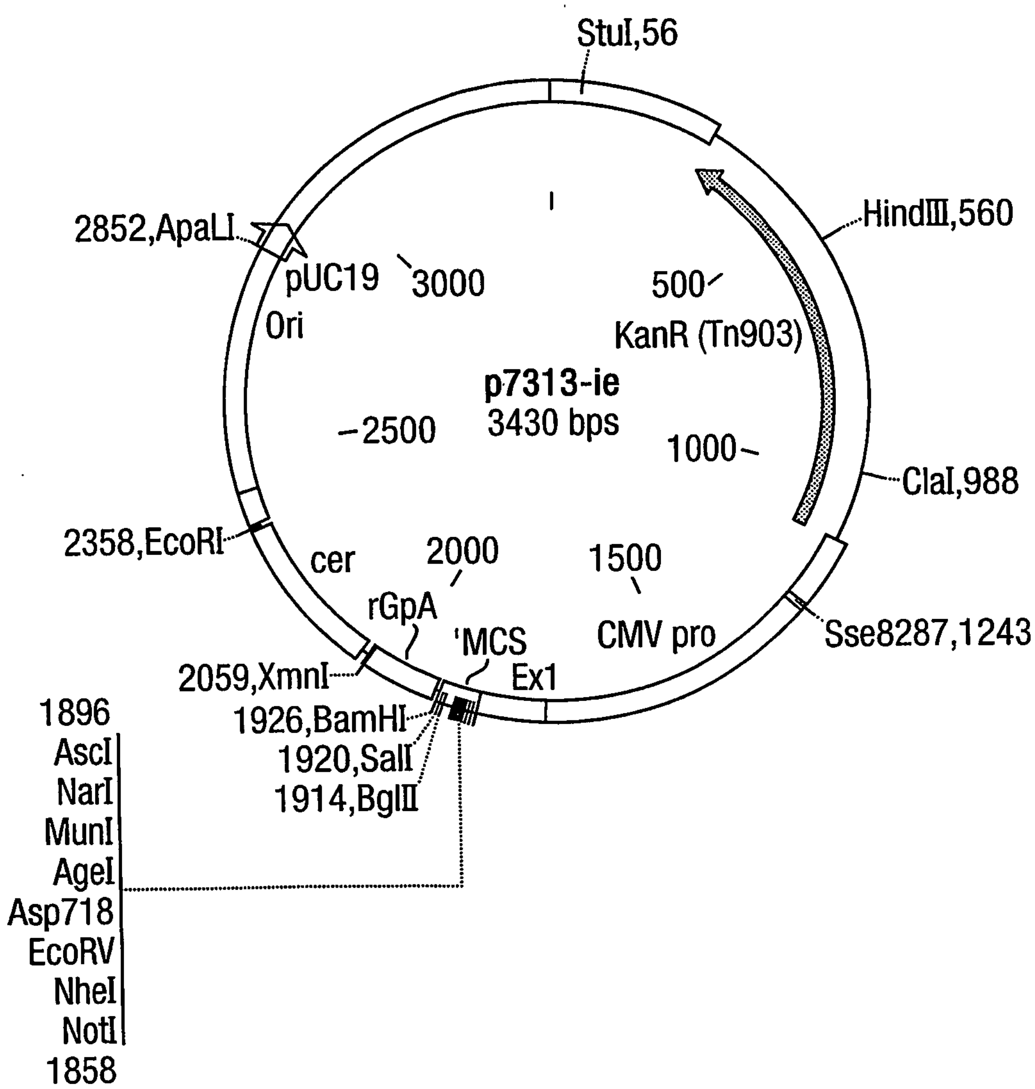
StuI,56
HindIII,560
ClaI,988
Sse8287,1243
2852,ApaLI
pUC19
Ori
3000
500
KanR (Tn903)
p7313-ie
3430 bps
2500
1000
2358,EcoRI
cer
2000
1500
rGpA
'MCS
CMV pro
Ex1
2059,XmnI
1926,BamHI
1920,SalI
1914,BglII
1896
AscI
NarI
MunI
AgeI
Asp718
EcoRV
NheI
NotI
1858

# Fig.2.

Map of pgp 120c:

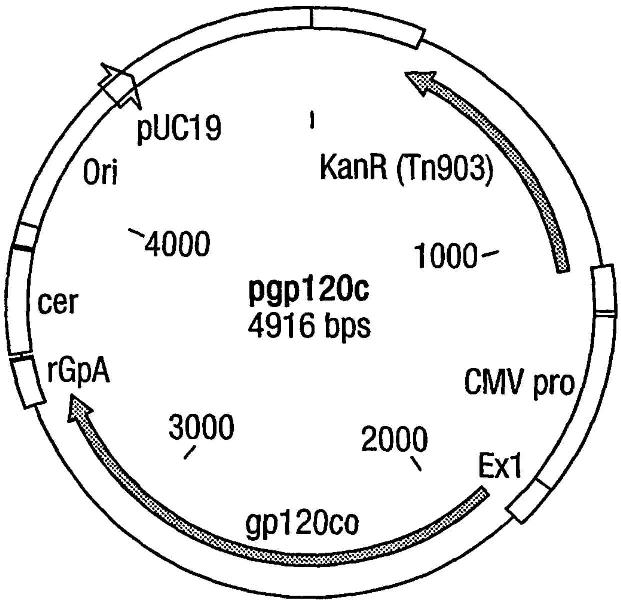


The amino acid sequence of the W61D gp120 is below. The signal sequence is underlined and bold, up to the predicted cleavage site between amino acids 29 and 30. This is the sequence removed in dsgp120 (pRix12 etc).

M**KVKETRKNYQHLWRWGTMLLGMLMICSA**AEQLWVTVYYGVPVWKEATTTLFCASDAKAYDTEVHNVWATH
ACVPTDPNPQEVVLGNVTEYFNMWKNNMVDQMHEDIISLWDQSLKPCVKLTPLCVTLDCDDVNTTNSTTTT
SNGWTGEIRKGEIKNCSFNITTSIRDKVQKEYALFYNLDVVPIDDDNATTKNKTTRNFRLIHCNSSVMTQA
CPKVSFEPIPIHYCAPAGFAILKCNNKTFDGKGLCTNVSTVQCTHGIRPVVSTQLLLNGSLAEEEVVIRSD
NFMDNTKTIIVQLNESVAINCTRPNNNTRKGIHIGPGRAFYAARKIIGDIRQAHCNLSRAQWNNTLKQIVI
KLREHFGNKTIKFNQSSGGDPEIVRHSFNCGGEFFYCDTTQLFNSTWNGTEGNNTEGNSTITLPCRIKQII
NMWQEVGKAMYAPPIGGQIRCSSNITGLLLTRDGGTEGNGTENETEIFRPGGGDMRDNWRSELYKYKVVKV
EPLGVAPTRAKRRVVQR [SEQ ID NO: 49]

The codon optimised DNA sequence for the W61D gp120 gene is:

ATGAAGGTCAAGGAGACCAGAAAGAACTACCAGCATCTGTGGCGCTGGGGCACCATGCTCCTGGGAATGCT
GATGATCTGCTCCGCCGCCGAGCAGCTGTGGGTCACCGTCTACTACGGCGTGCCTGTGTGGAAGGAGGCCA
CGACCACCCTCTTCTGCGCGAGCGACGCCAAGGCCTACGACACGGAAGTGCATAACGTGTGGGCGACGCAT
GCTTGCGTGCCTACGGACCCCAACCCCCAGGAGGTGGTGCTGGGAAACGTGACCGAGTACTTCAACATGTG
GAAGAATAACATGGTGGATCAGATGCACGAGGACATCATCTCTCTGTGGGACCAGTCCCTGAAGCCCTGCG
TGAAGCTGACGCCTCTCTGCGTGACACTGGACTGTGACGACGTCAACACCACCAACAGCACTACCACCACC
AGCAACGGCTGGACCGGAGAGATTCGGAAGGGCGAGATCAAGAACTGCTCCTTCAATATCACGACCTCGAT
CAGAGACAAGGTGCAGAAGGAATACGCGCTGTTTTATAATCTCGATGTGGTCCCCATCGACGACGACAATG
CCACCACCAAGAACAAGACGACGCGTAATTTCAGACTCATTCACTGCAACAGCAGCGTCATGACGCAGGCC
TGCCCCAAGGTGTCCTTCGAACCAATCCCGATCCATTACTGTGCCCCTGCCGGATTCGCGATCCTCAAGTG
TAACAACAAGACCTTCGACGGGAAGGGCCTGTGCACCAACGTCAGCACGGTGCAGTGCACCCATGGCATCC

# Fig.2 (Cont).

GCCCCGTCGTGAGCACCCAGCTGCTGCTGAACGGGTCCCTGGCTGAGGAGGAGGTGGTGATCCGGTCGGAC
AACTTCATGGACAACACCAAGACAATCATCGTCCAGCTGAACGAGTCTGTGGCGATTAACTGTACCCGGCC
TAACAACAACACCCGTAAGGGCATCCACATCGGGCCTGGACGGGCCTTCTATGCCGCCCGCAAGATCATCG
GCGACATCCGGCAGGCCCATTGCAACCTCTCCCGCGCCCAGTGGAATAACACCCTGAAGCAGATCGTGATC
AAGCTGAGAGAGCACTTTGGAAACAAGACCATCAAGTTCAATCAGAGTTCTGGCGGAGACCCCGAGATCGT
GCGGCACTCCTTCAACTGCGGGGGCGAGTTCTTCTACTGCGATACGACACAGCTCTTCAACTCCACCTGGA
ACGGCACCGAGGGCAACAACACAGAGGGAAACTCCACTATCACCCTCCCTTGCCGCATCAAGCAGATCATC
AACATGTGGCAGGAGGTGGGAAAGGCCATGTATGCCCCCCCCATCGGGGGCCAGATCCGCTGCTCCTCCAA
CATCACCGGCCTGCTGCTCACCAGAGACGGGGGCACCGAGGGCAACGGCACGGAGAACGAGACGGAGATCT
TCAGGCCCGGCGGCGGCGACATGAGGGATAACTGGCGGAGCGAGCTGTACAAGTACAAGGTGGTGAAGGTG
GAGCCGCTCGGCGTGGCCCCCACCCGGGCCAAGCGCCGCGTCGTGCAGAGATGA [SEQ ID NO: 50]

# Fig.3.

Map of pRix15244:

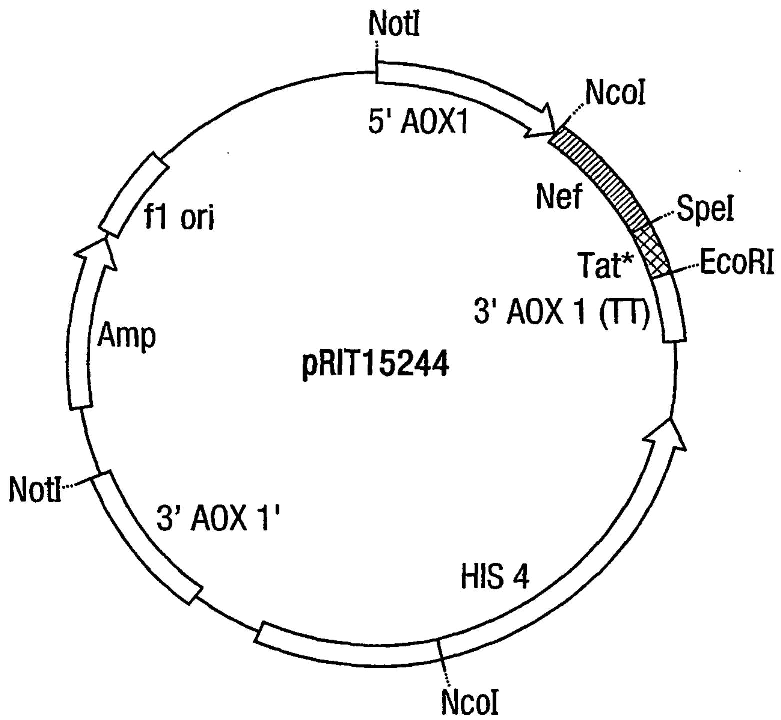

# Fig.4.

Plasmid pNTm:

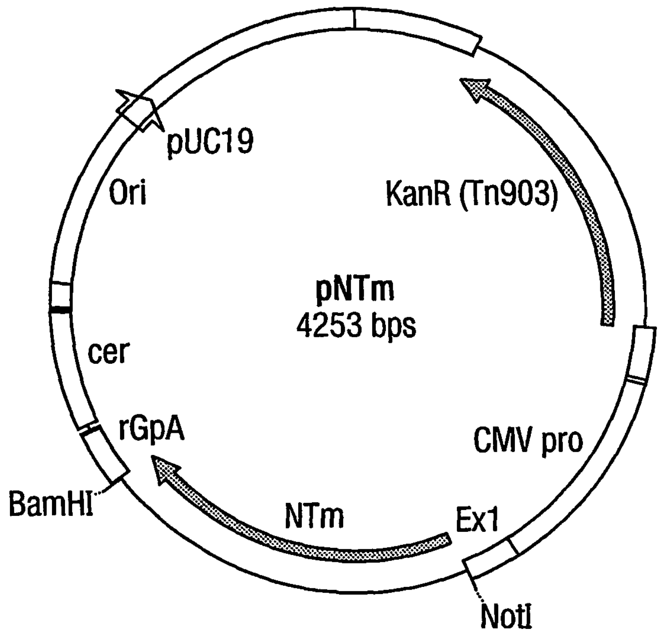


Sequence of insert:

ATGGGTGGCAAGTGGTCAAAAAGTAGTGTGGTTGGATGGCCTACTGTAAGGGAAAGAATGAGACGAGCTGA
GCCAGCAGCAGATGGGGTGGGAGCAGCATCTCGAGACCTGGAAAAACATGGAGCAATCACAAGTAGCAATA
CAGCAGCTACCAATGCTGCTTGTGCCTGGCTAGAAGCACAAGAGGAGGAGGAGGTGGGTTTTCCAGTCACA
CCTCAGGTACCTTTAAGACCAATGACTTACAAGGCAGCTGTAGATCTTAGCCACTTTTTAAAAGAAAAGGG
GGGACTGGAAGGGCTAATTCACTCCCAACGAAGACAAGATATCCTTGATCTGTGGATCTACCACACACAAG
GCTACTTCCCTGATTGGCAGAACTACACACCAGGGCCAGGGGTCAGATATCCACTGACCTTTGGATGGTGC
TACAAGCTAGTACCAGTTGAGCCAGATAAGGTAGAAGAGGCCAATAAAGGAGAGAACACCAGCTTGTTACA
CCCTGTGAGCCTGCATGGAATGGATGACCCTGAGAGAGAAGTGTTAGAGTGGAGGTTTGACAGCCGCCTAG
CATTTCATCACGTGGCCCGAGAGCTGCATCCGGAGTACTTCAAGAACTGCACTAGTGAGCCAGTAGATCCT
AGACTAGAGCCCTGGAAGCATCCAGGAAGTCAGCCTAAAACTGCTTGTACCAATTGCTATTGTAAAAAGTG
TTGCTTTCATTGCCAAGTTTGTTTCATAACAGCTGCCTTAGGCATCTCCTATGGCAGGAAGAAGCGGAGAC
AGCGACGAAGACCTCCTCAAGGCAGTCAGACTCATCAAGTTTCTCTATCAAAGCAACCCACCTCCCAATCC
AAAGGGGAGCCGACAGGCCCGAAGGAATAA [SEQ ID NO: 51]

Amino acid sequence of antigen:

MGGKWSKSSVVGWPTVRERMRRAEPAADGVGAASRDLEKHGAITSSNTAATNAACAWLEAQEE
EEVGFPVTPQVPLRPMTYKAAVDLSHFLKEKGGLEGLIHSQRRQDILDLWIYHTQGYFPDWQNYT
PGPGVRYPLTFGWCYKLVPVEPDKVEEANKGENTSLLHPVSLHGMDDPEREVLEWRFDSRLAFH
HVARELHPEYFKNCTSEPVDPRLEPWKHPGSQPKTACTNCYCKKCCFHCQVCFITAALGISYGRK
KRRQRRRPPQGSQTHQVSLSKQPTSQSKGEPTGPKE [SEQ ID NO: 52]

# Fig.5.

Plasmid ptrNTm:

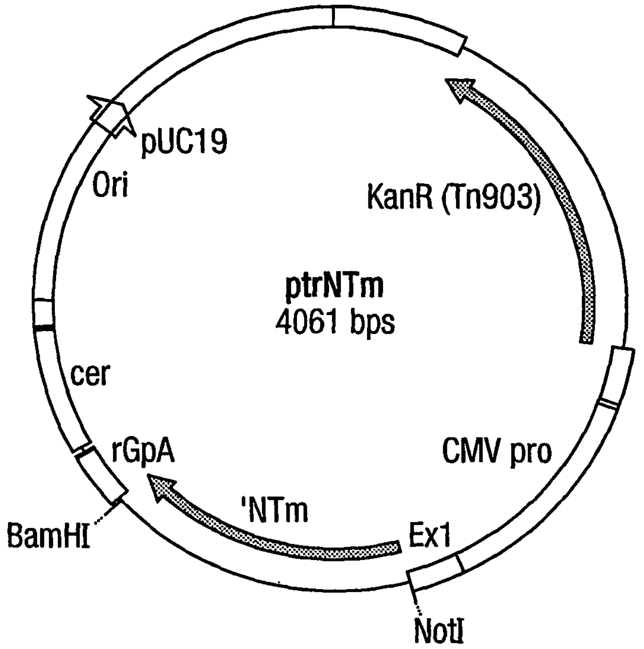


Sequence of insert:

ATGGTGGGTTTTCCAGTCACACCTCAGGTACCTTTAAGACCAATGACTTACAAGGCAGCTGTAGATCTTAG
CCACTTTTTAAAAGAAAAGGGGGACTGGAAGGGCTAATTCACTCCCAACGAAGACAAGATATCCTTGATC
TGTGGATCTACCACACACAAGGCTACTTCCCTGATTGGCAGAACTACACACCAGGGCCAGGGGTCAGATAT
CCACTGACCTTTGGATGGTGCTACAAGCTAGTACCAGTTGAGCCAGATAAGGTAGAAGAGGCCAATAAAGG
AGAGAACACCAGCTTGTTACACCCTGTGAGCCTGCATGGAATGGATGACCCTGAGAGAGAAGTGTTAGAGT
GGAGGTTTGACAGCCGCCTAGCATTTCATCACGTGGCCCGAGAGCTGCATCCGGAGTACTTCAAGAACTGC
ACTAGTGAGCCAGTAGATCCTAGACTAGAGCCCTGGAAGCATCCAGGAAGTCAGCCTAAAACTGCTTGTAC
CAATTGCTATTGTAAAAAGTGTTGCTTTCATTGCCAAGTTTGTTTCATAACAGCTGCCTTAGGCATCTCCT
ATGGCAGGAAGAAGCGGAGACAGCGACGAAGACCTCCTCAAGGCAGTCAGACTCATCAAGTTTCTCTATCA
AAGCAACCCACCTCCCAATCCAAAGGGGAGCCGACAGGCCCGAAGGAATAA [SEQ ID NO: 53]

Amino acid sequence of antigen:

MVGFPVTPQVPLRPMTYKAAVDLSHFLKEKGGLEGLIHSQRRQDILDLWIYHTQGYFPDWQNYTPGPGVRY
PLTFGWCYKLVPVEPDKVEEANKGENTSLLHPVSLHGMDDPEREVLEWRFDSRLAFHHVARELHPEYFKNC
TSEPVDPRLEPWKHPGSQPKTACTNCYCKKCCFHCQVCFITAALGISYGRKKRRQRRRPPQGSQTHQVSLS
KQPTSQSKGEPTGPKE [SEQ ID NO: 54]

# Fig.6.

Plasmid pRix12:

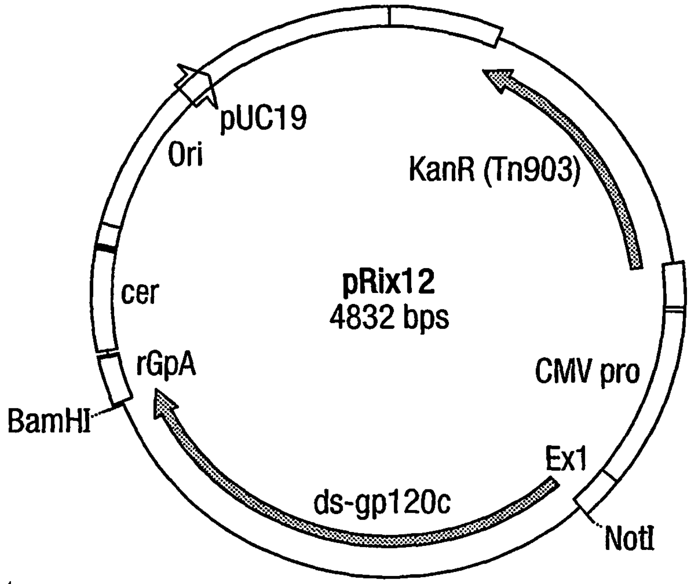


Sequence of insert:

ATGGCCGAGCAGCTGTGGGTCACCGTCTACTACGGCGTGCCTGTGTGGAAGGAGGCCACGACCACCCTCTT
CTGCGCGAGCGACGCCAAGGCCTACGACACGGAAGTGCATAACGTGTGGGCGACGCATGCTTGCGTGCCTA
CGGACCCCAACCCCCAGGAGGTGGTGCTGGGAAACGTGACCGAGTACTTCAACATGTGGAAGAATAACATG
GTGGATCAGATGCACGAGGACATCATCTCTCTGTGGGACCAGTCCCTGAAGCCCTGCGTGAAGCTGACGCC
TCTCTGCGTGACACTGGACTGTGACGACGTCAACACCACCAACAGCACTACCACCACCAGCAACGGCTGGA
CCGGAGAGATTCGGAAGGGCGAGATCAAGAACTGCTCCTTCAATATCACGACCTCGATCAGAGACAAGGTG
CAGAAGGAATACGCGCTGTTTTATAATCTCGATGTGGTCCCCATCGACGACGACAATGCCACCACCAAGAA
CAAGACGACGCGTAATTTCAGACTCATTCACTGCAACAGCAGCGTCATGACGCAGGCCTGCCCCAAGGTGT
CCTTCGAACCAATCCCGATCCATTACTGTGCCCCTGCCGGATTCGCGATCCTCAAGTGTAACAACAAGACC
TTCGACGGGAAGGGCCTGTGCACCAACGTCAGCACGGTGCAGTGCACCCATGGCATCCGCCCCGTCGTGAG
CACCCAGCTGCTGCTGAACGGGTCCCTGGCTGAGGAGGAGGTGGTGATCCGGTCGGACAACTTCATGGACA
ACACCAAGACAATCATCGTCCAGCTGAACGAGTCTGTGGCGATTAACTGTACCCGGCCTAACAACAACACC
CGTAAGGGCATCCACATCGGGCCTGGACGGGCCTTCTATGCCGCCCGCAAGATCATCGGCGACATCCGGCA
GGCCCATTGCAACCTCTCCCGCGCCCAGTGGAATAACACCCTGAAGCAGATCGTGATCAAGCTGAGAGAGC
ACTTTGGAAACAAGACCATCAAGTTCAATCAGAGTTCTGGCGGAGACCCCGAGATCGTGCGGCACTCCTTC
AACTGCGGGGGCGAGTTCTTCTACTGCGATACGACACAGCTCTTCAACTCCACCTGGAACGGCACCGAGGG
CAACAACACAGAGGGAAACTCCACTATCACCCTCCCTTGCCGCATCAAGCAGATCATCAACATGTGGCAGG
AGGTGGGAAAGGCCATGTATGCCCCCCCCATCGGGGGCCAGATCCGCTGCTCCTCCAACATCACCGGCCTG
CTGCTCACCAGAGACGGGGGCACCGAGGGCAACGGCACGGAGAACGAGACGGAGATCTTCAGGCCCGGCGG
CGGCGACATGAGGGATAACTGGCGGAGCGAGCTGTACAAGTACAAGGTGGTGAAGGTGGAGCCGCTCGGCG
TGGCCCCCACCCGGGCCAAGCGCCGCGTCGTGCAGAGATGA [SEQ ID NO: 55]

Amino acid sequence of antigen:

MAEQLWVTVYYGVPVWKEATTTLFCASDAKAYDTEVHNVWATHACVPTDPNPQEVVLGNVTEYFNMWKNNM
VDQMHEDIISLWDQSLKPCVKLTPLCVTLDCDDVNTTNSTTTTSNGWTGEIRKGEIKNCSFNITTSIRDKV
QKEYALFYNLDVVPIDDDNATTKNKTTRNFRLIHCNSSVMTQACPKVSFEPIPIHYCAPAGFAILKCNNKT
FDGKGLCTNVSTVQCTHGIRPVVSTQLLLNGSLAEEEVVIRSDNFMDNTKTIIVQLNESVAINCTRPNNNT
RKGIHIGPGRAFYAARKIIGDIRQAHCNLSRAQWNNTLKQIVIKLREHFGNKTIKFNQSSGGDPEIVRHSF
NCGGEFFYCDTTQLFNSTWNGTEGNNTEGNSTITLPCRIKQIINMWQEVGKAMYAPPIGGQIRCSSNITGL
LLTRDGGTEGNGTENETEIFRPGGGDMRDNWRSELYKYKVVKVEPLGVAPTRAKRRVVQR [SEQ ID NO: 56]

# Fig.7.

Plasmid pRix28:

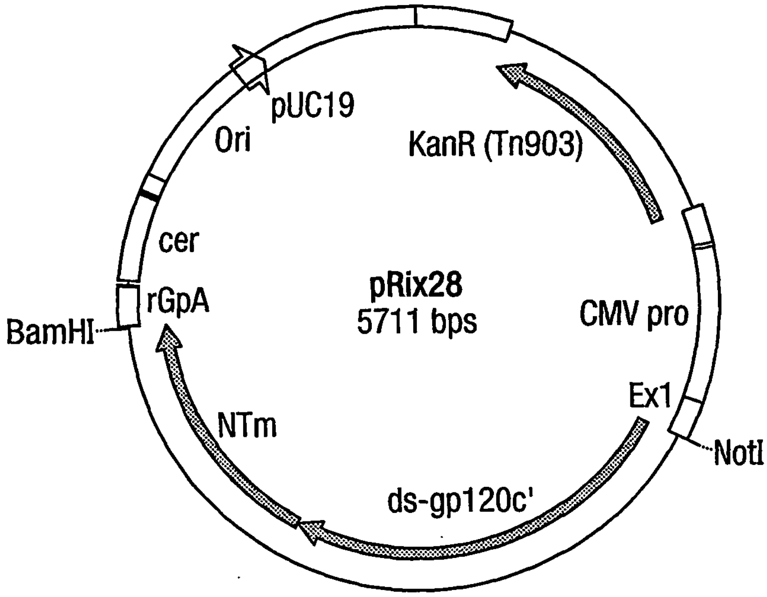


Sequence of insert:

ATGGCCGAGCAGCTGTGGGTCACCGTCTACTACGGCGTGCCTGTGTGGAAGGAGGCCACGACCACCCTCTT
CTGCGCGAGCGACGCCAAGGCCTACGACACGGAAGTGCATAACGTGTGGGCGACGCATGCTTGCGTGCCTA
CGGACCCCAACCCCCAGGAGGTGGTGCTGGGAAACGTGACCGAGTACTTCAACATGTGGAAGAATAACATG
GTGGATCAGATGCACGAGGACATCATCTCTCTGTGGGACCAGTCCCTGAAGCCCTGCGTGAAGCTGACGCC
TCTCTGCGTGACACTGGACTGTGACGACGTCAACACCACCAACAGCACTACCACCACCAGCAACGGCTGGA
CCGGAGAGATTCGGAAGGGCGAGATCAAGAACTGCTCCTTCAATATCACGACCTCGATCAGAGACAAGGTG
CAGAAGGAATACGCGCTGTTTTATAATCTCGATGTGGTCCCCATCGACGACGACAATGCCACCACCAAGAA
CAAGACGACGCGTAATTTCAGACTCATTCACTGCAACAGCAGCGTCATGACGCAGGCCTGCCCCAAGGTGT
CCTTCGAACCAATCCCGATCCATTACTGTGCCCCTGCCGGATTCGCGATCCTCAAGTGTAACAACAAGACC
TTCGACGGGAAGGGCCTGTGCACCAACGTCAGCACGGTGCAGTGCACCCATGGCATCCGCCCCGTCGTGAG
CACCCAGCTGCTGCTGAACGGGTCCCTGGCTGAGGAGGAGGTGGTGATCCGGTCGGACAACTTCATGGACA
ACACCAAGACAATCATCGTCCAGCTGAACGAGTCTGTGGCGATTAACTGTACCCGGCCTAACAACAACACC
CGTAAGGGCATCCACATCGGGCCTGGACGGGCCTTCTATGCCGCCCGCAAGATCATCGGCGACATCCGGCA
GGCCCATTGCAACCTCTCCCGCGCCCAGTGGAATAACACCCTGAAGCAGATCGTGATCAAGCTGAGAGAGC
ACTTTGGAAACAAGACCATCAAGTTCAATCAGAGTTCTGGCGGAGACCCCGAGATCGTGCGGCACTCCTTC
AACTGCGGGGGCGAGTTCTTCTACTGCGATACGACACAGCTCTTCAACTCCACCTGGAACGGCACCGAGGG
CAACAACACAGAGGGAAACTCCACTATCACCCTCCCTTGCCGCATCAAGCAGATCATCAACATGTGGCAGG
AGGTGGGAAAGGCCATGTATGCCCCCCCCATCGGGGGCCAGATCCGCTGCTCCTCCAACATCACCGGCCTG
CTGCTCACCAGAGACGGGGGCACCGAGGGCAACGGCACGGAGAACGAGACGGAGATCTTCAGGCCCGGCGG
CGGCGACATGAGGGATAACTGGCGGAGCGAGCTGTACAAGTACAAGGTGGTGAAGGTGGAGCCGCTCGGCG
TGGCCCCCACCCGGGCCAAGCGCCGCGTCGTGCAGAGAATGGGTGGCAAGTGGTCAAAAAGTAGTGTGGTT
GGATGGCCTACTGTAAGGGAAAGAATGAGACGAGCTGAGCCAGCAGCAGATGGGGTGGGAGCAGCATCTCG
AGACCTGGAAAAACATGGAGCAATCACAAGTAGCAATACAGCAGCTACCAATGCTGCTTGTGCCTGGCTAG
AAGCACAAGAGGAGGAGGAGGTGGGTTTTCCAGTCACACCTCAGGTACCTTTAAGACCAATGACTTACAAG
GCAGCTGTAGATCTTAGCCACTTTTTAAAAGAAAAGGGGGGACTGGAAGGGCTAATTCACTCCCAACGAAG
ACAAGATATCCTTGATCTGTGGATCTACCACACACAAGGCTACTTCCCTGATTGGCAGAACTACACACCAG
GGCCAGGGGTCAGATATCCACTGACCTTTGGATGGTGCTACAAGCTAGTACCAGTTGAGCCAGATAAGGTA
GAAGAGGCCAATAAAGGAGAGAACACCAGCTTGTTACACCCTGTGAGCCTGCATGGAATGGATGACCCTGA
GAGAGAAGTGTTAGAGTGGAGGTTTGACAGCCGCCTAGCATTTCATCACGTGGCCCGAGAGCTGCATCCGG
AGTACTTCAAGAACTGCACTAGTGAGCCAGTAGATCCTAGACTAGAGCCCTGGAAGCATCCAGGAAGTCAG

# Fig.7 (Cont).

CCTAAAACTGCTTGTACCAATTGCTATTGTAAAAAGTGTTGCTTTCATTGCCAAGTTTGTTTCATAACAGC
TGCCTTAGGCATCTCCTATGGCAGGAAGAAGCGGAGACAGCGACGAAGACCTCCTCAAGGCAGTCAGACTC
ATCAAGTTTCTCTATCAAAGCAACCCACCTCCCAATCCAAAGGGGAGCCGACAGGCCCGAAGGAATAA

[SEQ ID NO: 57]

**Amino acid sequence of antigen:**

MAEQLWVTVYYGVPVWKEATTTLFCASDAKAYDTEVHNVWATHACVPTDPNPQEVVLGNVTEYFNMWKNNM
VDQMHEDIISLWDQSLKPCVKLTPLCVTLDCDDVNTTNSTTTTSNGWTGEIRKGEIKNCSFNITTSIRDKV
QKEYALFYNLDVVPIDDDNATTKNKTTRNFRLIHCNSSVMTQACPKVSFEPIPIHYCAPAGFAILKCNNKT
FDGKGLCTNVSTVQCTHGIRPVVSTQLLLNGSLAEEEVVIRSDNFMDNTKTIIVQLNESVAINCTRPNNNT
RKGIHIGPGRAFYAARKIIGDIRQAHCNLSRAQWNNTLKQIVIKLREHFGNKTIKFNQSSGGDPEIVRHSF
NCGGEFFYCDTTQLFNSTWNGTEGNNTEGNSTITLPCRIKQIINMWQEVGKAMYAPPIGGQIRCSSNITGL
LLTRDGGTEGNGTENETEIFRPGGGDMRDNWRSELYKYKVVKVEPLGVAPTRAKRRVVQRMGGKWSKSSVV
GWPTVRERMRRAEPAADGVGAASRDLEKHGAITSSNTAATNAACAWLEAQEEEEVGFPVTPQVPLRPMTYK
AAVDLSHFLKEKGGLEGLIHSQRRQDILDLWIYHTQGYFPDWQNYTPGPGVRYPLTFGWCYKLVPVEPDKV
EEANKGENTSLLHPVSLHGMDDPEREVLEWRFDSRLAFHHVARELHPEYFKNCTSEPVDPRLEPWKHPGSQ
PKTACTNCYCKKCCFHCQVCFITAALGISYGRKKRRQRRRPPQGSQTHQVSLSKQPTSQSKGEPTGPKE
[SEQ ID NO: 58]

# Fig.8.

Plasmid pRix29:

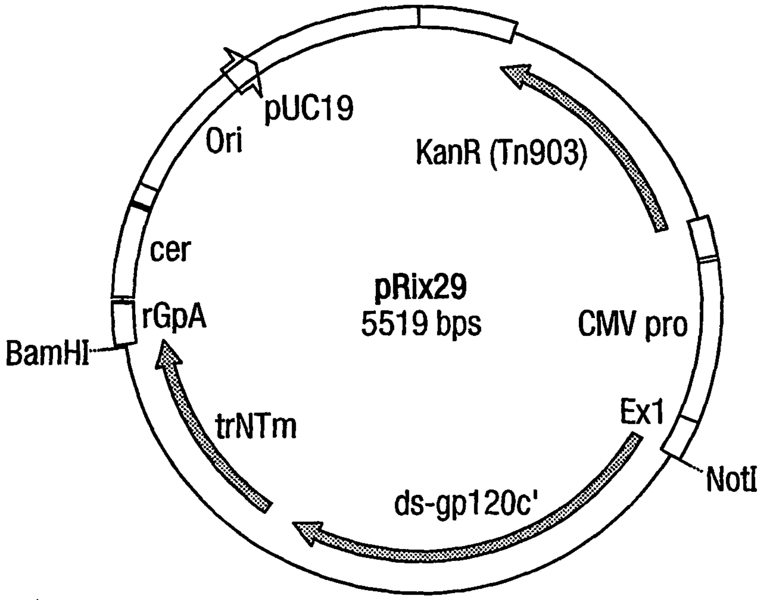


Sequence of insert:

ATGGCCGAGCAGCTGTGGGTCACCGTCTACTACGGCGTGCCTGTGTGGAAGGAGGCCACGACCACCCTCTT
CTGCGCGAGCGACGCCAAGGCCTACGACACGGAAGTGCATAACGTGTGGGCGACGCATGCTTGCGTGCCTA
CGGACCCCAACCCCCAGGAGGTGGTGCTGGGAAACGTGACCGAGTACTTCAACATGTGGAAGAATAACATG
GTGGATCAGATGCACGAGGACATCATCTCTCTGTGGGACCAGTCCCTGAAGCCCTGCGTGAAGCTGACGCC
TCTCTGCGTGACACTGGACTGTGACGACGTCAACACCACCAACAGCACTACCACCACCAGCAACGGCTGGA
CCGGAGAGATTCGGAAGGGCGAGATCAAGAACTGCTCCTTCAATATCACGACCTCGATCAGAGACAAGGTG
CAGAAGGAATACGCGCTGTTTTATAATCTCGATGTGGTCCCCATCGACGACGACAATGCCACCACCAAGAA
CAAGACGACGCGTAATTTCAGACTCATTCACTGCAACAGCAGCGTCATGACGCAGGCCTGCCCCAAGGTGT
CCTTCGAACCAATCCCGATCCATTACTGTGCCCCTGCCGGATTCGCGATCCTCAAGTGTAACAACAAGACC
TTCGACGGGAAGGGCCTGTGCACCAACGTCAGCACGGTGCAGTGCACCCATGGCATCCGCCCCGTCGTGAG
CACCCAGCTGCTGCTGAACGGGTCCCTGGCTGAGGAGGAGGTGGTGATCCGGTCGGACAACTTCATGGACA
ACACCAAGACAATCATCGTCCAGCTGAACGAGTCTGTGGCGATTAACTGTACCCGGCCTAACAACAACACC
CGTAAGGGCATCCACATCGGGCCTGGACGGGCCTTCTATGCCGCCCGCAAGATCATCGGCGACATCCGGCA
GGCCCATTGCAACCTCTCCCGCGCCCAGTGGAATAACACCCTGAAGCAGATCGTGATCAAGCTGAGAGAGC
ACTTTGGAAACAAGACCATCAAGTTCAATCAGAGTTCTGGCGGAGACCCCGAGATCGTGCGGCACTCCTTC
AACTGCGGGGGCGAGTTCTTCTACTGCGATACGACACAGCTCTTCAACTCCACCTGGAACGGCACCGAGGG
CAACAACACAGAGGGAAACTCCACTATCACCCTCCCTTGCCGCATCAAGCAGATCATCAACATGTGGCAGG
AGGTGGGAAAGGCCATGTATGCCCCCCCCATCGGGGGCCAGATCCGCTGCTCCTCCAACATCACCGGCCTG
CTGCTCACCAGAGACGGGGGCACCGAGGGCAACGGCACGGAGAACGAGACGGAGATCTTCAGGCCCGGCGG
CGGCGACATGAGGGATAACTGGCGGAGCGAGCTGTACAAGTACAAGGTGGTGAAGGTGGAGCCGCTCGGCG
TGGCCCCCACCCGGGCCAAGCGCCGCGTCGTGCAGAGAATGGTGGGTTTTCCAGTCACACCTCAGGTACCT
TTAAGACCAATGACTTACAAGGCAGCTGTAGATCTTAGCCACTTTTTAAAAGAAAAGGGGGGACTGGAAGG
GCTAATTCACTCCCAACGAAGACAAGATATCCTTGATCTGTGGATCTACCACACACAAGGCTACTTCCCTG
ATTGGCAGAACTACACACCAGGGCCAGGGGTCAGATATCCACTGACCTTTGGATGGTGCTACAAGCTAGTA
CCAGTTGAGCCAGATAAGGTAGAAGAGGCCAATAAAGGAGAGAACACCAGCTTGTTACACCCTGTGAGCCT
GCATGGAATGGATGACCCTGAGAGAGAAGTGTTAGAGTGGAGGTTTGACAGCCGCCTAGCATTTCATCACG
TGGCCCGAGAGCTGCATCCGGAGTACTTCAAGAACTGCACTAGTGAGCCAGTAGATCCTAGACTAGAGCCC
TGGAAGCATCCAGGAAGTCAGCCTAAAACTGCTTGTACCAATTGCTATTGTAAAAAGTGTTGCTTTCATTG
CCAAGTTTGTTTCATAACAGCTGCCTTAGGCATCTCCTATGGCAGGAAGAAGCGGAGACAGCGACGAAGAC
CTCCTCAAGGCAGTCAGACTCATCAAGTTTCTCTATCAAAGCAACCCACCTCCCAATCCAAAGGGGAGCCG
ACAGGCCCGAAGGAATAA [SEQ ID NO: 59]

# Fig.8 (Cont).

**Amino acid sequence of antigen:**

MAEQLWVTVYYGVPVWKEATTTLFCASDAKAYDTEVHNVWATHACVPTDPNPQEVVLGNVTEYFNMWKNNM
VDQMHEDIISLWDQSLKPCVKLTPLCVTLDCDDVNTTNSTTTTSNGWTGEIRKGEIKNCSFNITTSIRDKV
QKEYALFYNLDVVPIDDDNATTKNKTTRNFRLIHCNSSVMTQACPKVSFEPIPIHYCAPAGFAILKCNNKT
FDGKGLCTNVSTVQCTHGIRPVVSTQLLLNGSLAEEEVVIRSDNFMDNTKTIIVQLNESVAINCTRPNNNT
RKGIHIGPGRAFYAARKIIGDIRQAHCNLSRAQWNNTLKQIVIKLREHFGNKTIKFNQSSGGDPEIVRHSF
NCGGEFFYCDTTQLFNSTWNGTEGNNTEGNSTITLPCRIKQIINMWQEVGKAMYAPPIGGQIRCSSNITGL
LLTRDGGTEGNGTENETEIFRPGGGDMRDNWRSELYKYKVVKVEPLGVAPTRAKRRVVQRMVGFPVTPQVP
LRPMTYKAAVDLSHFLKEKGGLEGLIHSQRRQDILDLWIYHTQGYFPDWQNYTPGPGVRYPLTFGWCYKLV
PVEPDKVEEANKGENTSLLHPVSLHGMDDPEREVLEWRFDSRLAFHHVARELHPEYFKNCTSEPVDPRLEP
WKHPGSQPKTACTNCYCKKCCFHCQVCFITAALGISYGRKKRRQRRRPPQGSQTHQVSLSKQPTSQSKGEP
TGPKE [SEQ ID NO: 60]

# Fig.9.

Plasmid pRix31:

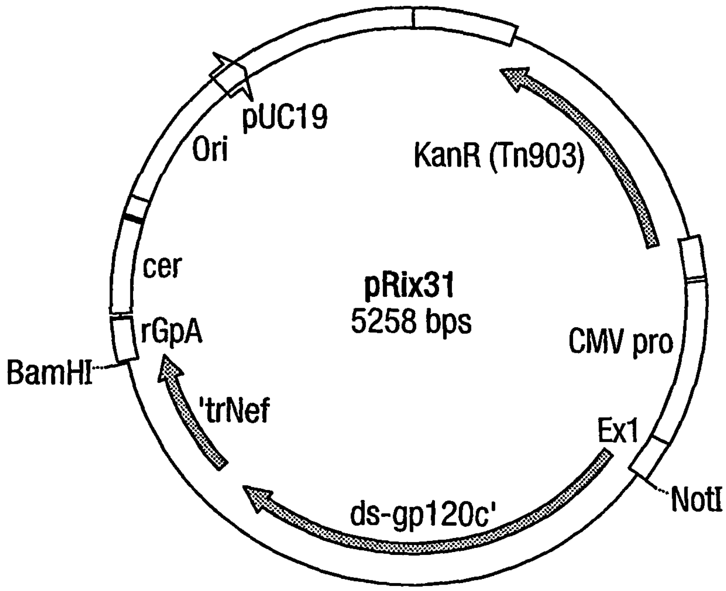


Sequence of insert:

ATGGCCGAGCAGCTGTGGGTCACCGTCTACTACGGCGTGCCTGTGTGGAAGGAGGCCACGACCACCCTCTT
CTGCGCGAGCGACGCCAAGGCCTACGACACGGAAGTGCATAACGTGTGGGCGACGCATGCTTGCGTGCCTA
CGGACCCCAACCCCCAGGAGGTGGTGCTGGGAAACGTGACCGAGTACTTCAACATGTGGAAGAATAACATG
GTGGATCAGATGCACGAGGACATCATCTCTCTGTGGGACCAGTCCCTGAAGCCCTGCGTGAAGCTGACGCC
TCTCTGCGTGACACTGGACTGTGACGACGTCAACACCACCAACAGCACTACCACCACCAGCAACGGCTGGA
CCGGAGAGATTCGGAAGGGCGAGATCAAGAACTGCTCCTTCAATATCACGACCTCGATCAGAGACAAGGTG
CAGAAGGAATACGCGCTGTTTTATAATCTCGATGTGGTCCCCATCGACGACGACAATGCCACCACCAAGAA
CAAGACGACGCGTAATTTCAGACTCATTCACTGCAACAGCAGCGTCATGACGCAGGCCTGCCCCAAGGTGT
CCTTCGAACCAATCCCGATCCATTACTGTGCCCCTGCCGGATTCGCGATCCTCAAGTGTAACAACAAGACC
TTCGACGGGAAGGGCCTGTGCACCAACGTCAGCACGGTGCAGTGCACCCATGGCATCCGCCCCGTCGTGAG
CACCCAGCTGCTGCTGAACGGGTCCCTGGCTGAGGAGGAGGTGGTGATCCGGTCGGACAACTTCATGGACA
ACACCAAGACAATCATCGTCCAGCTGAACGAGTCTGTGGCGATTAACTGTACCCGGCCTAACAACAACACC
CGTAAGGGCATCCACATCGGGCCTGGACGGGCCTTCTATGCCGCCCGCAAGATCATCGGCGACATCCGGCA
GGCCCATTGCAACCTCTCCCGCGCCCAGTGGAATAACACCCTGAAGCAGATCGTGATCAAGCTGAGAGAGC
ACTTTGGAAACAAGACCATCAAGTTCAATCAGAGTTCTGGCGGAGACCCCGAGATCGTGCGGCACTCCTTC
AACTGCGGGGGCGAGTTCTTCTACTGCGATACGACACAGCTCTTCAACTCCACCTGGAACGGCACCGAGGG
CAACAACACAGAGGGAAACTCCACTATCACCCTCCCTTGCCGCATCAAGCAGATCATCAACATGTGGCAGG
AGGTGGGAAAGGCCATGTATGCCCCCCCCATCGGGGGCCAGATCCGCTGCTCCTCCAACATCACCGGCCTG
CTGCTCACCAGAGACGGGGGCACCGAGGGCAACGGCACGGAGAACGAGACGGAGATCTTCAGGCCCGGCGG
CGGCGACATGAGGGATAACTGGCGGAGCGAGCTGTACAAGTACAAGGTGGTGAAGGTGGAGCCGCTCGGCG
TGGCCCCCACCCGGGCCAAGCGCCGCGTCGTGCAGAGAATGGTGGGTTTTCCAGTCACACCTCAGGTACCT
TTAAGACCAATGACTTACAAGGCAGCTGTAGATCTTAGCCACTTTTTAAAAGAAAAGGGGGGACTGGAAGG
GCTAATTCACTCCCAACGAAGACAAGATATCCTTGATCTGTGGATCTACCACACACAAGGCTACTTCCCTG
ATTGGCAGAACTACACACCAGGGCCAGGGGTCAGATATCCACTGACCTTTGGATGGTGCTACAAGCTAGTA
CCAGTTGAGCCAGATAAGGTAGAAGAGGCCAATAAAGGAGAGAACACCAGCTTGTTACACCCTGTGAGCCT
GCATGGAATGGATGACCCTGAGAGAGAAGTGTTAGAGTGGAGGTTTGACAGCCGCCTAGCATTTCATCACG
TGGCCCGAGAGCTGCATCCGGAGTACTTCAAGAACTGCTAA [SEQ ID NO: 61]

Amino acid sequence of antigen:

# Fig.9 (Cont).

MAEQLWVTVYYGVPVWKEATTTLFCASDAKAYDTEVHNVWATHACVPTDPNPQEVVLGNVTEYFNMWKNNM
VDQMHEDIISLWDQSLKPCVKLTPLCVTLDCDDVNTTNSTTTTSNGWTGEIRKGEIKNCSFNITTSIRDKV
QKEYALFYNLDVVPIDDDNATTKNKTTRNFRLIHCNSSVMTQACPKVSFEPIPIHYCAPAGFAILKCNNKT
FDGKGLCTNVSTVQCTHGIRPVVSTQLLLNGSLAEEEVVIRSDNFMDNTKTIIVQLNESVAINCTRPNNNT
RKGIHIGPGRAFYAARKIIGDIRQAHCNLSRAQWNNTLKQIVIKLREHFGNKTIKFNQSSGGDPEIVRHSF
NCGGEFFYCDTTQLFNSTWNGTEGNNTEGNSTITLPCRIKQIINMWQEVGKAMYAPPIGGQIRCSSNITGL
LLTRDGGTEGNGTENETEIFRPGGGDMRDNWRSELYKYKVVKVEPLGVAPTRAKRRVVQRMVGFPVTPQVP
LRPMTYKAAVDLSHFLKEKGGLEGLIHSQRRQDILDLWIYHTQGYFPDWQNYTPGPGVRYPLTFGWCYKLV
PVEPDKVEEANKGENTSLLHPVSLHGMDDPEREVLEWRFDSRLAFHHVARELHPEYFKNC
[SEQ ID NO: 62]

# Fig.10.

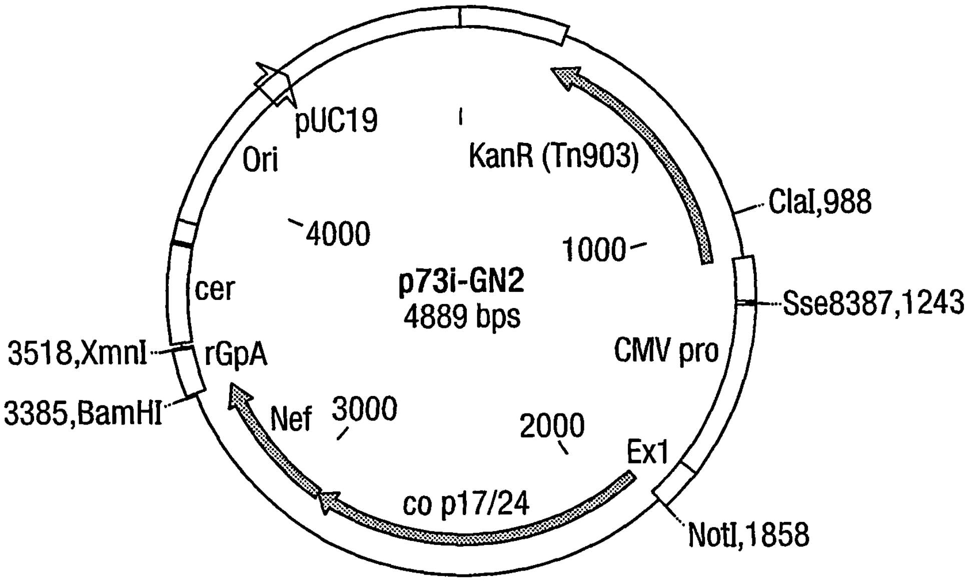


Sequence of insert:

TGGGTGCCCGAGCTTCGGTACTGTCTGGTGGAGAGCTGGACAGATGGGAGAAAATTAGGCTGCGCCCGGGA
GGCAAAAAGAAATACAAGCTCAAGCATATCGTGTGGGCCTCGAGGGAGCTTGAACGGTTTGCCGTGAACCC
AGGCCTGCTGGAAACATCTGAGGGATGTCGCCAGATCCTGGGGCAATTGCAGCCATCCCTCCAGACCGGGA
GTGAAGAGCTGAGGTCCTTGTATAACACAGTGGCTACCCTCTACTGCGTACACCAGAGGATCGAGATTAAG
GATACCAAGGAGGCCTTGGACAAAATTGAGGAGGAGCAAAACAAGAGCAAGAAGAAGGCCCAGCAGGCAGC
TGCTGACACTGGGCATAGCAACCAGGTATCACAGAACTATCCTATTGTCCAAAACATTCAGGGCCAGATGG
TTCATCAGGCCATCAGCCCCCGGACGCTCAATGCCTGGGTGAAGGTTGTCGAAGAGAAGGCCTTTTCTCCT
GAGGTTATCCCCATGTTCTCCGCTTTGAGTGAGGGGGCCACTCCTCAGGACCTCAATACAATGCTTAATAC
CGTGGGCGGCCATCAGGCCGCCATGCAAATGTTGAAGGAGACTATCAACGAGGAGGCAGCCGAGTGGGACA
GAGTGCATCCCGTCCACGCTGGCCCAATCGCGCCCGGACAGATGCGGGAGCCTCGCGGCTCTGACATTGCC
GGCACCACCTCTACACTGCAAGAGCAAATCGGATGGATGACCAACAATCCTCCCATCCCAGTTGGAGAAAT
CTATAAACGGTGGATCATTCTCGGTCTCAATAAAATTGTTAGAATGTACTCTCCGACATCCATCCTTGACA
TTAGACAGGGACCCAAAGAGCCTTTTAGGGATTACGTCGACCGGTTTTATAAGACCCTGCGAGCAGAGCAG
GCCTCTCAGGAGGTCAAAAACTGGATGACGGAGACACTCCTGGTACAGAACGCTAACCCCGACTGCAAAAC
AATCTTGAAGGCACTAGGCCCGGCTGCCACCCTGGAAGAGATGATGACCGCCTGTCAGGGAGTAGGCGGAC
CCGGACACAAAGCCAGAGTGTTG*ATGGTGGGTTTTCCAGTCACACCTCAGGTACCTTTAAGACCAATGAC
TTACAAGGCAGCTGTAGATCTTAGCCACTTTTTAAAAGAAAAGGGGGGACTGGAAGGGCTAATTCACTCCC
AAAGAAGACAAGATATCCTTGATCTGTGGATCTACCACACACAAGGCTACTTCCCTGATTGGCAGAACTAC
ACACCAGGGCCAGGGGTCAGATATCCACTGACCTTTGGATGGTGCTACAAGCTAGTACCAGTTGAGCCAGA
TAAGGTAGAAGAGGCCAATAAAGGAGAGAACACCAGCTTGTTACACCCTGTGAGCCTGCATGGGATGGATG
ACCCGGAGAGAGAAGTGTTAGAGTGGAGGTTTGACAGCCGCCTAGCATTTCATCACGTGGCCCGAGAGCTG
CATCCGGAGTACTTCAAGAACTGCTGA [SEQ ID NO: 63]

# Fig.11.

Plasmid pRix33:

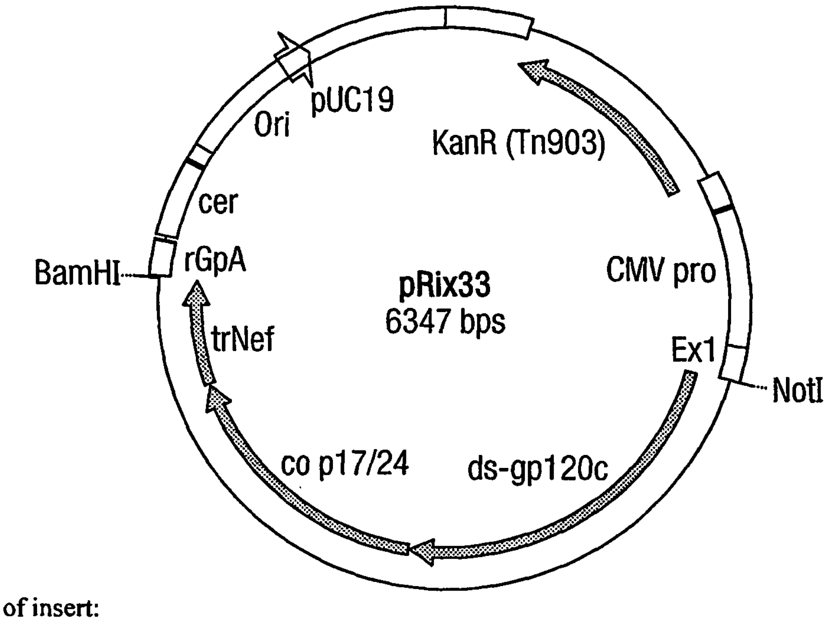


Sequence of insert:

ATGGCCGAGCAGCTGTGGGTCACCGTCTACTACGGCGTGCCTGTGTGGAAGGAGGCCACGACCACCCTCTT
CTGCGCGAGCGACGCCAAGGCCTACGACACGGAAGTGCATAACGTGTGGGCGACGCATGCTTGCGTGCCTA
CGGACCCCAACCCCCAGGAGGTGGTGCTGGGAAACGTGACCGAGTACTTCAACATGTGGAAGAATAACATG
GTGGATCAGATGCACGAGGACATCATCTCTCTGTGGGACCAGTCCCTGAAGCCCTGCGTGAAGCTGACGCC
TCTCTGCGTGACACTGGACTGTGACGACGTCAACACCACCAACAGCACTACCACCACCAGCAACGGCTGGA
CCGGAGAGATTCGGAAGGGCGAGATCAAGAACTGCTCCTTCAATATCACGACCTCGATCAGAGACAAGGTG
CAGAAGGAATACGCGCTGTTTTATAATCTCGATGTGGTCCCCATCGACGACGACAATGCCACCACCAAGAA
CAAGACGACGCGTAATTTCAGACTCATTCACTGCAACAGCAGCGTCATGACGCAGGCCTGCCCCAAGGTGT
CCTTCGAACCAATCCCGATCCATTACTGTGCCCCTGCCGGATTCGCGATCCTCAAGTGTAACAACAAGACC
TTCGACGGGAAGGGCCTGTGCACCAACGTCAGCACGGTGCAGTGCACCCATGGCATCCGCCCCGTCGTGAG
CACCCAGCTGCTGCTGAACGGGTCCCTGGCTGAGGAGGAGGTGGTGATCCGGTCGGACAACTTCATGGACA
ACACCAAGACAATCATCGTCCAGCTGAACGAGTCTGTGGCGATTAACTGTACCCGGCCTAACAACAACACC
CGTAAGGGCATCCACATCGGGCCTGGACGGGCCTTCTATGCCGCCCGCAAGATCATCGGCGACATCCGGCA
GGCCCATTGCAACCTCTCCCGCGCCCAGTGGAATAACACCCTGAAGCAGATCGTGATCAAGCTGAGAGAGC
ACTTTGGAAACAAGACCATCAAGTTCAATCAGAGTTCTGGCGGAGACCCCGAGATCGTGCGGCACTCCTTC
AACTGCGGGGGCGAGTTCTTCTACTGCGATACGACACAGCTCTTCAACTCCACCTGGAACGGCACCGAGGG
CAACAACACAGAGGGAAACTCCACTATCACCCTCCCTTGCCGCATCAAGCAGATCATCAACATGTGGCAGG
AGGTGGGAAAGGCCATGTATGCCCCCCCCATCGGGGGCCAGATCCGCTGCTCCTCCAACATCACCGGCCTG
CTGCTCACCAGAGACGGGGGCACCGAGGGCAACGGCACGGAGAACGAGACGGAGATCTTCAGGCCCGGCGG
CGGCGACATGAGGGATAACTGGCGGAGCGAGCTGTACAAGTACAAGGTGGTGAAGGTGGAGCCGCTCGGCG
TGGCCCCCACCCGGGCCAAGCGCCGCGTCGTGCAGAGAATGGGTGCCCGAGCTTCGGTACTGTCTGGTGGA
GAGCTGGACAGATGGGAGAAAATTAGGCTGCGCCCGGGAGGCAAAAAGAAATACAAGCTCAAGCATATCGT
GTGGGCCTCGAGGGAGCTTGAACGGTTTGCCGTGAACCCAGGCCTGCTGGAAACATCTGAGGGATGTCGCC
AGATCCTGGGGCAATTGCAGCCATCCCTCCAGACCGGGAGTGAAGAGCTGAGGTCCTTGTATAACACAGTG
GCTACCCTCTACTGCGTACACCAGAGGATCGAGATTAAGGATACCAAGGAGGCCTTGGACAAAATTGAGGA
GGAGCAAAACAAGAGCAAGAAGAAGGCCCAGCAGGCAGCTGCTGACACTGGGCATAGCAACCAGGTATCAC
AGAACTATCCTATTGTCCAAAACATTCAGGGCCAGATGGTTCATCAGGCCATCAGCCCCCGGACGCTCAAT
GCCTGGGTGAAGGTTGTCGAAGAGAAGGCCTTTTCTCCTGAGGTTATCCCCATGTTCTCCGCTTTGAGTGA
GGGGGCCACTCCTCAGGACCTCAATACAATGCTTAATACCGTGGGCGGCCATCAGGCCGCCATGCAAATGT
TGAAGGAGACTATCAACGAGGAGGCAGCCGAGTGGGACAGAGTGCATCCCGTCCACGCTGGCCCAATCGCG
CCCGGACAGATGCGGGAGCCTCGCGGCTCTGACATTGCCGGCACCACCTCTACACTGCAAGAGCAAATCGG

# Fig.11 (Cont).

ATGGATGACCAACAATCCTCCCATCCCAGTTGGAGAAATCTATAAACGGTGGATCATTCTCGGTCTCAATA
AAATTGTTAGAATGTACTCTCCGACATCCATCCTTGACATTAGACAGGGACCCAAAGAGCCTTTTAGGGAT
TACGTCGACCGGTTTTATAAGACCCTGCGAGCAGAGCAGGCCTCTCAGGAGGTCAAAAACTGGATGACGGA
GACACTCCTGGTACAGAACGCTAACCCCGACTGCAAAACAATCTTGAAGGCACTAGGCCCGGCTGCCACCC
TGGAAGAGATGATGACCGCCTGTCAGGGAGTAGGCGGACCCGGACACAAAGCCAGAGTGTTGATGGTGGGT
TTTCCAGTCACACCTCAGGTACCTTTAAGACCAATGACTTACAAGGCAGCTGTAGATCTTAGCCACTTTTT
AAAAGAAAAGGGGGGACTGGAAGGGCTAATTCACTCCCAACGAAGACAAGATATCCTTGATCTGTGGATCT
ACCACACACAAGGCTACTTCCCTGATTGGCAGAACTACACACCAGGGCCAGGGGTCAGATATCCACTGACC
TTTGGATGGTGCTACAAGCTAGTACCAGTTGAGCCAGATAAGGTAGAAGAGGCCAATAAAGGAGAGAACAC
CAGCTTGTTACACCCTGTGAGCCTGCATGGAATGGATGACCCTGAGAGAGAAGTGTTAGAGTGGAGGTTTG
ACAGCCGCCTAGCATTTCATCACGTGGCCCGAGAGCTGCATCCGGAGTACTTCAAGAACTGCTAA
[SEQ ID NO: 64]

Amino acid sequence of antigen:

MAEQLWVTVYYGVPVWKEATTTLFCASDAKAYDTEVHNVWATHACVPTDPNPQEVVLGNVTEYFNMWKNNM
VDQMHEDIISLWDQSLKPCVKLTPLCVTLDCDDVNTTNSTTTTSNGWTGEIRKGEIKNCSFNITTSIRDKV
QKEYALFYNLDVVPIDDDNATTKNKTTRNFRLIHCNSSVMTQACPKVSFEPIPIHYCAPAGFAILKCNNKT
FDGKGLCTNVSTVQCTHGIRPVVSTQLLLNGSLAEEEVVIRSDNFMDNTKTIIVQLNESVAINCTRPNNNT
RKGIHIGPGRAFYAARKIIGDIRQAHCNLSRAQWNNTLKQIVIKLREHFGNKTIKFNQSSGGDPEIVRHSF
NCGGEFFYCDTTQLFNSTWNGTEGNNTEGNSTITLPCRIKQIINMWQEVGKAMYAPPIGGQIRCSSNITGL
LLTRDGGTEGNGTENETEIFRPGGGDMRDNWRSELYKYKVVKVEPLGVAPTRAKRRVVQRMGARASVLSGG
ELDRWEKIRLRPGGKKKYKLKHIVWASRELERFAVNPGLLETSEGCRQILGQLQPSLQTGSEELRSLYNTV
ATLYCVHQRIEIKDTKEALDKIEEEQNKSKKKAQQAAADTGHSNQVSQNYPIVQNIQGQMVHQAISPRTLN
AWVKVVEEKAFSPEVIPMFSALSEGATPQDLNTMLNTVGGHQAAMQMLKETINEEAAEWDRVHPVHAGPIA
PGQMREPRGSDIAGTTSTLQEQIGWMTNNPPIPVGEIYKRWIILGLNKIVRMYSPTSILDIRQGPKEPFRD
YVDRFYKTLRAEQASQEVKNWMTETLLVQNANPDCKTILKALGPAATLEEMMTACQGVGGPGHKARVLMVG
FPVTPQVPLRPMTYKAAVDLSHFLKEKGGLEGLIHSQRRQDILDLWIYHTQGYFPDWQNYTPGPGVRYPLT
FGWCYKLVPVEPDKVEEANKGENTSLLHPVSLHGMDDPEREVLEWRFDSRLAFHHVARELHPEYFKNC

[SEQ ID NO: 65]

# Fig.12.

Plasmid pRix35:

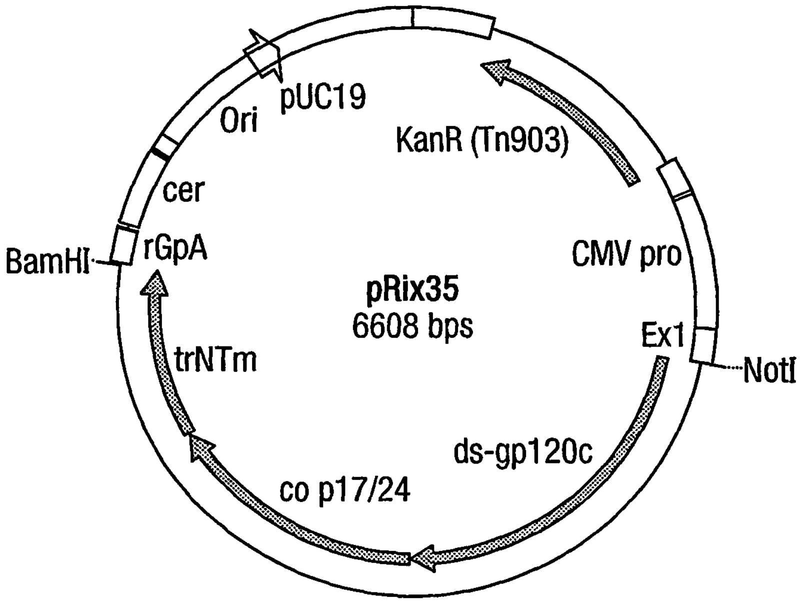


Sequence of insert

ATGGCCGAGCAGCTGTGGGTCACCGTCTACTACGGCGTGCCTGTGTGGAAGGAGGCCACGACCACCCTCTT
CTGCGCGAGCGACGCCAAGGCCTACGACACGGAAGTGCATAACGTGTGGGCGACGCATGCTTGCGTGCCTA
CGGACCCCAACCCCCAGGAGGTGGTGCTGGGAAACGTGACCGAGTACTTCAACATGTGGAAGAATAACATG
GTGGATCAGATGCACGAGGACATCATCTCTCTGTGGGACCAGTCCCTGAAGCCCTGCGTGAAGCTGACGCC
TCTCTGCGTGACACTGGACTGTGACGACGTCAACACCACCAACAGCACTACCACCACCAGCAACGGCTGGA
CCGGAGAGATTCGGAAGGGCGAGATCAAGAACTGCTCCTTCAATATCACGACCTCGATCAGAGACAAGGTG
CAGAAGGAATACGCGCTGTTTTATAATCTCGATGTGGTCCCCATCGACGACGACAATGCCACCACCAAGAA
CAAGACGACGCGTAATTTCAGACTCATTCACTGCAACAGCAGCGTCATGACGCAGGCCTGCCCCAAGGTGT
CCTTCGAACCAATCCCGATCCATTACTGTGCCCCTGCCGGATTCGCGATCCTCAAGTGTAACAACAAGACC
TTCGACGGGAAGGGCCTGTGCACCAACGTCAGCACGGTGCAGTGCACCCATGGCATCCGCCCCGTCGTGAG
CACCCAGCTGCTGCTGAACGGGTCCCTGGCTGAGGAGGAGGTGGTGATCCGGTCGGACAACTTCATGGACA
ACACCAAGACAATCATCGTCCAGCTGAACGAGTCTGTGGCGATTAACTGTACCCGGCCTAACAACAACACC
CGTAAGGGCATCCACATCGGGCCTGGACGGGCCTTCTATGCCGCCCGCAAGATCATCGGCGACATCCGGCA
GGCCCATTGCAACCTCTCCCGCGCCCAGTGGAATAACACCCTGAAGCAGATCGTGATCAAGCTGAGAGAGC
ACTTTGGAAACAAGACCATCAAGTTCAATCAGAGTTCTGGCGGAGACCCCGAGATCGTGCGGCACTCCTTC
AACTGCGGGGGCGAGTTCTTCTACTGCGATACGACACAGCTCTTCAACTCCACCTGGAACGGCACCGAGGG
CAACAACACAGAGGGAAACTCCACTATCACCCTCCCTTGCCGCATCAAGCAGATCATCAACATGTGGCAGG
AGGTGGGAAAGGCCATGTATGCCCCCCCCATCGGGGGCCAGATCCGCTGCTCCTCCAACATCACCGGCCTG
CTGCTCACCAGAGACGGGGGCACCGAGGGCAACGGCACGGAGAACGAGACGGAGATCTTCAGGCCCGGCGG
CGGCGACATGAGGGATAACTGGCGGAGCGAGCTGTACAAGTACAAGGTGGTGAAGGTGGAGCCGCTCGGCG
TGGCCCCCACCCGGGCCAAGCGCCGCGTCGTGCAGAGAATGGGTGCCCGAGCTTCGGTACTGTCTGGTGGA
GAGCTGGACAGATGGGAGAAAATTAGGCTGCGCCCGGGAGGCAAAAAGAAATACAAGCTCAAGCATATCGT
GTGGGCCTCGAGGGAGCTTGAACGGTTTGCCGTGAACCCAGGCCTGCTGGAAACATCTGAGGGATGTCGCC
AGATCCTGGGGCAATTGCAGCCATCCCTCCAGACCGGGAGTGAAGAGCTGAGGTCCTTGTATAACACAGTG
GCTACCCTCTACTGCGTACACCAGAGGATCGAGATTAAGGATACCAAGGAGGCCTTGGACAAAATTGAGGA
GGAGCAAAACAAGAGCAAGAAGAAGGCCCAGCAGGCAGCTGCTGACACTGGGCATAGCAACCAGGTATCAC
AGAACTATCCTATTGTCCAAAACATTCAGGGCCAGATGGTTCATCAGGCCATCAGCCCCCGGACGCTCAAT
GCCTGGGTGAAGGTTGTCGAAGAGAAGGCCTTTTCTCCTGAGGTTATCCCCATGTTCTCCGCTTTGAGTGA
GGGGGCCACTCCTCAGGACCTCAATACAATGCTTAATACCGTGGGCGGCCATCAGGCCGCCATGCAAATGT
TGAAGGAGACTATCAACGAGGAGGCAGCCGAGTGGGACAGAGTGCATCCCGTCCACGCTGGCCCAATCGCG
CCCGGACAGATGCGGGAGCCTCGCGGCTCTGACATTGCCGGCACCACCTCTACACTGCAAGAGCAAATCGG

# Fig.12 (Cont).

ATGGATGACCAACAATCCTCCCATCCCAGTTGGAGAAATCTATAAACGGTGGATCATTCTCGGTCTCAATA
AAATTGTTAGAATGTACTCTCCGACATCCATCCTTGACATTAGACAGGGACCCAAAGAGCCTTTTAGGGAT
TACGTCGACCGGTTTTATAAGACCCTGCGAGCAGAGCAGGCCTCTCAGGAGGTCAAAAACTGGATGACGGA
GACACTCCTGGTACAGAACGCTAACCCCGACTGCAAAACAATCTTGAAGGCACTAGGCCCGGCTGCCACCC
TGGAAGAGATGATGACCGCCTGTCAGGGAGTAGGCGGACCCGGACACAAAGCCAGAGTGTTGATGGTGGGT
TTTCCAGTCACACCTCAGGTACCTTTAAGACCAATGACTTACAAGGCAGCTGTAGATCTTAGCCACTTTTT
AAAAGAAAAGGGGGGACTGGAAGGGCTAATTCACTCCCAACGAAGACAAGATATCCTTGATCTGTGGATCT
ACCACACACAAGGCTACTTCCCTGATTGGCAGAACTACACACCAGGGCCAGGGGTCAGATATCCACTGACC
TTTGGATGGTGCTACAAGCTAGTACCAGTTGAGCCAGATAAGGTAGAAGAGGCCAATAAAGGAGAGAACAC
CAGCTTGTTACACCCTGTGAGCCTGCATGGAATGGATGACCCTGAGAGAGAAGTGTTAGAGTGGAGGTTTG
ACAGCCGCCTAGCATTTCATCACGTGGCCCGAGAGCTGCATCCGGAGTACTTCAAGAACTGCACTAGTGAG
CCAGTAGATCCTAGACTAGAGCCCTGGAAGCATCCAGGAAGTCAGCCTAAAACTGCTTGTACCAATTGCTA
TTGTAAAAAGTGTTGCTTTCATTGCCAAGTTTGTTTCATAACAGCTGCCTTAGGCATCTCCTATGGCAGGA
AGAAGCGGAGACAGCGACGAAGACCTCCTCAAGGCAGTCAGACTCATCAAGTTTCTCTATCAAAGCAACCC
ACCTCCCAATCCAAAGGGGAGCCGACAGGCCCGAAGGAATAA [SEQ ID NO: 66]

Amino acid sequence of antigen:

MAEQLWVTVYYGVPVWKEATTTLFCASDAKAYDTEVHNVWATHACVPTDPNPQEVVLGNVTEYFNMWKNNM
VDQMHEDIISLWDQSLKPCVKLTPLCVTLDCDDVNTTNSTTTTSNGWTGEIRKGEIKNCSFNITTSIRDKV
QKEYALFYNLDVVPIDDDNATTKNKTTRNFRLIHCNSSVMTQACPKVSFEPIPIHYCAPAGFAILKCNNKT
FDGKGLCTNVSTVQCTHGIRPVVSTQLLLNGSLAEEEVVIRSDNFMDNTKTIIVQLNESVAINCTRPNNNT
RKGIHIGPGRAFYAARKIIGDIRQAHCNLSRAQWNNTLKQIVIKLREHFGNKTIKFNQSSGGDPEIVRHSF
NCGGEFFYCDTTQLFNSTWNGTEGNNTEGNSTITLPCRIKQIINMWQEVGKAMYAPPIGGQIRCSSNITGL
LLTRDGGTEGNGTENETEIFRPGGGDMRDNWRSELYKYKVVKVEPLGVAPTRAKRRVVQRMGARASVLSGG
ELDRWEKIRLRPGGKKKYKLKHIVWASRELERFAVNPGLLETSEGCRQILGQLQPSLQTGSEELRSLYNTV
ATLYCVHQRIEIKDTKEALDKIEEEQNKSKKKAQQAAADTGHSNQVSQNYPIVQNIQGQMVHQAISPRTLN
AWVKVVEEKAFSPEVIPMFSALSEGATPQDLNTMLNTVGGHQAAMQMLKETINEEAAEWDRVHPVHAGPIA
PGQMREPRGSDIAGTTSTLQEQIGWMTNNPPIPVGEIYKRWIILGLNKIVRMYSPTSILDIRQGPKEPFRD
YVDRFYKTLRAEQASQEVKNWMTETLLVQNANPDCKTILKALGPAATLEEMMTACQGVGGPGHKARVLMVG
FPVTPQVPLRPMTYKAAVDLSHFLKEKGGLEGLIHSQRRQDILDLWIYHTQGYFPDWQNYTPGPGVRYPLT
FGWCYKLVPVEPDKVEEANKGENTSLLHPVSLHGMDDPEREVLEWRFDSRLAFHHVARELHPEYFKNCTSE
PVDPRLEPWKHPGSQPKTACTNCYCKKCCFHCQVCFITAALGISYGRKKRRQRRRPPQGSQTHQVSLSKQP
TSQSKGEPTGPKE [SEQ ID NO: 67]

# Fig.13.

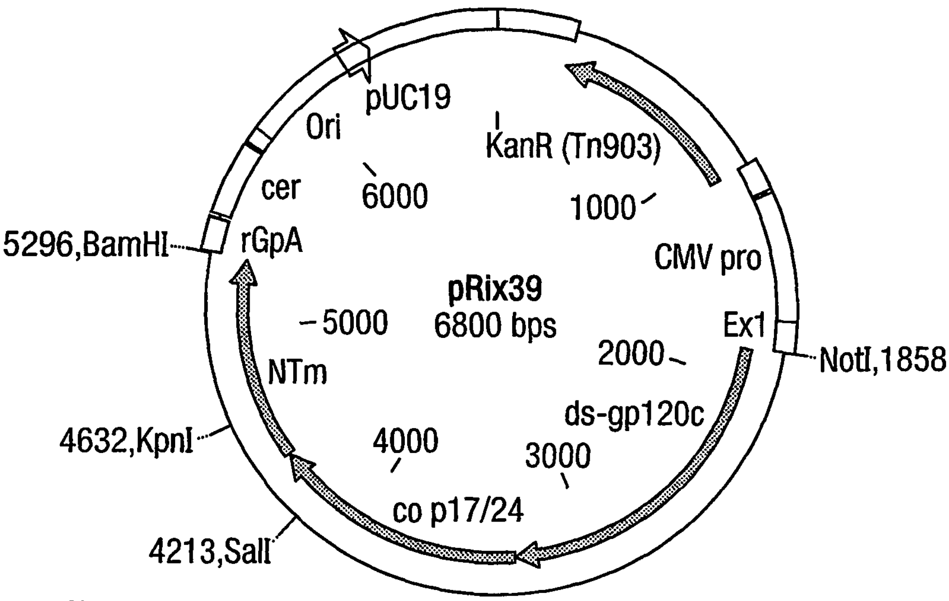


Sequence of insert:

ATGGCCGAGCAGCTGTGGGTCACCGTCTACTACGGCGTGCCTGTGTGGAAGGAGGCCACGACCACCCTCTT
CTGCGCGAGCGACGCCAAGGCCTACGACACGGAAGTGCATAACGTGTGGGCGACGCATGCTTGCGTGCCTA
CGGACCCCAACCCCCAGGAGGTGGTGCTGGGAAACGTGACCGAGTACTTCAACATGTGGAAGAATAACATG
GTGGATCAGATGCACGAGGACATCATCTCTCTGTGGGACCAGTCCCTGAAGCCCTGCGTGAAGCTGACGCC
TCTCTGCGTGACACTGGACTGTGACGACGTCAACACCACCAACAGCACTACCACCACCAGCAACGGCTGGA
CCGGAGAGATTCGGAAGGGCGAGATCAAGAACTGCTCCTTCAATATCACGACCTCGATCAGAGACAAGGTG
CAGAAGGAATACGCGCTGTTTTATAATCTCGATGTGGTCCCCATCGACGACGACAATGCCACCACCAAGAA
CAAGACGACGCGTAATTTCAGACTCATTCACTGCAACAGCAGCGTCATGACGCAGGCCTGCCCCAAGGTGT
CCTTCGAACCAATCCCGATCCATTACTGTGCCCCTGCCGGATTCGCGATCCTCAAGTGTAACAACAAGACC
TTCGACGGGAAGGGCCTGTGCACCAACGTCAGCACGGTGCAGTGCACCCATGGCATCCGCCCCGTCGTGAG
CACCCAGCTGCTGCTGAACGGGTCCCTGGCTGAGGAGGAGGTGGTGATCCGGTCGGACAACTTCATGGACA
ACACCAAGACAATCATCGTCCAGCTGAACGAGTCTGTGGCGATTAACTGTACCCGGCCTAACAACAACACC
CGTAAGGGCATCCACATCGGGCCTGGACGGGCCTTCTATGCCGCCCGCAAGATCATCGGCGACATCCGGCA
GGCCCATTGCAACCTCTCCCGCGCCCAGTGGAATAACACCCTGAAGCAGATCGTGATCAAGCTGAGAGAGC
ACTTTGGAAACAAGACCATCAAGTTCAATCAGAGTTCTGGCGGAGACCCCGAGATCGTGCGGCACTCCTTC
AACTGCGGGGGCGAGTTCTTCTACTGCGATACGACACAGCTCTTCAACTCCACCTGGAACGGCACCGAGGG
CAACAACACAGAGGGAAACTCCACTATCACCCTCCCTTGCCGCATCAAGCAGATCATCAACATGTGGCAGG
AGGTGGGAAAGGCCATGTATGCCCCCCCCATCGGGGGCCAGATCCGCTGCTCCTCCAACATCACCGGCCTG
CTGCTCACCAGAGACGGGGGCACCGAGGGCAACGGCACGGAGAACGAGACGGAGATCTTCAGGCCCGGCGG
CGGCGACATGAGGGATAACTGGCGGAGCGAGCTGTACAAGTACAAGGTGGTGAAGGTGGAGCCGCTCGGCG
TGGCCCCCACCCGGGCCAAGCGCCGCGTCGTGCAGAGAATGGGTGCCCGAGCTTCGGTACTGTCTGGTGGA
GAGCTGGACAGATGGGAGAAAATTAGGCTGCGCCCGGGAGGCAAAAAGAAATACAAGCTCAAGCATATCGT
GTGGGCCTCGAGGGAGCTTGAACGGTTTGCCGTGAACCCAGGCCTGCTGGAAACATCTGAGGGATGTCGCC
AGATCCTGGGGCAATTGCAGCCATCCCTCCAGACCGGGAGTGAAGAGCTGAGGTCCTTGTATAACACAGTG
GCTACCCTCTACTGCGTACACCAGAGGATCGAGATTAAGGATACCAAGGAGGCCTTGGACAAAATTGAGGA
GGAGCAAAACAAGAGCAAGAAGAAGGCCCAGCAGGCAGCTGCTGACACTGGGCATAGCAACCAGGTATCAC
AGAACTATCCTATTGTCCAAAACATTCAGGGCCAGATGGTTCATCAGGCCATCAGCCCCCGGACGCTCAAT
GCCTGGGTGAAGGTTGTCGAAGAGAAGGCCTTTTCTCCTGAGGTTATCCCCATGTTCTCCGCTTTGAGTGA
GGGGGCCACTCCTCAGGACCTCAATACAATGCTTAATACCGTGGGCGGCCATCAGGCCGCCATGCAAATGT
TGAAGGAGACTATCAACGAGGAGGCAGCCGAGTGGGACAGAGTGCATCCCGTCCACGCTGGCCCAATCGCG
CCCGGACAGATGCGGGAGCCTCGCGGCTCTGACATTGCCGGCACCACCTCTACACTGCAAGAGCAAATCGG
ATGGATGACCAACAATCCTCCCATCCCAGTTGGAGAAATCTATAAACGGTGGATCATTCTCGGTCTCAATA
AAATTGTTAGAATGTACTCTCCGACATCCATCCTTGACATTAGACAGGGACCCAAAGAGCCTTTTAGGGAT

# Fig.13 (Cont).

TACGTCGACCGGTTTTATAAGACCCTGCGAGCAGAGCAGGCCTCTCAGGAGGTCAAAAACTGGATGACGGA
GACACTCCTGGTACAGAACGCTAACCCCGACTGCAAAACAATCTTGAAGGCACTAGGCCCGGCTGCCACCC
TGGAAGAGATGATGACCGCCTGTCAGGGAGTAGGCGGACCCGGACACAAAGCCAGAGTGTTGATGGGTGGC
AAGTGGTCAAAAAGTAGTGTGGTTGGATGGCCTACTGTAAGGGAAAGAATGAGACGAGCTGAGCCAGCAGC
AGATGGGGTGGGAGCAGCATCTCGAGACCTGGAAAAACATGGAGCAATCACAAGTAGCAATACAGCAGCTA
CCAATGCTGCTTGTGCCTGGCTAGAAGCACAAGAGGAGGAGGAGGTGGGTTTTCCAGTCACACCTCAGGTA
CCTTTAAGACCAATGACTTACAAGGCAGCTGTAGATCTTAGCCACTTTTTAAAAGAAAAGGGGGGACTGGA
AGGGCTAATTCACTCCCAACGAAGACAAGATATCCTTGATCTGTGGATCTACCACACACAAGGCTACTTCC
CTGATTGGCAGAACTACACACCAGGGCCAGGGGTCAGATATCCACTGACCTTTGGATGGTGCTACAAGCTA
GTACCAGTTGAGCCAGATAAGGTAGAAGAGGCCAATAAAGGAGAGAACACCAGCTTGTTACACCCTGTGAG
CCTGCATGGAATGGATGACCCTGAGAGAGAAGTGTTAGAGTGGAGGTTTGACAGCCGCCTAGCATTTCATC
ACGTGGCCCGAGAGCTGCATCCGGAGTACTTCAAGAACTGCACTAGTGAGCCAGTAGATCCTAGACTAGAG
CCCTGGAAGCATCCAGGAAGTCAGCCTAAAACTGCTTGTACCAATTGCTATTGTAAAAAGTGTTGCTTTCA
TTGCCAAGTTTGTTTCATAACAGCTGCCTTAGGCATCTCCTATGGCAGGAAGAAGCGGAGACAGCGACGAA
GACCTCCTCAAGGCAGTCAGACTCATCAAGTTTCTCTATCAAAGCAACCCACCTCCCAATCCAAAGGGGAG
CCGACAGGCCCGAAGGAATAA [SEQ ID NO: 68]

Amino acid sequence of antigen:

MAEQLWVTVYYGVPVWKEATTTLFCASDAKAYDTEVHNVWATHACVPTDPNPQEVVLGNVTEYFNMWKNNM
VDQMHEDIISLWDQSLKPCVKLTPLCVTLDCDDVNTTNSTTTTSNGWTGEIRKGEIKNCSFNITTSIRDKV
QKEYALFYNLDVVPIDDDNATTKNKTTRNFRLIHCNSSVMTQACPKVSFEPIPIHYCAPAGFAILKCNNKT
FDGKGLCTNVSTVQCTHGIRPVVSTQLLLNGSLAEEEVVIRSDNFMDNTKTIIVQLNESVAINCTRPNNNT
RKGIHIGPGRAFYAARKIIGDIRQAHCNLSRAQWNNTLKQIVIKLREHFGNKTIKFNQSSGGDPEIVRHSF
NCGGEFFYCDTTQLFNSTWNGTEGNNTEGNSTITLPCRIKQIINMWQEVGKAMYAPPIGGQIRCSSNITGL
LLTRDGGTEGNGTENETEIFRPGGGDMRDNWRSELYKYKVVKVEPLGVAPTRAKRRVVQRMGARASVLSGG
ELDRWEKIRLRPGGKKKYKLKHIVWASRELERFAVNPGLLETSEGCRQILGQLQPSLQTGSEELRSLYNTV
ATLYCVHQRIEIKDTKEALDKIEEEQNKSKKKAQQAAADTGHSNQVSQNYPIVQNIQGQMVHQAISPRTLN
AWVKVVEEKAFSPEVIPMFSALSEGATPQDLNTMLNTVGGHQAAMQMLKETINEEAAEWDRVHPVHAGPIA
PGQMREPRGSDIAGTTSTLQEQIGWMTNNPPIPVGEIYKRWIILGLNKIVRMYSPTSILDIRQGPKEPFRD
YVDRFYKTLRAEQASQEVKNWMTETLLVQNANPDCKTILKALGPAATLEEMMTACQGVGGPGHKARVLMGG
KWSKSSVVGWPTVRERMRRAEPAADGVGAASRDLEKHGAITSSNTAATNAACAWLEAQEEEEVGFPVTPQV
PLRPMTYKAAVDLSHFLKEKGGLEGLIHSQRRQDILDLWIYHTQGYFPDWQNYTPGPGVRYPLTFGWCYKL
VPVEPDKVEEANKGENTSLLHPVSLHGMDDPEREVLEWRFDSRLAFHHVARELHPEYFKNCTSEPVDPRLE
PWKHPGSQPKTACTNCYCKKCCFHCQVCFITAALGISYGRKKRRQRRRPPQGSQTHQVSLSKQPTSQSKGE
PTGPKE [SEQ ID NO: 69]

# Fig.14.

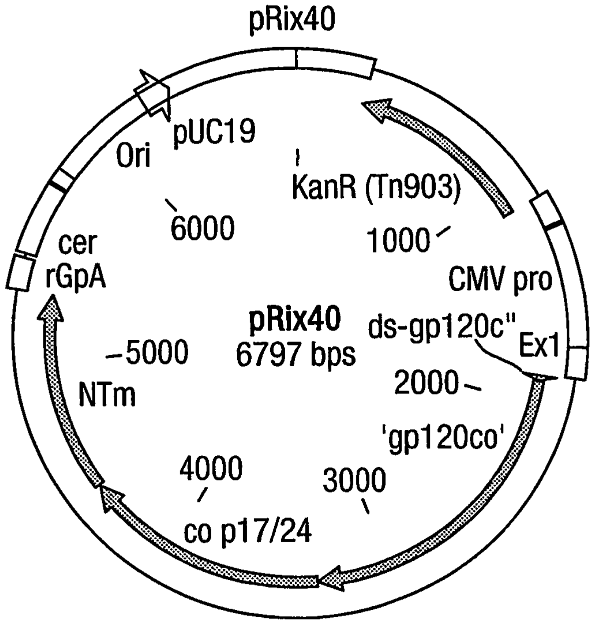


DNA sequence of insert

ATGGCCGAGCAGCTGTGGGTCACCGTCTACTACGGCGTGCCTGTGTGGAAGGAGGCCACGACCACCCTCTT
CTGCGCGAGCGACGCCAAGGCCTACGACACGGAAGTGCATAACGTGTGGGCGACGCATGCTTGCGTGCCTA
CGGACCCCAACCCCCAGGAGGTGGTGCTGGGAAACGTGACCGAGTACTTCAACATGTGGAAGAATAACATG
GTGGATCAGATGCACGAGGACATCATCTCTCTGTGGGACCAGTCCCTGAAGCCCTGCGTGAAGCTGACGCC
TCTCTGCGTGACACTGGACTGTGACGACGTCAACACCACCAACAGCACTACCACCACCAGCAACGGCTGGA
CCGGAGAGATTCGGAAGGGCGAGATCAAGAACTGCTCCTTCAATATCACGACCTCGATCAGAGACAAGGTG
CAGAAGGAATACGCGCTGTTTTATAATCTCGATGTGGTCCCCATCGACGACGACAATGCCACCACCAAGAA
CAAGACGACGCGTAATTTCAGACTCATTCACTGCAACAGCAGCGTCATGACGCAGGCCTGCCCCAAGGTGT
CCTTCGAACCAATCCCGATCCATTACTGTGCCCCTGCCGGATTCGCGATCCTCAAGTGTAACAACAAGACC
TTCGACGGGAAGGGCCTGTGCACCAACGTCAGCACGGTGCAGTGCACCCATGGCATCCGCCCCGTCGTGAG
CACCCAGCTGCTGCTGAACGGGTCCCTGGCTGAGGAGGAGGTGGTGATCCGGTCGGACAACTTCATGGACA
ACACCAAGACAATCATCGTCCAGCTGAACGAGTCTGTGGCGATTAACTGTACCCGGCCTAACAACAACACC
CGTAAGGGCATCCACATCGGGCCTGGACGGGCCTTCTATGCCGCCCGCAAGATCATCGGCGACATCCGGCA
GGCCCATTGCAACCTCTCCCGCGCCCAGTGGAATAACACCCTGAAGCAGATCGTGATCAAGCTGAGAGAGC
ACTTTGGAAACAAGACCATCAAGTTCAATCAGAGTTCTGGCGGAGACCCCGAGATCGTGCGGCACTCCTTC
AACTGCGGGGGCGAGTTCTTCTACTGCGATACGACACAGCTCTTCAACTCCACCTGGAACGGCACCGAGGG
CAACAACACAGAGGGAAACTCCACTATCACCCTCCCTTGCCGCATCAAGCAGATCATCAACATGTGGCAGG
AGGTGGGAAAGGCCATGTATGCCCCCCCCATCGGGGGCCAGATCCGCTGCTCCTCCAACATCACCGGCCTG
CTGCTCACCAGAGACGGGGGCACCGAGGGCAACGGCACGGAGAACGAGACGGAGATCTTCAGGCCCGGCGG
CGGCGACATGAGGGATAACTGGCGGAGCGAGCTGTACAAGTACAAGGTGGTGAAGGTGGAGCCGCTCGGCG
TGGCCCCCACCCGGGCCAAGCGCCGCGTCGTGCAGAGAATGGGTGCCCGAGCTTCGGTACTGTCTGGTGGA
GAGCTGGACAGATGGGAGAAAATTAGGCTGCGCCCGGGAGGCAAAAAGAAATACAAGCTCAAGCATATCGT
GTGGGCCTCGAGGGAGCTTGAACGGTTTGCCGTGAACCCAGGCCTGCTGGAAACATCTGAGGGATGTCGCC
AGATCCTGGGGCAATTGCAGCCATCCCTCCAGACCGGGAGTGAAGAGCTGAGGTCCTTGTATAACACAGTG
GCTACCCTCTACTGCGTACACCAGAGGATCGAGATTAAGGATACCAAGGAGGCCTTGGACAAAATTGAGGA
GGAGCAAAACAAGAGCAAGAAGAAGGCCCAGCAGGCAGCTGCTGACACTGGGCATAGCAACCAGGTATCAC
AGAACTATCCTATTGTCCAAAACATTCAGGGCCAGATGGTTCATCAGGCCATCAGCCCCCGGACGCTCAAT
GCCTGGGTGAAGGTTGTCGAAGAGAAGGCCTTTTCTCCTGAGGTTATCCCCATGTTCTCCGCTTTGAGTGA
GGGGGCCACTCCTCAGGACCTCAATACAATGCTTAATACCGTGGGCGGCCATCAGGCCGCCATGCAAATGT
TGAAGGAGACTATCAACGAGGAGGCAGCCGAGTGGGACAGAGTGCATCCCGTCCACGCTGGCCCAATCGCG

# Fig.14 (Cont).

CCCGGACAGATGCGGGAGCCTCGCGGCTCTGACATTGCCGGCACCACCTCTACACTGCAAGAGCAAATCGG
ATGGATGACCAACAATCCTCCCATCCCAGTTGGAGAAATCTATAAACGGTGGATCATTCTCGGTCTCAATA
AAATTGTTAGAATGTACTCTCCGACATCCATCCTTGACATTAGACAGGGACCCAAAGAGCCTTTTAGGGAT
TACGTCGACCGGTTTTATAAGACCCTGCGAGCAGAGCAGGCCTCTCAGGAGGTCAAAAACTGGATGACGGA
GACACTCCTGGTACAGAACGCTAACCCCGACTGCAAAACAATCTTGAAGGCACTAGGCCCGGCTGCCACCC
TGGAAGAGATGATGACCGCCTGTCAGGGAGTAGGCGGACCCGGACACAAAGCCAGAGTGTTGATGGGCAAG
TGGTCAAAAAGTAGTGTGGTTGGATGGCCTACTGTAAGGGAAAGAATGAGACGAGCTGAGCCAGCAGCAGA
TGGGGTGGGAGCAGCATCTCGAGACCTGGAAAAACATGGAGCAATCACAAGTAGCAATACAGCAGCTACCA
ATGCTGCTTGTGCCTGGCTAGAAGCACAAGAGGAGGAGGAGGTGGGTTTTCCAGTCACACCTCAGGTACCT
TTAAGACCAATGACTTACAAGGCAGCTGTAGATCTTAGCCACTTTTTAAAAGAAAAGGGGGGACTGGAAGG
GCTAATTCACTCCCAACGAAGACAAGATATCCTTGATCTGTGGATCTACCACACACAAGGCTACTTCCCTG
ATTGGCAGAACTACACACCAGGGCCAGGGGTCAGATATCCACTGACCTTTGGATGGTGCTACAAGCTAGTA
CCAGTTGAGCCAGATAAGGTAGAAGAGGCCAATAAAGGAGAGAACACCAGCTTGTTACACCCTGTGAGCCT
GCATGGAATGGATGACCCTGAGAGAGAAGTGTTAGAGTGGAGGTTTGACAGCCGCCTAGCATTTCATCACG
TGGCCCGAGAGCTGCATCCGGAGTACTTCAAGAACTGCACTAGTGAGCCAGTAGATCCTAGACTAGAGCCC
TGGAAGCATCCAGGAAGTCAGCCTAAAACTGCTTGTACCAATTGCTATTGTAAAAAGTGTTGCTTTCATTG
CCAAGTTTGTTTCATAACAGCTGCCTTAGGCATCTCCTATGGCAGGAAGAAGCGGAGACAGCGACGAAGAC
CTCCTCAAGGCAGTCAGACTCATCAAGTTTCTCTATCAAAGCAACCCACCTCCCAATCCAAAGGGGAGCCG
ACAGGCCCGAAGGAATAA [SEQ ID NO: 70]

**Aminoacid sequence of insert**

MAEQLWVTVYYGVPVWKEATTTLFCASDAKAYDTEVHNVWATHACVPTDPNPQEVVLGNVTEYFNMWKNNM
VDQMHEDIISLWDQSLKPCVKLTPLCVTLDCDDVNTTNSTTTTSNGWTGEIRKGEIKNCSFNITTSIRDKV
QKEYALFYNLDVVPIDDDNATTKNKTTRNFRLIHCNSSVMTQACPKVSFEPIPIHYCAPAGFAILKCNNKT
FDGKGLCTNVSTVQCTHGIRPVVSTQLLLNGSLAEEEVVIRSDNFMDNTKTIIVQLNESVAINCTRPNNNT
RKGIHIGPGRAFYAARKIIGDIRQAHCNLSRAQWNNTLKQIVIKLREHFGNKTIKFNQSSGGDPEIVRHSF
NCGGEFFYCDTTQLFNSTWNGTEGNNTEGNSTITLPCRIKQIINMWQEVGKAMYAPPIGGQIRCSSNITGL
LLTRDGGTEGNGTENETEIFRPGGGDMRDNWRSELYKYKVVKVEPLGVAPTRAKRRVVQRMGARASVLSGG
ELDRWEKIRLRPGGKKKYKLKHIVWASRELERFAVNPGLLETSEGCRQILGQLQPSLQTGSEELRSLYNTV
ATLYCVHQRIEIKDTKEALDKIEEEQNKSKKKAQQAAADTGHSNQVSQNYPIVQNIQGQMVHQAISPRTLN
AWVKVVEEKAFSPEVIPMFSALSEGATPQDLNTMLNTVGGHQAAMQMLKETINEEAAEWDRVHPVHAGPIA
PGQMREPRGSDIAGTTSTLQEQIGWMTNNPPIPVGEIYKRWIILGLNKIVRMYSPTSILDIRQGPKEPFRD
YVDRFYKTLRAEQASQEVKNWMTETLLVQNANPDCKTILKALGPAATLEEMMTACQGVGGPGHKARVLMGK
WSKSSVVGWPTVRERMRRAEPAADGVGAASRDLEKHGAITSSNTAATNAACAWLEAQEEEVGFPVTPQVP
LRPMTYKAAVDLSHFLKEKGGLEGLIHSQRRQDILDLWIYHTQGYFPDWQNYTPGPGVRYPLTFGWCYKLV
PVEPDKVEEANKGENTSLLHPVSLHGMDDPEREVLEWRFDSRLAFHHVARELHPEYFKNCTSEPVDPRLEP
WKHPGSQPKTACTNCYCKKCCFHCQVCFITAALGISYGRKKRRQRRRPPQGSQTHQVSLSKQPTSQSKGEP
TGPKE [SEQ ID NO: 71]

# Fig.15.

pRix41

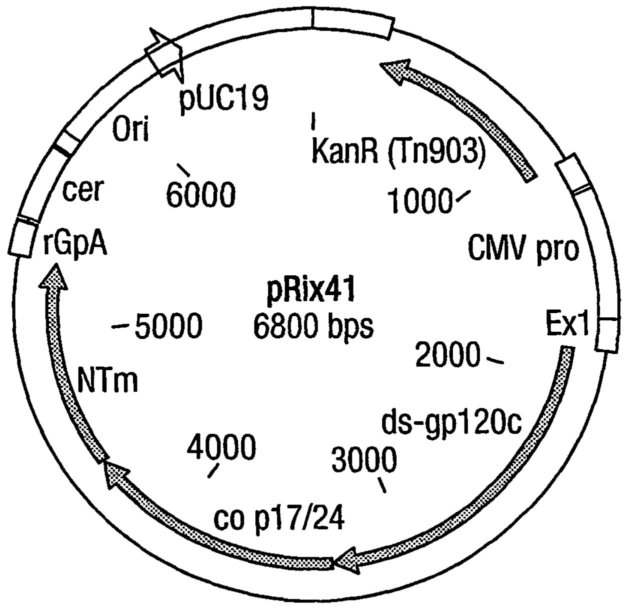


**DNA sequence of insert**

ATGGCCGAGCAGCTGTGGGTCACCGTCTACTACGGCGTGCCTGTGTGGAAGGAGGCCACGACCACCCTCTT
CTGCGCGAGCGACGCCAAGGCCTACGACACGGAAGTGCATAACGTGTGGGCGACGCATGCTTGCGTGCCTA
CGGACCCCAACCCCCAGGAGGTGGTGCTGGGAAACGTGACCGAGTACTTCAACATGTGGAAGAATAACATG
GTGGATCAGATGCACGAGGACATCATCTCTCTGTGGGACCAGTCCCTGAAGCCCTGCGTGAAGCTGACGCC
TCTCTGCGTGACACTGGACTGTGACGACGTCAACACCACCAACAGCACTACCACCACCAGCAACGGCTGGA
CCGGAGAGATTCGGAAGGGCGAGATCAAGAACTGCTCCTTCAATATCACGACCTCGATCAGAGACAAGGTG
CAGAAGGAATACGCGCTGTTTTATAATCTCGATGTGGTCCCCATCGACGACGACAATGCCACCACCAAGAA
CAAGACGACGCGTAATTTCAGACTCATTCACTGCAACAGCAGCGTCATGACGCAGGCCTGCCCCAAGGTGT
CCTTCGAACCAATCCCGATCCATTACTGTGCCCCTGCCGGATTCGCGATCCTCAAGTGTAACAACAAGACC
TTCGACGGGAAGGGCCTGTGCACCAACGTCAGCACGGTGCAGTGCACCCATGGCATCCGCCCCGTCGTGAG
CACCCAGCTGCTGCTGAACGGGTCCCTGGCTGAGGAGGAGGTGGTGATCCGGTCGGACAACTTCATGGACA
ACACCAAGACAATCATCGTCCAGCTGAACGAGTCTGTGGCGATTAACTGTACCCGGCCTAACAACAACACC
CGTAAGGGCATCCACATCGGGCCTGGACGGGCCTTCTATGCCGCCCGCAAGATCATCGGCGACATCCGGCA
GGCCCATTGCAACCTCTCCCGCGCCCAGTGGAATAACACCCTGAAGCAGATCGTGATCAAGCTGAGAGAGC
ACTTTGGAAACAAGACCATCAAGTTCAATCAGAGTTCTGGCGGAGACCCCGAGATCGTGCGGCACTCCTTC
AACTGCGGGGGCGAGTTCTTCTACTGCGATACGACACAGCTCTTCAACTCCACCTGGAACGGCACCGAGGG
CAACAACACAGAGGGAAACTCCACTATCACCCTCCCTTGCCGCATCAAGCAGATCATCAACATGTGGCAGG
AGGTGGGAAAGGCCATGTATGCCCCCCCCATCGGGGGCCAGATCCGCTGCTCCTCCAACATCACCGGCCTG
CTGCTCACCAGAGACGGGGGCACCGAGGGCAACGGCACGGAGAACGAGACGGAGATCTTCAGGCCCGGCGG
CGGCGACATGAGGGATAACTGGCGGAGCGAGCTGTACAAGTACAAGGTGGTGAAGGTGGAGCCGCTCGGCG
TGGCCCCCACCCGGGCCAAGCGCCGCGTCGTGCAGAGAATGGGTGCCCGAGCTTCGGTACTGTCTGGTGGA
GAGCTGGACAGATGGGAGAAAATTAGGCTGCGCCCGGGAGGCAAAAAGAAATACAAGCTCAAGCATATCGT
GTGGGCCTCGAGGGAGCTTGAACGGTTTGCCGTGAACCCAGGCCTGCTGGAAACATCTGAGGGATGTCGCC
AGATCCTGGGGCAATTGCAGCCATCCCTCCAGACCGGGAGTGAAGAGCTGAGGTCCTTGTATAACACAGTG
GCTACCCTCTACTGCGTACACCAGAGGATCGAGATTAAGGATACCAAGGAGGCCTTGGACAAAATTGAGGA
GGAGCAAAACAAGAGCAAGAAGAAGGCCCAGCAGGCAGCTGCTGACACTGGGCATAGCAACCAGGTATCAC
AGAACTATCCTATTGTCCAAAACATTCAGGGCCAGATGGTTCATCAGGCCATCAGCCCCCGGACGCTCAAT
GCCTGGGTGAAGGTTGTCGAAGAGAAGGCCTTTTCTCCTGAGGTTATCCCCATGTTCTCCGCTTTGAGTGA
GGGGGCCACTCCTCAGGACCTCAATACAATGCTTAATACCGTGGGCGGCCATCAGGCCGCCATGCAAATGT

# Fig.15 (Cont).

TGAAGGAGACTATCAACGAGGAGGCAGCCGAGTGGGACAGAGTGCATCCCGTCCACGCTGGCCCAATCGCG
CCCGGACAGATGCGGGAGCCTCGCGGCTCTGACATTGCCGGCACCACCTCTACACTGCAAGAGCAAATCGG
ATGGATGACCAACAATCCTCCCATCCCAGTTGGAGAAATCTATAAACGGTGGATCATTCTCGGTCTCAATA
AAATTGTTAGAATGTACTCTCCGACATCCATCCTTGACATTAGACAGGGACCCAAAGAGCCTTTTAGGGAT
TACGTCGACCGGTTTTATAAGACCCTGCGAGCAGAGCAGGCCTCTCAGGAGGTCAAAAACTGGATGACGGA
GACACTCCTGGTACAGAACGCTAACCCCGACTGCAAAACAATCTTGAAGGCACTAGGCCCGGCTGCCACCC
TGGAAGAGATGATGACCGCCTGTCAGGGAGTAGGCGGACCCGGACACAAAGCCAGAGTGTTGATGGGTGGC
AAGTGGTCAAAAAGTAGTGTGGTTGGATGGCCTACTGTAAGGGAAAGAATGAGACGAGCTGAGCCAGCAGC
AGATGGGGTGGGAGCAGCATCTCGAGACCTGGAAAAACATGGAGCAATCACAAGTAGCAATACAGCAGCTA
CCAATGCTGCTTGTGCCTGGCTAGAAGCACAAGAGGAGGAGGAGGTGGGTTTTCCAGTCACACCTCAGGTA
CCTTTAAGACCAATGACTTACAAGGCAGCTGTAGATCTTAGCCACTTTTTAAAAGAAAAGGGGGGACTGGA
AGGGCTAATTCACTCCCAACGAAGACAAGATATCCTTGATCTGTGGATCTACCACACACAAGGCTACTTCC
CTGATTGGCAGAACTACACACCAGGGCCAGGGGTCAGATATCCACTGACCTTTGGATGGTGCTACAAGCTA
GTACCAGTTGAGCCAGATAAGGTAGAAGAGGCCAATAAAGGAGAGAACACCAGCGCCTTACACCCTGTGAG
CCTGCATGGAATGGATGACCCTGAGAGAGAAGTGTTAGAGTGGAGGTTTGACAGCCGCCTAGCATTTCATC
ACGTGGCCCGAGAGCTGCATCCGGAGTACTTCAAGAACTGCACTAGTGAGCCAGTAGATCCTAGACTAGAG
CCCTGGAAGCATCCAGGAAGTCAGCCTAAAACTGCTTGTACCAATTGCTATTGTAAAAAGTGTTGCTTTCA
TTGCCAAGTTTGTTTCATAACAGCTGCCTTAGGCATCTCCTATGGCAGGAAGAAGCGGAGACAGCGACGAA
GACCTCCTCAAGGCAGTCAGACTCATCAAGTTTCTCTATCAAAGCAACCCACCTCCCAATCCAAAGGGGAG
CCGACAGGCCCGAAGGAATAA [SEQ ID NO: 72]

**Aminoacid sequence of insert**

MAEQLWVTVYYGVPVWKEATTTLFCASDAKAYDTEVHNVWATHACVPTDPNPQEVVLGNVTEYFNMWKNNM
VDQMHEDIISLWDQSLKPCVKLTPLCVTLDCDDVNTTNSTTTSNGWTGEIRKGEIKNCSFNITTSIRDKV
QKEYALFYNLDVVPIDDDNATTKNKTTRNFRLIHCNSSVMTQACPKVSFEPIPIHYCAPAGFAILKCNNKT
FDGKGLCTNVSTVQCTHGIRPVVSTQLLLNGSLAEEEVVIRSDNFMDNTKTIIVQLNESVAINCTRPNNNT
RKGIHIGPGRAFYAARKIIGDIRQAHCNLSRAQWNNTLKQIVIKLREHFGNKTIKFNQSSGGDPEIVRHSF
NCGGEFFYCDTTQLFNSTWNGTEGNNTEGNSTITLPCRIKQIINMWQEVGKAMYAPPIGGQIRCSSNITGL
LLTRDGGTEGNGTENETEIFRPGGGDMRDNWRSELYKYKVVKVEPLGVAPTRAKRRVVQRMGARASVLSGG
ELDRWEKIRLRPGGKKKYKLKHIVWASRELERFAVNPGLLETSEGCRQILGQLQPSLQTGSEELRSLYNTV
ATLYCVHQRIEIKDTKEALDKIEEEQNKSKKKAQQAAADTGHSNQVSQNYPIVQNIQGQMVHQAISPRTLN
AWVKVVEEKAFSPEVIPMFSALSEGATPQDLNTMLNTVGGHQAAMQMLKETINEEAAEWDRVHPVHAGPIA
PGQMREPRGSDIAGTTSTLQEQIGWMTNNPPIPVGEIYKRWIILGLNKIVRMYSPTSILDIRQGPKEPFRD
YVDRFYKTLRAEQASQEVKNWMTETLLVQNANPDCKTILKALGPAATLEEMMTACQGVGGPGHKARVLMGG
KWSKSSVVGWPTVRERMRRAEPAADGVGAASRDLEKHGAITSSNTAATNAACAWLEAQEEEEVGFPVTPQV
PLRPMTYKAAVDLSHFLKEKGGLEGLIHSQRRQDILDLWIYHTQGYFPDWQNYTPGPGVRYPLTFGWCYKL
VPVEPDKVEEANKGENTSALHPVSLHGMDDPEREVLEWRFDSRLAFHHVARELHPEYFKNCTSEPVDPRLE
PWKHPGSQPKTACTNCYCKKCCFHCQVCFITAALGISYGRKKRRQRRRPPQGSQTHQVSLSKQPTSQSKGE
PTGPKE [SEQ ID NO: 73]

Fig.16.

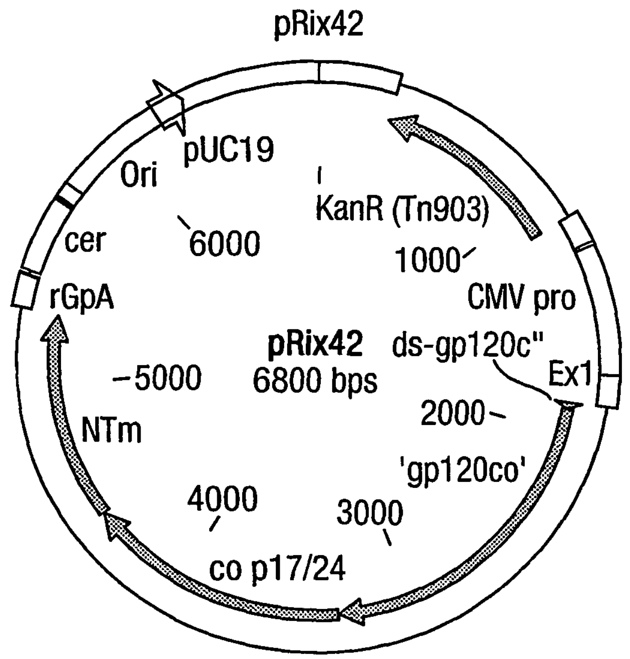


**DNA sequence of insert**

ATGGCCGAGCAGCTGTGGGTCACCGTCTACTACGGCGTGCCTGTGTGGAAGGAGGCCACGACCACCCTCTT
CTGCGCGAGCGACGCCAAGGCCTACGACACGGAAGTGCATAACGTGTGGGCGACGCATGCTTGCGTGCCTA
CGGACCCCAACCCCCAGGAGGTGGTGCTGGGAAACGTGACCGAGTACTTCAACATGTGGAAGAATAACATG
GTGGATCAGATGCACGAGGACATCATCTCTCTGTGGGACCAGTCCCTGAAGCCCTGCGTGAAGCTGACGCC
TCTCTGCGTGACACTGGACTGTGACGACGTCAACACCACCAACAGCACTACCACCACCAGCAACGGCTGGA
CCGGAGAGATTCGGAAGGGCGAGATCAAGAACTGCTCCTTCAATATCACGACCTCGATCAGAGACAAGGTG
CAGAAGGAATACGCGCTGTTTTATAATCTCGATGTGGTCCCCATCGACGACGACAATGCCACCACCAAGAA
CAAGACGACGCGTAATTTCAGACTCATTCACTGCAACAGCAGCGTCATGACGCAGGCCTGCCCCAAGGTGT
CCTTCGAACCAATCCCGATCCATTACTGTGCCCCTGCCGGATTCGCGATCCTCAAGTGTAACAACAAGACC
TTCGACGGGAAGGGCCTGTGCACCAACGTCAGCACGGTGCAGTGCACCCATGGCATCCGCCCCGTCGTGAG
CACCCAGCTGCTGCTGAACGGGTCCCTGGCTGAGGAGGAGGTGGTGATCCGGTCGGACAACTTCATGGACA
ACACCAAGACAATCATCGTCCAGCTGAACGAGTCTGTGGCGATTAACTGTACCCGGCCTAACAACAACACC
CGTAAGGGCATCCACATCGGGCCTGGACGGGCCTTCTATGCCGCCCGCAAGATCATCGGCGACATCCGGCA
GGCCCATTGCAACCTCTCCCGCGCCCAGTGGAATAACACCCTGAAGCAGATCGTGATCAAGCTGAGAGAGC
ACTTTGGAAACAAGACCATCAAGTTCAATCAGAGTTCTGGCGGAGACCCCGAGATCGTGCGGCACTCCTTC
AACTGCGGGGGCGAGTTCTTCTACTGCGATACGACACAGCTCTTCAACTCCACCTGGAACGGCACCGAGGG
CAACAACACAGAGGGAAACTCCACTATCACCCTCCCTTGCCGCATCAAGCAGATCATCAACATGTGGCAGG
AGGTGGGAAAGGCCATGTATGCCCCCCCCATCGGGGGCCAGATCCGCTGCTCCTCCAACATCACCGGCCTG
CTGCTCACCAGAGACGGGGGCACCGAGGGCAACGGCACGGAGAACGAGACGGAGATCTTCAGGCCCGGCGG
CGGCGACATGAGGGATAACTGGCGGAGCGAGCTGTACAAGTACAAGGTGGTGAAGGTGGAGCCGCTCGGCG
TGGCCCCCACCCGGGCCAAGCGCCGCGTCGTGCAGAGAATGGGTGCCCGAGCTTCGGTACTGTCTGGTGGA
GAGCTGGACAGATGGGAGAAAATTAGGCTGCGCCCGGGAGGCAAAAAGAAATACAAGCTCAAGCATATCGT
GTGGGCCTCGAGGGAGCTTGAACGGTTTGCCGTGAACCCAGGCCTGCTGGAAACATCTGAGGGATGTCGCC
AGATCCTGGGGCAATTGCAGCCATCCCTCCAGACCGGGAGTGAAGAGCTGAGGTCCTTGTATAACACAGTG
GCTACCCTCTACTGCGTACACCAGAGGATCGAGATTAAGGATACCAAGGAGGCCTTGGACAAAATTGAGGA
GGAGCAAAACAAGAGCAAGAAGAAGGCCCAGCAGGCAGCTGCTGACACTGGGCATAGCAACCAGGTATCAC
AGAACTATCCTATTGTCCAAAACATTCAGGGCCAGATGGTTCATCAGGCCATCAGCCCCCGGACGCTCAAT
GCCTGGGTGAAGGTTGTCGAAGAGAAGGCCTTTTCTCCTGAGGTTATCCCCATGTTCTCCGCTTTGAGTGA
GGGGGCCACTCCTCAGGACCTCAATACAATGCTTAATACCGTGGGCGGCCATCAGGCCGCCATGCAAATGT

# Fig.16 (Cont).

TGAAGGAGACTATCAACGAGGAGGCAGCCGAGTGGGACAGAGTGCATCCCGTCCACGCTGGCCCAATCGCG
CCCGGACAGATGCGGGAGCCTCGCGGCTCTGACATTGCCGGCACCACCTCTACACTGCAAGAGCAAATCGG
ATGGATGACCAACAATCCTCCCATCCCAGTTGGAGAAATCTATAAACGGTGGATCATTCTCGGTCTCAATA
AAATTGTTAGAATGTACTCTCCGACATCCATCCTTGACATTAGACAGGGACCCAAAGAGCCTTTTAGGGAT
TACGTCGACCGGTTTTATAAGACCCTGCGAGCAGAGCAGGCCTCTCAGGAGGTCAAAAACTGGATGACGGA
GACACTCCTGGTACAGAACGCTAACCCCGACTGCAAAACAATCTTGAAGGCACTAGGCCCGGCTGCCACCC
TGGAAGAGATGATGACCGCCTGTCAGGGAGTAGGCGGACCCGGACACAAAGCCAGAGTGTTGATGGGTGGC
AAGTGGTCAAAAAGTAGTGTGGTTGGATGGCCTACTGTAAGGGAAAGAATGAGACGAGCTGAGCCAGCAGC
AGATGGGGTGGGAGCAGCATCTCGAGACCTGGAAAAACATGGAGCAATCACAAGTAGCAATACAGCAGCTA
CCAATGCTGCTTGTGCCTGGCTAGAAGCACAAGAGGAGGAGGAGGTGGGTTTTCCAGTCACACCTCAGGTA
CCTTTAAGACCAATGACTTACAAGGCAGCTGTAGATCTTAGCCACTTTTTAAAAGAAAAGGGGGGACTGGA
AGGGCTAATTCACTCCCAACGAAGACAAGATATCCTTGATCTGTGGATCTACCACACACAAGGCTACTTCC
CTGATTGGCAGAACTACACACCAGGGCCAGGGGTCAGATATCCACTGACCTTTGGATGGTGCTACAAGCTA
GTACCAGTTGAGCCAGATAAGGTAGAAGAGGCCAATAAAGGAGAGAACACCAGCTTGGCACACCCTGTGAG
CCTGCATGGAATGGATGACCCTGAGAGAGAAGTGTTAGAGTGGAGGTTTGACAGCCGCCTAGCATTTCATC
ACGTGGCCCGAGAGCTGCATCCGGAGTACTTCAAGAACTGCACTAGTGAGCCAGTAGATCCTAGACTAGAG
CCCTGGAAGCATCCAGGAAGTCAGCCTAAAACTGCTTGTACCAATTGCTATTGTAAAAAGTGTTGCTTTCA
TTGCCAAGTTTGTTTCATAACAGCTGCCTTAGGCATCTCCTATGGCAGGAAGAAGCGGAGACAGCGACGAA
GACCTCCTCAAGGCAGTCAGACTCATCAAGTTTCTCTATCAAAGCAACCCACCTCCCAATCCAAAGGGGAG
CCGACAGGCCCGAAGGAATAA [SEQ ID NO: 74]

**Aminoacid sequence of insert**

MAEQLWVTVYYGVPVWKEATTTLFCASDAKAYDTEVHNVWATHACVPTDPNPQEVVLGNVTEYFNMWKNNM
VDQMHEDIISLWDQSLKPCVKLTPLCVTLDCDDVNTTNSTTTTSNGWTGEIRKGEIKNCSFNITTSIRDKV
QKEYALFYNLDVVPIDDDNATTKNKTTRNFRLIHCNSSVMTQACPKVSFEPIPIHYCAPAGFAILKCNNKT
FDGKGLCTNVSTVQCTHGIRPVVSTQLLLNGSLAEEEVVIRSDNFMDNTKTIIVQLNESVAINCTRPNNNT
RKGIHIGPGRAFYAARKIIGDIRQAHCNLSRAQWNNTLKQIVIKLREHFGNKTIKFNQSSGGDPEIVRHSF
NCGGEFFYCDTTQLFNSTWNGTEGNNTEGNSTITLPCRIKQIINMWQEVGKAMYAPPIGGQIRCSSNITGL
LLTRDGGTEGNGTENETEIFRPGGGDMRDNWRSELYKYKVVKVEPLGVAPTRAKRRVVQRMGARASVLSGG
ELDRWEKIRLRPGGKKKYKLKHIVWASRELERFAVNPGLLETSEGCRQILGQLQPSLQTGSEELRSLYNTV
ATLYCVHQRIEIKDTKEALDKIEEEQNKSKKKAQQAAADTGHSNQVSQNYPIVQNIQGQMVHQAISPRTLN
AWVKVVEEKAFSPEVIPMFSALSEGATPQDLNTMLNTVGGHQAAMQMLKETINEEAAEWDRVHPVHAGPIA
PGQMREPRGSDIAGTTSTLQEQIGWMTNNPPIPVGEIYKRWIILGLNKIVRMYSPTSILDIRQGPKEPFRD
YVDRFYKTLRAEQASQEVKNWMTETLLVQNANPDCKTILKALGPAATLEEMMTACQGVGGPGHKARVLMGG
KWSKSSVVGWPTVRERMRRAEPAADGVGAASRDLEKHGAITSSNTAATNAACAWLEAQEEEEVGFPVTPQV
PLRPMTYKAAVDLSHFLKEKGGLEGLIHSQRRQDILDLWIYHTQGYFPDWQNYTPGPGVRYPLTFGWCYKL
VPVEPDKVEEANKGENTSLAHPVSLHGMDDPEREVLEWRFDSRLAFHHVARELHPEYFKNCTSEPVDPRLE
PWKHPGSQPKTACTNCYCKKCCFHCQVCFITAALGISYGRKKRRQRRRPPQGSQTHQVSLSKQPTSQSKGE
PTGPKE [SEQ ID NO: 75]

Fig.17.

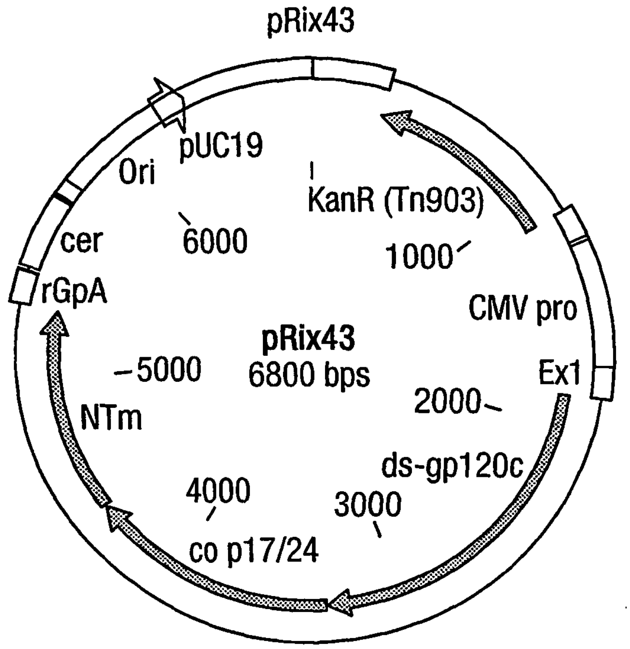


DNA sequence of insert

ATGGCCGAGCAGCTGTGGGTCACCGTCTACTACGGCGTGCCTGTGTGGAAGGAGGCCACGACCACCCTCTT
CTGCGCGAGCGACGCCAAGGCCTACGACACGGAAGTGCATAACGTGTGGGCGACGCATGCTTGCGTGCCTA
CGGACCCCAACCCCCAGGAGGTGGTGCTGGGAAACGTGACCGAGTACTTCAACATGTGGAAGAATAACATG
GTGGATCAGATGCACGAGGACATCATCTCTCTGTGGGACCAGTCCCTGAAGCCCTGCGTGAAGCTGACGCC
TCTCTGCGTGACACTGGACTGTGACGACGTCAACACCACCAACAGCACTACCACCACCAGCAACGGCTGGA
CCGGAGAGATTCGGAAGGGCGAGATCAAGAACTGCTCCTTCAATATCACGACCTCGATCAGAGACAAGGTG
CAGAAGGAATACGCGCTGTTTTATAATCTCGATGTGGTCCCCATCGACGACGACAATGCCACCACCAAGAA
CAAGACGACGCGTAATTTCAGACTCATTCACTGCAACAGCAGCGTCATGACGCAGGCCTGCCCCAAGGTGT
CCTTCGAACCAATCCCGATCCATTACTGTGCCCCTGCCGGATTCGCGATCCTCAAGTGTAACAACAAGACC
TTCGACGGGAAGGGCCTGTGCACCAACGTCAGCACGGTGCAGTGCACCCATGGCATCCGCCCCGTCGTGAG
CACCCAGCTGCTGCTGAACGGGTCCCTGGCTGAGGAGGAGGTGGTGATCCGGTCGGACAACTTCATGGACA
ACACCAAGACAATCATCGTCCAGCTGAACGAGTCTGTGGCGATTAACTGTACCCGGCCTAACAACAACACC
CGTAAGGGCATCCACATCGGGCCTGGACGGGCCTTCTATGCCGCCCGCAAGATCATCGGCGACATCCGGCA
GGCCCATTGCAACCTCTCCCGCGCCCAGTGGAATAACACCCTGAAGCAGATCGTGATCAAGCTGAGAGAGC
ACTTTGGAAACAAGACCATCAAGTTCAATCAGAGTTCTGGCGGAGACCCCGAGATCGTGCGGCACTCCTTC
AACTGCGGGGGCGAGTTCTTCTACTGCGATACGACACAGCTCTTCAACTCCACCTGGAACGGCACCGAGGG
CAACAACACAGAGGGAAACTCCACTATCACCCTCCCTTGCCGCATCAAGCAGATCATCAACATGTGGCAGG
AGGTGGGAAAGGCCATGTATGCCCCCCCCATCGGGGGCCAGATCCGCTGCTCCTCCAACATCACCGGCCTG
CTGCTCACCAGAGACGGGGGCACCGAGGGCAACGGCACGGAGAACGAGACGGAGATCTTCAGGCCCGGCGG
CGGCGACATGAGGGATAACTGGCGGAGCGAGCTGTACAAGTACAAGGTGGTGAAGGTGGAGCCGCTCGGCG
TGGCCCCCACCCGGGCCAAGCGCCGCGTCGTGCAGAGAATGGGTGCCCGAGCTTCGGTACTGTCTGGTGGA
GAGCTGGACAGATGGGAGAAAATTAGGCTGCGCCCGGGAGGCAAAAAGAAATACAAGCTCAAGCATATCGT
GTGGGCCTCGAGGGAGCTTGAACGGTTTGCCGTGAACCCAGGCCTGCTGGAAACATCTGAGGGATGTCGCC
AGATCCTGGGGCAATTGCAGCCATCCCTCCAGACCGGGAGTGAAGAGCTGAGGTCCTTGTATAACACAGTG
GCTACCCTCTACTGCGTACACCAGAGGATCGAGATTAAGGATACCAAGGAGGCCTTGGACAAAATTGAGGA
GGAGCAAAACAAGAGCAAGAAGAAGGCCCAGCAGGCAGCTGCTGACACTGGGCATAGCAACCAGGTATCAC
AGAACTATCCTATTGTCCAAAACATTCAGGGCCAGATGGTTCATCAGGCCATCAGCCCCCGGACGCTCAAT
GCCTGGGTGAAGGTTGTCGAAGAGAAGGCCTTTTCTCCTGAGGTTATCCCCATGTTCTCCGCTTTGAGTGA
GGGGGCCACTCCTCAGGACCTCAATACAATGCTTAATACCGTGGGCGGCCATCAGGCCGCCATGCAAATGT

# Fig.17 (Cont).

TGAAGGAGACTATCAACGAGGAGGCAGCCGAGTGGGACAGAGTGCATCCCGTCCACGCTGGCCCAATCGCG
CCCGGACAGATGCGGGAGCCTCGCGGCTCTGACATTGCCGGCACCACCTCTACACTGCAAGAGCAAATCGG
ATGGATGACCAACAATCCTCCCATCCCAGTTGGAGAAATCTATAAACGGTGGATCATTCTCGGTCTCAATA
AAATTGTTAGAATGTACTCTCCGACATCCATCCTTGACATTAGACAGGGACCCAAAGAGCCTTTTAGGGAT
TACGTCGACCGGTTTTATAAGACCCTGCGAGCAGAGCAGGCCTCTCAGGAGGTCAAAAACTGGATGACGGA
GACACTCCTGGTACAGAACGCTAACCCCGACTGCAAAACAATCTTGAAGGCACTAGGCCCGGCTGCCACCC
TGGAAGAGATGATGACCGCCTGTCAGGGAGTAGGCGGACCCGGACACAAAGCCAGAGTGTTGATGGGTGGC
AAGTGGTCAAAAAGTAGTGTGGTTGGATGGCCTACTGTAAGGGAAAGAATGAGACGAGCTGAGCCAGCAGC
AGATGGGGTGGGAGCAGCATCTCGAGACCTGGAAAAACATGGAGCAATCACAAGTAGCAATACAGCAGCTA
CCAATGCTGCTTGTGCCTGGCTAGAAGCACAAGAGGAGGAGGAGGTGGGTTTTCCAGTCACACCTCAGGTA
CCTTTAAGACCAATGACTTACAAGGCAGCTGTAGATCTTAGCCACTTTTTAAAAGAAAAGGGGGGACTGGA
AGGGCTAATTCACTCCCAACGAAGACAAGATATCCTTGATCTGTGGATCTACCACACACAAGGCTACTTCC
CTGATTGGCAGAACTACACACCAGGGCCAGGGGTCAGATATCCACTGACCTTTGGATGGTGCTACAAGCTA
GTACCAGTTGAGCCAGATAAGGTAGAAGAGGCCAATAAAGGAGAGAACACCAGCGCCGCACACCCTGTGAG
CCTGCATGGAATGGATGACCCTGAGAGAGAAGTGTTAGAGTGGAGGTTTGACAGCCGCCTAGCATTTCATC
ACGTGGCCCGAGAGCTGCATCCGGAGTACTTCAAGAACTGCACTAGTGAGCCAGTAGATCCTAGACTAGAG
CCCTGGAAGCATCCAGGAAGTCAGCCTAAAACTGCTTGTACCAATTGCTATTGTAAAAAGTGTTGCTTTCA
TTGCCAAGTTTGTTTCATAACAGCTGCCTTAGGCATCTCCTATGGCAGGAAGAAGCGGAGACAGCGACGAA
GACCTCCTCAAGGCAGTCAGACTCATCAAGTTTCTCTATCAAAGCAACCCACCTCCCAATCCAAAGGGGAG
CCGACAGGCCCGAAGGAATAA [SEQ ID NO: 76]

**Aminoacid sequence of insert**

MAEQLWVTVYYGVPVWKEATTTLFCASDAKAYDTEVHNVWATHACVPTDPNPQEVVLGNVTEYFNMWKNNM
VDQMHEDIISLWDQSLKPCVKLTPLCVTLDCDDVNTTNSTTTTSNGWTGEIRKGEIKNCSFNITTSIRDKV
QKEYALFYNLDVVPIDDDNATTKNKTTRNFRLIHCNSSVMTQACPKVSFEPIPIHYCAPAGFAILKCNNKT
FDGKGLCTNVSTVQCTHGIRPVVSTQLLLNGSLAEEEVVIRSDNFMDNTKTIIVQLNESVAINCTRPNNNT
RKGIHIGPGRAFYAARKIIGDIRQAHCNLSRAQWNNTLKQIVIKLREHFGNKTIKFNQSSGGDPEIVRHSF
NCGGEFFYCDTTQLFNSTWNGTEGNNTEGNSTITLPCRIKQIINMWQEVGKAMYAPPIGGQIRCSSNITGL
LLTRDGGTEGNGTENETEIFRPGGGDMRDNWRSELYKYKVVKVEPLGVAPTRAKRRVVQRMGARASVLSGG
ELDRWEKIRLRPGGKKKYKLKHIVWASRELERFAVNPGLLETSEGCRQILGQLQPSLQTGSEELRSLYNTV
ATLYCVHQRIEIKDTKEALDKIEEEQNKSKKKAQQAAADTGHSNQVSQNYPIVQNIQGQMVHQAISPRTLN
AWVKVVEEKAFSPEVIPMFSALSEGATPQDLNTMLNTVGGHQAAMQMLKETINEEAAEWDRVHPVHAGPIA
PGQMREPRGSDIAGTTSTLQEQIGWMTNNPPIPVGEIYKRWIILGLNKIVRMYSPTSILDIRQGPKEPFRD
YVDRFYKTLRAEQASQEVKNWMTETLLVQNANPDCKTILKALGPAATLEEMMTACQGVGGPGHKARVLMGG
KWSKSSVVGWPTVRERMRRAEPAADGVGAASRDLEKHGAITSSNTAATNAACAWLEAQEEEEVGFPVTPQV
PLRPMTYKAAVDLSHFLKEKGGLEGLIHSQRRQDILDLWIYHTQGYFPDWQNYTPGPGVRYPLTFGWCYKL
VPVEPDKVEEANKGENTSAAHPVSLHGMDDPEREVLEWRFDSRLAFHHVARELHPEYFKNCTSEPVDPRLE
PWKHPGSQPKTACTNCYCKKCCFHCQVCFITAALGISYGRKKRRQRRRPPQGSQTHQVSLSKQPTSQSKGE
PTGPKE [SEQ ID NO: 77]

# Fig.18.

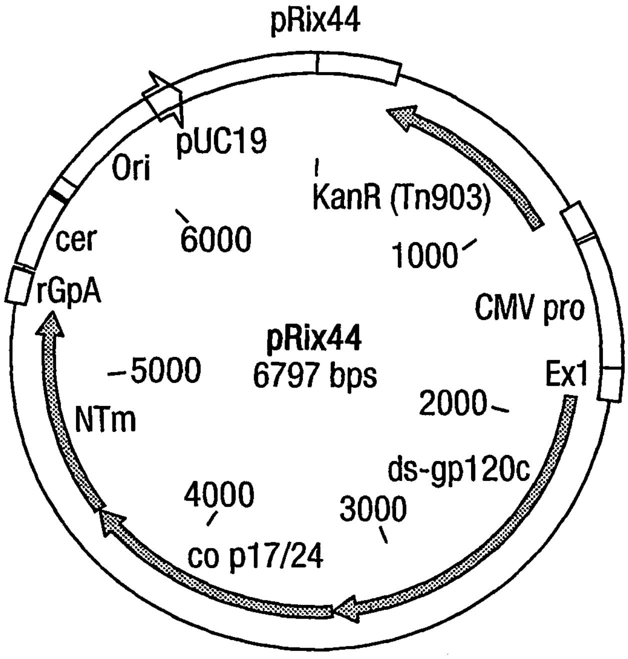


DNA sequence of insert

ATGGCCGAGCAGCTGTGGGTCACCGTCTACTACGGCGTGCCTGTGTGGAAGGAGGCCACGACCACCCTCTT
CTGCGCGAGCGACGCCAAGGCCTACGACACGGAAGTGCATAACGTGTGGGCGACGCATGCTTGCGTGCCTA
CGGACCCCAACCCCCAGGAGGTGGTGCTGGGAAACGTGACCGAGTACTTCAACATGTGGAAGAATAACATG
GTGGATCAGATGCACGAGGACATCATCTCTCTGTGGGACCAGTCCCTGAAGCCCTGCGTGAAGCTGACGCC
TCTCTGCGTGACACTGGACTGTGACGACGTCAACACCACCAACAGCACTACCACCACCAGCAACGGCTGGA
CCGGAGAGATTCGGAAGGGCGAGATCAAGAACTGCTCCTTCAATATCACGACCTCGATCAGAGACAAGGTG
CAGAAGGAATACGCGCTGTTTTATAATCTCGATGTGGTCCCCATCGACGACGACAATGCCACCACCAAGAA
CAAGACGACGCGTAATTTCAGACTCATTCACTGCAACAGCAGCGTCATGACGCAGGCCTGCCCCAAGGTGT
CCTTCGAACCAATCCCGATCCATTACTGTGCCCCTGCCGGATTCGCGATCCTCAAGTGTAACAACAAGACC
TTCGACGGGAAGGGCCTGTGCACCAACGTCAGCACGGTGCAGTGCACCCATGGCATCCGCCCCGTCGTGAG
CACCCAGCTGCTGCTGAACGGGTCCCTGGCTGAGGAGGAGGTGGTGATCCGGTCGGACAACTTCATGGACA
ACACCAAGACAATCATCGTCCAGCTGAACGAGTCTGTGGCGATTAACTGTACCCGGCCTAACAACAACACC
CGTAAGGGCATCCACATCGGGCCTGGACGGGCCTTCTATGCCGCCCGCAAGATCATCGGCGACATCCGGCA
GGCCCATTGCAACCTCTCCCGCGCCCAGTGGAATAACACCCTGAAGCAGATCGTGATCAAGCTGAGAGAGC
ACTTTGGAAACAAGACCATCAAGTTCAATCAGAGTTCTGGCGGAGACCCCGAGATCGTGCGGCACTCCTTC
AACTGCGGGGGCGAGTTCTTCTACTGCGATACGACACAGCTCTTCAACTCCACCTGGAACGGCACCGAGGG
CAACAACACAGAGGGAAACTCCACTATCACCCTCCCTTGCCGCATCAAGCAGATCATCAACATGTGGCAGG
AGGTGGGAAAGGCCATGTATGCCCCCCCCATCGGGGGCCAGATCCGCTGCTCCTCCAACATCACCGGCCTG
CTGCTCACCAGAGACGGGGGCACCGAGGGCAACGGCACGGAGAACGAGACGGAGATCTTCAGGCCCGGCGG
CGGCGACATGAGGGATAACTGGCGGAGCGAGCTGTACAAGTACAAGGTGGTGAAGGTGGAGCCGCTCGGCG
TGGCCCCCACCCGGGCCAAGCGCCGCGTCGTGCAGAGAATGGGTGCCCGAGCTTCGGTACTGTCTGGTGGA
GAGCTGGACAGATGGGAGAAAATTAGGCTGCGCCCGGGAGGCAAAAAGAAATACAAGCTCAAGCATATCGT
GTGGGCCTCGAGGGAGCTTGAACGGTTTGCCGTGAACCCAGGCCTGCTGGAAACATCTGAGGGATGTCGCC
AGATCCTGGGGCAATTGCAGCCATCCCTCCAGACCGGGAGTGAAGAGCTGAGGTCCTTGTATAACACAGTG
GCTACCCTCTACTGCGTACACCAGAGGATCGAGATTAAGGATACCAAGGAGGCCTTGGACAAAATTGAGGA
GGAGCAAAACAAGAGCAAGAAGAAGGCCCAGCAGGCAGCTGCTGACACTGGGCATAGCAACCAGGTATCAC
AGAACTATCCTATTGTCCAAAACATTCAGGGCCAGATGGTTCATCAGGCCATCAGCCCCCGGACGCTCAAT
GCCTGGGTGAAGGTTGTCGAAGAGAAGGCCTTTTCTCCTGAGGTTATCCCCATGTTCTCCGCTTTGAGTGA
GGGGGCCACTCCTCAGGACCTCAATACAATGCTTAATACCGTGGGCGGCCATCAGGCCGCCATGCAAATGT

# Fig.18 (Cont).

TGAAGGAGACTATCAACGAGGAGGCAGCCGAGTGGGACAGAGTGCATCCCGTCCACGCTGGCCCAATCGCG
CCCGGACAGATGCGGGAGCCTCGCGGCTCTGACATTGCCGGCACCACCTCTACACTGCAAGAGCAAATCGG
ATGGATGACCAACAATCCTCCCATCCCAGTTGGAGAAATCTATAAACGGTGGATCATTCTCGGTCTCAATA
AAATTGTTAGAATGTACTCTCCGACATCCATCCTTGACATTAGACAGGGACCCAAAGAGCCTTTTAGGGAT
TACGTCGACCGGTTTTATAAGACCCTGCGAGCAGAGCAGGCCTCTCAGGAGGTCAAAAACTGGATGACGGA
GACACTCCTGGTACAGAACGCTAACCCCGACTGCAAAACAATCTTGAAGGCACTAGGCCCGGCTGCCACCC
TGGAAGAGATGATGACCGCCTGTCAGGGAGTAGGCGGACCCGGACACAAAGCCAGAGTGTTGATGGGCAAG
TGGTCAAAAAGTAGTGTGGTTGGATGGCCTACTGTAAGGGAAAGAATGAGACGAGCTGAGCCAGCAGCAGA
TGGGGTGGGAGCAGCATCTCGAGACCTGGAAAAACATGGAGCAATCACAAGTAGCAATACAGCAGCTACCA
ATGCTGCTTGTGCCTGGCTAGAAGCACAAGAGGAGGAGGAGGTGGGTTTTCCAGTCACACCTCAGGTACCT
TTAAGACCAATGACTTACAAGGCAGCTGTAGATCTTAGCCACTTTTTAAAAGAAAAGGGGGGACTGGAAGG
GCTAATTCACTCCCAACGAAGACAAGATATCCTTGATCTGTGGATCTACCACACACAAGGCTACTTCCCTG
ATTGGCAGAACTACACACCAGGGCCAGGGGTCAGATATCCACTGACCTTTGGATGGTGCTACAAGCTAGTA
CCAGTTGAGCCAGATAAGGTAGAAGAGGCCAATAAAGGAGAGAACACCAGCGCCGCACACCCTGTGAGCCT
GCATGGAATGGATGACCCTGAGAGAGAAGTGTTAGAGTGGAGGTTTGACAGCCGCCTAGCATTTCATCACG
TGGCCCGAGAGCTGCATCCGGAGTACTTCAAGAACTGCACTAGTGAGCCAGTAGATCCTAGACTAGAGCCC
TGGAAGCATCCAGGAAGTCAGCCTAAAACTGCTTGTACCAATTGCTATTGTAAAAAGTGTTGCTTTCATTG
CCAAGTTTGTTTCATAACAGCTGCCTTAGGCATCTCCTATGGCAGGAAGAAGCGGAGACAGCGACGAAGAC
CTCCTCAAGGCAGTCAGACTCATCAAGTTTCTCTATCAAAGCAACCCACCTCCCAATCCAAAGGGGAGCCG
ACAGGCCCGAAGGAATAA [SEQ ID NO: 78]

**Aminoacid sequence of insert**

MAEQLWVTVYYGVPVWKEATTTLFCASDAKAYDTEVHNVWATHACVPTDPNPQEVVLGNVTEYFNMWKNNM
VDQMHEDIISLWDQSLKPCVKLTPLCVTLDCDDVNTTNSTTTSNGWTGEIRKGEIKNCSFNITTSIRDKV
QKEYALFYNLDVVPIDDDNATTKNKTTRNFRLIHCNSSVMTQACPKVSFEPIPIHYCAPAGFAILKCNNKT
FDGKGLCTNVSTVQCTHGIRPVVSTQLLLNGSLAEEEVVIRSDNFMDNTKTIIVQLNESVAINCTRPNNNT
RKGIHIGPGRAFYAARKIIGDIRQAHCNLSRAQWNNTLKQIVIKLREHFGNKTIKFNQSSGGDPEIVRHSF
NCGGEFFYCDTTQLFNSTWNGTEGNNTEGNSTITLPCRIKQIINMWQEVGKAMYAPPIGGQIRCSSNITGL
LLTRDGGTEGNGTENETEIFRPGGGDMRDNWRSELYKYKVVKVEPLGVAPTRAKRRVVQRMGARASVLSGG
ELDRWEKIRLRPGGKKKYKLKHIVWASRELERFAVNPGLLETSEGCRQILGQLQPSLQTGSEELRSLYNTV
ATLYCVHQRIEIKDTKEALDKIEEEQNKSKKKAQQAAADTGHSNQVSQNYPIVQNIQGQMVHQAISPRTLN
AWVKVVEEKAFSPEVIPMFSALSEGATPQDLNTMLNTVGGHQAAMQMLKETINEEAAEWDRVHPVHAGPIA
PGQMREPRGSDIAGTTSTLQEQIGWMTNNPPIPVGEIYKRWIILGLNKIVRMYSPTSILDIRQGPKEPFRD
YVDRFYKTLRAEQASQEVKNWMTETLLVQNANPDCKTILKALGPAATLEEMMTACQGVGGPGHKARVLMGK
WSKSSVVGWPTVRERMRRAEPAADGVGAASRDLEKHGAITSSNTAATNAACAWLEAQEEEEVGFPVTPQVP
LRPMTYKAAVDLSHFLKEKGGLEGLIHSQRRQDILDLWIYHTQGYFPDWQNYTPGPGVRYPLTFGWCYKLV
PVEPDKVEEANKGENTSAAHPVSLHGMDDPEREVLEWRFDSRLAFHHVARELHPEYFKNCTSEPVDPRLEP
WKHPGSQPKTACTNCYCKKCCFHCQVCFITAALGISYGRKKRRQRRRPPQGSQTHQVSLSKQPTSQSKGEP
TGPKE [SEQ ID NO: 79]

# Fig.19.

pRix46

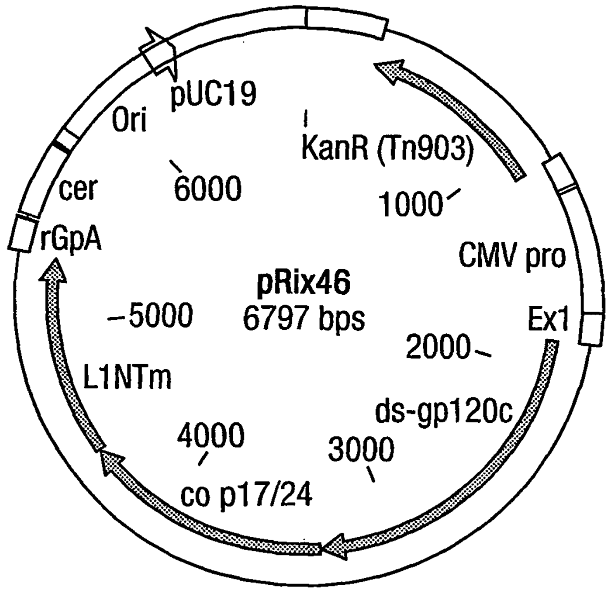


**DNA sequence of insert**

ATGGCCGAGCAGCTGTGGGTCACCGTCTACTACGGCGTGCCTGTGTGGAAGGAGGCCACGACCACCCTCTT
CTGCGCGAGCGACGCCAAGGCCTACGACACGGAAGTGCATAACGTGTGGGCGACGCATGCTTGCGTGCCTA
CGGACCCCAACCCCCAGGAGGTGGTGCTGGGAAACGTGACCGAGTACTTCAACATGTGGAAGAATAACATG
GTGGATCAGATGCACGAGGACATCATCTCTCTGTGGGACCAGTCCCTGAAGCCCTGCGTGAAGCTGACGCC
TCTCTGCGTGACACTGGACTGTGACGACGTCAACACCACCAACAGCACTACCACCACCAGCAACGGCTGGA
CCGGAGAGATTCGGAAGGGCGAGATCAAGAACTGCTCCTTCAATATCACGACCTCGATCAGAGACAAGGTG
CAGAAGGAATACGCGCTGTTTTATAATCTCGATGTGGTCCCCATCGACGACGACAATGCCACCACCAAGAA
CAAGACGACGCGTAATTTCAGACTCATTCACTGCAACAGCAGCGTCATGACGCAGGCCTGCCCCAAGGTGT
CCTTCGAACCAATCCCGATCCATTACTGTGCCCCTGCCGGATTCGCGATCCTCAAGTGTAACAACAAGACC
TTCGACGGGAAGGGCCTGTGCACCAACGTCAGCACGGTGCAGTGCACCCATGGCATCCGCCCCGTCGTGAG
CACCCAGCTGCTGCTGAACGGGTCCCTGGCTGAGGAGGAGGTGGTGATCCGGTCGGACAACTTCATGGACA
ACACCAAGACAATCATCGTCCAGCTGAACGAGTCTGTGGCGATTAACTGTACCCGGCCTAACAACAACACC
CGTAAGGGCATCCACATCGGGCCTGGACGGGCCTTCTATGCCGCCCGCAAGATCATCGGCGACATCCGGCA
GGCCCATTGCAACCTCTCCCGCGCCCAGTGGAATAACACCCTGAAGCAGATCGTGATCAAGCTGAGAGAGC
ACTTTGGAAACAAGACCATCAAGTTCAATCAGAGTTCTGGCGGAGACCCCGAGATCGTGCGGCACTCCTTC
AACTGCGGGGGCGAGTTCTTCTACTGCGATACGACACAGCTCTTCAACTCCACCTGGAACGGCACCGAGGG
CAACAACACAGAGGGAAACTCCACTATCACCCTCCCTTGCCGCATCAAGCAGATCATCAACATGTGGCAGG
AGGTGGGAAAGGCCATGTATGCCCCCCCCATCGGGGGCCAGATCCGCTGCTCCTCCAACATCACCGGCCTG
CTGCTCACCAGAGACGGGGGCACCGAGGGCAACGGCACGGAGAACGAGACGGAGATCTTCAGGCCCGGCGG
CGGCGACATGAGGGATAACTGGCGGAGCGAGCTGTACAAGTACAAGGTGGTGAAGGTGGAGCCGCTCGGCG
TGGCCCCCACCCGGGCCAAGCGCCGCGTCGTGCAGAGAATGGGTGCCCGAGCTTCGGTACTGTCTGGTGGA
GAGCTGGACAGATGGGAGAAAATTAGGCTGCGCCCGGGAGGCAAAAAGAAATACAAGCTCAAGCATATCGT
GTGGGCCTCGAGGGAGCTTGAACGGTTTGCCGTGAACCCAGGCCTGCTGGAAACATCTGAGGGATGTCGCC
AGATCCTGGGGCAATTGCAGCCATCCCTCCAGACCGGGAGTGAAGAGCTGAGGTCCTTGTATAACACAGTG
GCTACCCTCTACTGCGTACACCAGAGGATCGAGATTAAGGATACCAAGGAGGCCTTGGACAAAATTGAGGA
GGAGCAAAACAAGAGCAAGAAGAAGGCCCAGCAGGCAGCTGCTGACACTGGGCATAGCAACCAGGTATCAC
AGAACTATCCTATTGTCCAAAACATTCAGGGCCAGATGGTTCATCAGGCCATCAGCCCCCGGACGCTCAAT
GCCTGGGTGAAGGTTGTCGAAGAGAAGGCCTTTTCTCCTGAGGTTATCCCCATGTTCTCCGCTTTGAGTGA
GGGGGCCACTCCTCAGGACCTCAATACAATGCTTAATACCGTGGGCGGCCATCAGGCCGCCATGCAAATGT

# Fig.19 (Cont).

TGAAGGAGACTATCAACGAGGAGGCAGCCGAGTGGGACAGAGTGCATCCCGTCCACGCTGGCCCAATCGCG
CCCGGACAGATGCGGGAGCCTCGCGGCTCTGACATTGCCGGCACCACCTCTACACTGCAAGAGCAAATCGG
ATGGATGACCAACAATCCTCCCATCCCAGTTGGAGAAATCTATAAACGGTGGATCATTCTCGGTCTCAATA
AAATTGTTAGAATGTACTCTCCGACATCCATCCTTGACATTAGACAGGGACCCAAAGAGCCTTTTAGGGAT
TACGTCGACCGGTTTTATAAGACCCTGCGAGCAGAGCAGGCCTCTCAGGAGGTCAAAAACTGGATGACGGA
GACACTCCTGGTACAGAACGCTAACCCCGACTGCAAAACAATCTTGAAGGCACTAGGCCCGGCTGCCACCC
TGGAAGAGATGATGACCGCCTGTCAGGGAGTAGGCGGACCCGGACACAAAGCCAGAGTGTTGATGGGCAAG
TGGTCAAAAAGTAGTGTGGTTGGATGGCCTACTGTAAGGGAAAGAATGAGACGAGCTGAGCCAGCAGCAGA
TGGGGTGGGAGCAGCATCTCGAGACCTGGAAAAACATGGAGCAATCACAAGTAGCAATACAGCAGCTACCA
ATGCTGCTTGTGCCTGGCTAGAAGCACAAGAGGAGGAGGAGGTGGGTTTTCCAGTCACACCTCAGGTACCT
TTAAGACCAATGACTTACAAGGCAGCTGTAGATCTTAGCCACTTTTTAAAAGAAAAGGGGGGACTGGAAGG
GCTAATTCACTCCCAACGAAGACAAGATATCCTTGATCTGTGGATCTACCACACACAAGGCTACTTCCCTG
ATTGGCAGAACTACACACCAGGGCCAGGGGTCAGATATCCACTGACCTTTGGATGGTGCTACAAGCTAGTA
CCAGTTGAGCCAGATAAGGTAGAAGAGGCCAATAAAGGAGAGAACACCAGCGCCTTACACCCTGTGAGCCT
GCATGGAATGGATGACCCTGAGAGAGAAGTGTTAGAGTGGAGGTTTGACAGCCGCCTAGCATTTCATCACG
TGGCCCGAGAGCTGCATCCGGAGTACTTCAAGAACTGCACTAGTGAGCCAGTAGATCCTAGACTAGAGCCC
TGGAAGCATCCAGGAAGTCAGCCTAAAACTGCTTGTACCAATTGCTATTGTAAAAAGTGTTGCTTTCATTG
CCAAGTTTGTTTCATAACAGCTGCCTTAGGCATCTCCTATGGCAGGAAGAAGCGGAGACAGCGACGAAGAC
CTCCTCAAGGCAGTCAGACTCATCAAGTTTCTCTATCAAAGCAACCCACCTCCCAATCCAAAGGGGAGCCG
ACAGGCCCGAAGGAATAA [SEQ ID NO: 80]

**Aminoacid sequence of insert**

MAEQLWVTVYYGVPVWKEATTTLFCASDAKAYDTEVHNVWATHACVPTDPNPQEVVLGNVTEYFNMWKNNM
VDQMHEDIISLWDQSLKPCVKLTPLCVTLDCDDVNTTNSTTTTSNGWTGEIRKGEIKNCSFNITTSIRDKV
QKEYALFYNLDVVPIDDDNATTKNKTTRNFRLIHCNSSVMTQACPKVSFEPIPIHYCAPAGFAILKCNNKT
FDGKGLCTNVSTVQCTHGIRPVVSTQLLLNGSLAEEEVVIRSDNFMDNTKTIIVQLNESVAINCTRPNNNT
RKGIHIGPGRAFYAARKIIGDIRQAHCNLSRAQWNNTLKQIVIKLREHFGNKTIKFNQSSGGDPEIVRHSF
NCGGEFFYCDTTQLFNSTWNGTEGNNTEGNSTITLPCRIKQIINMWQEVGKAMYAPPIGGQIRCSSNITGL
LLTRDGGTEGNGTENETEIFRPGGGDMRDNWRSELYKYKVVKVEPLGVAPTRAKRRVVQRMGARASVLSGG
ELDRWEKIRLRPGGKKKYKLKHIVWASRELERFAVNPGLLETSEGCRQILGQLQPSLQTGSEELRSLYNTV
ATLYCVHQRIEIKDTKEALDKIEEEQNKSKKKAQQAAADTGHSNQVSQNYPIVQNIQGQMVHQAISPRTLN
AWVKVVEEKAFSPEVIPMFSALSEGATPQDLNTMLNTVGGHQAAMQMLKETINEEAAEWDRVHPVHAGPIA
PGQMREPRGSDIAGTTSTLQEQIGWMTNNPPIPVGEIYKRWIILGLNKIVRMYSPTSILDIRQGPKEPFRD
YVDRFYKTLRAEQASQEVKNWMTETLLVQNANPDCKTILKALGPAATLEEMMTACQGVGGPGHKARVLMGK
WSKSSVVGWPTVRERMRRAEPAADGVGAASRDLEKHGAITSSNTAATNAACAWLEAQEEEEVGFPVTPQVP
LRPMTYKAAVDLSHFLKEKGGLEGLIHSQRRQDILDLWIYHTQGYFPDWQNYTPGPGVRYPLTFGWCYKLV
PVEPDKVEEANKGENTSALHPVSLHGMDDPEREVLEWRFDSRLAFHHVARELHPEYFKNCTSEPVDPRLEP
WKHPGSQPKTACTNCYCKKCCFHCQVCFITAALGISYGRKKRRQRRRPPQGSQTHQVSLSKQPTSQSKGEP
TGPKE [SEQ ID NO: 81]

# Fig.20.

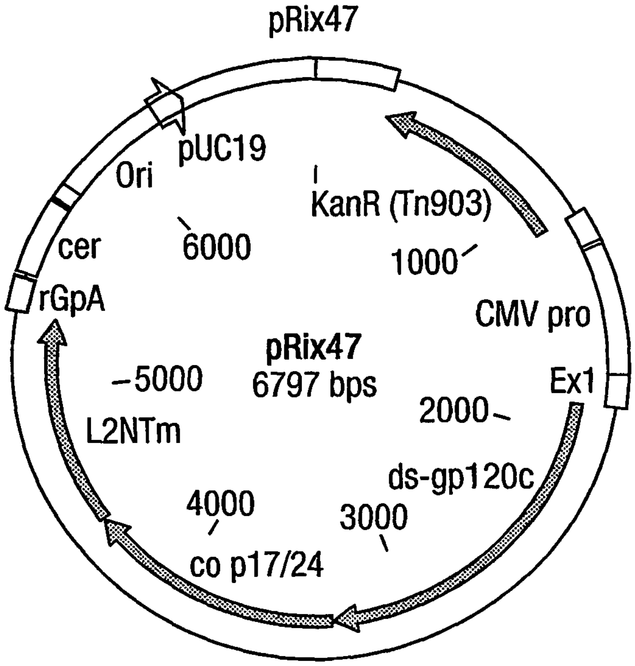


**DNA sequence of insert**

ATGGCCGAGCAGCTGTGGGTCACCGTCTACTACGGCGTGCCTGTGTGGAAGGAGGCCACGACCACCCTCTT
CTGCGCGAGCGACGCCAAGGCCTACGACACGGAAGTGCATAACGTGTGGGCGACGCATGCTTGCGTGCCTA
CGGACCCCAACCCCCAGGAGGTGGTGCTGGGAAACGTGACCGAGTACTTCAACATGTGGAAGAATAACATG
GTGGATCAGATGCACGAGGACATCATCTCTCTGTGGGACCAGTCCCTGAAGCCCTGCGTGAAGCTGACGCC
TCTCTGCGTGACACTGGACTGTGACGACGTCAACACCACCAACAGCACTACCACCACCAGCAACGGCTGGA
CCGGAGAGATTCGGAAGGGCGAGATCAAGAACTGCTCCTTCAATATCACGACCTCGATCAGAGACAAGGTG
CAGAAGGAATACGCGCTGTTTTATAATCTCGATGTGGTCCCCATCGACGACGACAATGCCACCACCAAGAA
CAAGACGACGCGTAATTTCAGACTCATTCACTGCAACAGCAGCGTCATGACGCAGGCCTGCCCCAAGGTGT
CCTTCGAACCAATCCCGATCCATTACTGTGCCCCTGCCGGATTCGCGATCCTCAAGTGTAACAACAAGACC
TTCGACGGGAAGGGCCTGTGCACCAACGTCAGCACGGTGCAGTGCACCCATGGCATCCGCCCCGTCGTGAG
CACCCAGCTGCTGCTGAACGGGTCCCTGGCTGAGGAGGAGGTGGTGATCCGGTCGGACAACTTCATGGACA
ACACCAAGACAATCATCGTCCAGCTGAACGAGTCTGTGGCGATTAACTGTACCCGGCCTAACAACAACACC
CGTAAGGGCATCCACATCGGGCCTGGACGGGCCTTCTATGCCGCCCGCAAGATCATCGGCGACATCCGGCA
GGCCCATTGCAACCTCTCCCGCGCCCAGTGGAATAACACCCTGAAGCAGATCGTGATCAAGCTGAGAGAGC
ACTTTGGAAACAAGACCATCAAGTTCAATCAGAGTTCTGGCGGAGACCCCGAGATCGTGCGGCACTCCTTC
AACTGCGGGGGCGAGTTCTTCTACTGCGATACGACACAGCTCTTCAACTCCACCTGGAACGGCACCGAGGG
CAACAACACAGAGGGAAACTCCACTATCACCCTCCCTTGCCGCATCAAGCAGATCATCAACATGTGGCAGG
AGGTGGGAAAGGCCATGTATGCCCCCCCCATCGGGGGCCAGATCCGCTGCTCCTCCAACATCACCGGCCTG
CTGCTCACCAGAGACGGGGGCACCGAGGGCAACGGCACGGAGAACGAGACGGAGATCTTCAGGCCCGGCGG
CGGCGACATGAGGGATAACTGGCGGAGCGAGCTGTACAAGTACAAGGTGGTGAAGGTGGAGCCGCTCGGCG
TGGCCCCCACCCGGGCCAAGCGCCGCGTCGTGCAGAGAATGGGTGCCCGAGCTTCGGTACTGTCTGGTGGA
GAGCTGGACAGATGGGAGAAAATTAGGCTGCGCCCGGGAGGCAAAAAGAAATACAAGCTCAAGCATATCGT
GTGGGCCTCGAGGGAGCTTGAACGGTTTGCCGTGAACCCAGGCCTGCTGGAAACATCTGAGGGATGTCGCC
AGATCCTGGGGCAATTGCAGCCATCCCTCCAGACCGGGAGTGAAGAGCTGAGGTCCTTGTATAACACAGTG
GCTACCCTCTACTGCGTACACCAGAGGATCGAGATTAAGGATACCAAGGAGGCCTTGGACAAAATTGAGGA
GGAGCAAAACAAGAGCAAGAAGAAGGCCCAGCAGGCAGCTGCTGACACTGGGCATAGCAACCAGGTATCAC
AGAACTATCCTATTGTCCAAAACATTCAGGGCCAGATGGTTCATCAGGCCATCAGCCCCCGGACGCTCAAT
GCCTGGGTGAAGGTTGTCGAAGAGAAGGCCTTTTCTCCTGAGGTTATCCCCATGTTCTCCGCTTTGAGTGA
GGGGGCCACTCCTCAGGACCTCAATACAATGCTTAATACCGTGGGCGGCCATCAGGCCGCCATGCAAATGT

# Fig.20 (Cont).

TGAAGGAGACTATCAACGAGGAGGCAGCCGAGTGGGACAGAGTGCATCCCGTCCACGCTGGCCCAATCGCG
CCCGGACAGATGCGGGAGCCTCGCGGCTCTGACATTGCCGGCACCACCTCTACACTGCAAGAGCAAATCGG
ATGGATGACCAACAATCCTCCCATCCCAGTTGGAGAAATCTATAAACGGTGGATCATTCTCGGTCTCAATA
AAATTGTTAGAATGTACTCTCCGACATCCATCCTTGACATTAGACAGGGACCCAAAGAGCCTTTTAGGGAT
TACGTCGACCGGTTTTATAAGACCCTGCGAGCAGAGCAGGCCTCTCAGGAGGTCAAAAACTGGATGACGGA
GACACTCCTGGTACAGAACGCTAACCCCGACTGCAAAACAATCTTGAAGGCACTAGGCCCGGCTGCCACCC
TGGAAGAGATGATGACCGCCTGTCAGGGAGTAGGCGGACCCGGACACAAAGCCAGAGTGTTGATGGGCAAG
TGGTCAAAAAGTAGTGTGGTTGGATGGCCTACTGTAAGGGAAAGAATGAGACGAGCTGAGCCAGCAGCAGA
TGGGGTGGGAGCAGCATCTCGAGACCTGGAAAAACATGGAGCAATCACAAGTAGCAATACAGCAGCTACCA
ATGCTGCTTGTGCCTGGCTAGAAGCACAAGAGGAGGAGGAGGTGGGTTTTCCAGTCACACCTCAGGTACCT
TTAAGACCAATGACTTACAAGGCAGCTGTAGATCTTAGCCACTTTTTAAAAGAAAAGGGGGGACTGGAAGG
GCTAATTCACTCCCAACGAAGACAAGATATCCTTGATCTGTGGATCTACCACACACAAGGCTACTTCCCTG
ATTGGCAGAACTACACACCAGGGCCAGGGGTCAGATATCCACTGACCTTTGGATGGTGCTACAAGCTAGTA
CCAGTTGAGCCAGATAAGGTAGAAGAGGCCAATAAAGGAGAGAACACCAGCTTGGCACACCCTGTGAGCCT
GCATGGAATGGATGACCCTGAGAGAGAAGTGTTAGAGTGGAGGTTTGACAGCCGCCTAGCATTTCATCACG
TGGCCCGAGAGCTGCATCCGGAGTACTTCAAGAACTGCACTAGTGAGCCAGTAGATCCTAGACTAGAGCCC
TGGAAGCATCCAGGAAGTCAGCCTAAAACTGCTTGTACCAATTGCTATTGTAAAAAGTGTTGCTTTCATTG
CCAAGTTTGTTTCATAACAGCTGCCTTAGGCATCTCCTATGGCAGGAAGAAGCGGAGACAGCGACGAAGAC
CTCCTCAAGGCAGTCAGACTCATCAAGTTTCTCTATCAAAGCAACCCACCTCCCAATCCAAAGGGGAGCCG
ACAGGCCCGAAGGAATAA [SEQ ID NO: 82]

**Aminoacid sequence of insert**

MAEQLWVTVYYGVPVWKEATTTLFCASDAKAYDTEVHNVWATHACVPTDPNPQEVVLGNVTEYFNMWKNNM
VDQMHEDIISLWDQSLKPCVKLTPLCVTLDCDDVNTTNSTTTTSNGWTGEIRKGEIKNCSFNITTSIRDKV
QKEYALFYNLDVVPIDDDNATTKNKTTRNFRLIHCNSSVMTQACPKVSFEPIPIHYCAPAGFAILKCNNKT
FDGKGLCTNVSTVQCTHGIRPVVSTQLLLNGSLAEEEVVIRSDNFMDNTKTIIVQLNESVAINCTRPNNNT
RKGIHIGPGRAFYAARKIIGDIRQAHCNLSRAQWNNTLKQIVIKLREHFGNKTIKFNQSSGGDPEIVRHSF
NCGGEFFYCDTTQLFNSTWNGTEGNNTEGNSTITLPCRIKQIINMWQEVGKAMYAPPIGGQIRCSSNITGL
LLTRDGGTEGNGTENETEIFRPGGGDMRDNWRSELYKYKVVKVEPLGVAPTRAKRRVVQRMGARASVLSGG
ELDRWEKIRLRPGGKKKYKLKHIVWASRELERFAVNPGLLETSEGCRQILGQLQPSLQTGSEELRSLYNTV
ATLYCVHQRIEIKDTKEALDKIEEEQNKSKKKAQQAAADTGHSNQVSQNYPIVQNIQGQMVHQAISPRTLN
AWVKVVEEKAFSPEVIPMFSALSEGATPQDLNTMLNTVGGHQAAMQMLKETINEEAAEWDRVHPVHAGPIA
PGQMREPRGSDIAGTTSTLQEQIGWMTNNPPIPVGEIYKRWIILGLNKIVRMYSPTSILDIRQGPKEPFRD
YVDRFYKTLRAEQASQEVKNWMTETLLVQNANPDCKTILKALGPAATLEEMMTACQGVGGPGHKARVLMGK
WSKSSVVGWPTVRERMRRAEPAADGVGAASRDLEKHGAITSSNTAATNAACAWLEAQEEEEVGFPVTPQVP
LRPMTYKAAVDLSHFLKEKGGLEGLIHSQRRQDILDLWIYHTQGYFPDWQNYTPGPGVRYPLTFGWCYKLV
PVEPDKVEEANKGENTSLAHPVSLHGMDDPEREVLEWRFDSRLAFHHVARELHPEYFKNCTSEPVDPRLEP
WKHPGSQPKTACTNCYCKKCCFHCQVCFITAALGISYGRKKRRQRRRPPQGSQTHQVSLSKQPTSQSKGEP
TGPKE [SEQ ID NO: 83]

# Fig.21.

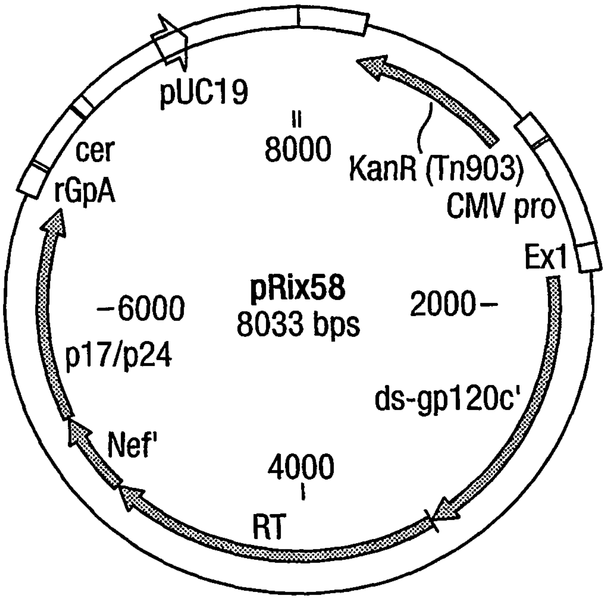


DNA sequence of insert

ATGGCCGAGCAGCTGTGGGTCACCGTCTACTACGGCGTGCCTGTGTGGAAGGAGGCCACGACCACCCTCTT
CTGCGCGAGCGACGCCAAGGCCTACGACACGGAAGTGCATAACGTGTGGGCGACGCATGCTTGCGTGCCTA
CGGACCCCAACCCCCAGGAGGTGGTGCTGGGAAACGTGACCGAGTACTTCAACATGTGGAAGAATAACATG
GTGGATCAGATGCACGAGGACATCATCTCTCTGTGGGACCAGTCCCTGAAGCCCTGCGTGAAGCTGACGCC
TCTCTGCGTGACACTGGACTGTGACGACGTCAACACCACCAACAGCACTACCACCACCAGCAACGGCTGGA
CCGGAGAGATTCGGAAGGGCGAGATCAAGAACTGCTCCTTCAATATCACGACCTCGATCAGAGACAAGGTG
CAGAAGGAATACGCGCTGTTTTATAATCTCGATGTGGTCCCCATCGACGACGACAATGCCACCACCAAGAA
CAAGACGACGCGTAATTTCAGACTCATTCACTGCAACAGCAGCGTCATGACGCAGGCCTGCCCCAAGGTGT
CCTTCGAACCAATCCCGATCCATTACTGTGCCCCTGCCGGATTCGCGATCCTCAAGTGTAACAACAAGACC
TTCGACGGGAAGGGCCTGTGCACCAACGTCAGCACGGTGCAGTGCACCCATGGCATCCGCCCCGTCGTGAG
CACCCAGCTGCTGCTGAACGGGTCCCTGGCTGAGGAGGAGGTGGTGATCCGGTCGGACAACTTCATGGACA
ACACCAAGACAATCATCGTCCAGCTGAACGAGTCTGTGGCGATTAACTGTACCCGGCCTAACAACAACACC
CGTAAGGGCATCCACATCGGGCCTGGACGGGCCTTCTATGCCGCCCGCAAGATCATCGGCGACATCCGGCA
GGCCCATTGCAACCTCTCCCGCGCCCAGTGGAATAACACCCTGAAGCAGATCGTGATCAAGCTGAGAGAGC
ACTTTGGAAACAAGACCATCAAGTTCAATCAGAGTTCTGGCGGAGACCCCGAGATCGTGCGGCACTCCTTC
AACTGCGGGGGCGAGTTCTTCTACTGCGATACGACACAGCTCTTCAACTCCACCTGGAACGGCACCGAGGG
CAACAACACAGAGGGAAACTCCACTATCACCCTCCCTTGCCGCATCAAGCAGATCATCAACATGTGGCAGG
AGGTGGGAAAGGCCATGTATGCCCCCCCCATCGGGGGCCAGATCCGCTGCTCCTCCAACATCACCGGCCTG
CTGCTCACCAGAGACGGGGGCACCGAGGGCAACGGCACGGAGAACGAGACGGAGATCTTCAGGCCCGGCGG
CGGCGACATGAGGGATAACTGGCGGAGCGAGCTGTACAAGTACAAGGTGGTGAAGGTGGAGCCGCTCGGCG
TGGCCCCCACCCGGGCCAAGCGCCGCGTCGTGCAGAGAATGGGCCCCATCAGTCCCATCGAGACCGTGCCG
GTGAAGCTGAAACCCGGGATGGACGGCCCCAAGGTCAAGCAGTGGCCACTCACCGAGGAGAAGATCAAGGC
CCTGGTGGAGATCTGCACCGAGATGGAGAAAGAGGGCAAGATCAGCAAGATCGGGCCTGAGAACCCATACA
ACACCCCCGTGTTTGCCATCAAGAAGAAGGACAGCACCAAGTGGCGCAAGCTGGTGGATTTCCGGGAGCTG
AATAAGCGGACCCAGGATTTCTGGGAGGTCCAGCTGGGCATCCCCCATCCGGCCGGCCTGAAGAAGAAGAA
GAGCGTGACCGTGCTGGACGTGGGCGACGCTTACTTCAGCGTCCCTCTGGACGAGGACTTTAGAAAGTACA
CCGCCTTTACCATCCCATCTATCAACAACGAGACCCCTGGCATCAGATATCAGTACAACGTCCTCCCCCAG
GGCTGGAAGGGCTCTCCCGCCATTTTCCAGAGCTCCATGACCAAGATCCTGGAGCCGTTTCGGAAGCAGAA
CCCCGATATCGTCATCTACCAGTACATGGACGACCTGTACGTGGGCTCTGACCTGGAAATCGGGCAGCATC

# Fig.21 (Cont I).

GCACGAAGATTGAGGAGCTGAGGCAGCATCTGCTGAGATGGGGCCTGACCACTCCGGACAAGAAGCATCAG
AAGGAGCCGCCATTCCTGAAGATGGGCTACGAGCTCCATCCCGACAAGTGGACCGTGCAGCCTATCGTCCT
CCCCGAGAAGGACAGCTGGACCGTGAACGACATCCAGAAGCTGGTGGGCAAGCTCAACTGGGCTAGCCAGA
TCTATCCCGGGATCAAGGTGCGCCAGCTCTGCAAGCTGCTGCGCGGCACCAAGGCCCTGACCGAGGTGATT
CCCCTCACGGAGGAAGCCGAGCTCGAGCTGGCTGAGAACCGGGAGATCCTGAAGGAGCCCGTGCACGGCGT
GTACTATGACCCCTCCAAGGACCTGATCGCCGAAATCCAGAAGCAGGGCCAGGGGCAGTGGACATACCAGA
TTTACCAGGAGCCTTTCAAGAACCTCAAGACCGGCAAGTACGCCCGCATGAGGGGCGCCCACACCAACGAT
GTCAAGCAGCTGACCGAGGCCGTCCAGAAGATCACGACCGAGTCCATCGTGATCTGGGGGAAGACACCCAA
GTTCAAGCTGCCTATCCAGAAGGAGACCTGGGAGACGTGGTGGACCGAATATTGGCAGGCCACCTGGATTC
CCGAGTGGGAGTTCGTGAATACACCTCCTCTGGTGAAGCTGTGGTACCAGCTCGAGAAGGAGCCCATCGTG
GGCGCGGAGACATTCTACGTGGACGGCGCGGCCAACCGCGAAACAAAGCTCGGGAAGGCCGGGTACGTCAC
CAACCGGGGCCGCCAGAAGGTCGTCACCCTGACCGACACCACCAACCAGAAGACGGAGCTGCAGGCCATCT
ATCTCGCTCTCCAGGACTCCGGCCTGGAGGTGAACATCGTGACGGACAGCCAGTACGCGCTGGGCATTATT
CAGGCCCAGCCGGACCAGTCCGAGAGCGAACTGGTGAACCAGATTATCGAGCAGCTGATCAAGAAAGAGAA
GGTCTACCTCGCCTGGGTCCCGGCCCATAAGGGCATTGGCGGCAACGAGCAGGTCGACAAGCTGGTGAGTG
CGGGGATTAGAAAGGTGCTGATGGTGGGTTTTCCAGTCACACCTCAGGTACCTTTAAGACCAATGACTTAC
AAGGCAGCTGTAGATCTTAGCCACTTTTTAAAAGAAAAGGGGGGACTGGAAGGGCTAATTCACTCCCAAAG
AAGACAAGATATCCTTGATCTGTGGATCTACCACACACAAGGCTACTTCCCTGATTGGCAGAACTACACAC
CAGGGCCAGGGGTCAGATATCCACTGACCTTTGGATGGTGCTACAAGCTAGTACCAGTTGAGCCAGATAAG
GTAGAAGAGGCCAATAAAGGAGAGAACACCAGCTTGTTACACCCTGTGAGCCTGCATGGGATGGATGACCC
GGAGAGAGAAGTGTTAGAGTGGAGGTTTGACAGCCGCCTAGCATTTCATCACGTGGCCCGAGAGCTGCATC
CGGAGTACTTCAAGAACTGCATGGGTGCCCGAGCTTCGGTACTGTCTGGTGGAGAGCTGGACAGATGGGAG
AAAATTAGGCTGCGCCCGGGAGGCAAAAAGAAATACAAGCTCAAGCATATCGTGTGGGCCTCGAGGGAGCT
TGAACGGTTTGCCGTGAACCCAGGCCTGCTGGAAACATCTGAGGGATGTCGCCAGATCCTGGGGCAATTGC
AGCCATCCCTCCAGACCGGGAGTGAAGAGCTGAGGTCCTTGTATAACACAGTGGCTACCCTCTACTGCGTA
CACCAGAGGATCGAGATTAAGGATACCAAGGAGGCCTTGGACAAAATTGAGGAGGAGCAAAACAAGAGCAA
GAAGAAGGCCCAGCAGGCAGCTGCTGACACTGGGCATAGCAACCAGGTATCACAGAACTATCCTATTGTCC
AAAACATTCAGGGCCAGATGGTTCATCAGGCCATCAGCCCCCGGACGCTCAATGCCTGGGTGAAGGTTGTC
GAAGAGAAGGCCTTTTCTCCTGAGGTTATCCCCATGTTCTCCGCTTTGAGTGAGGGGGCCACTCCTCAGGA
CCTCAATACAATGCTTAATACCGTGGGCGGCCATCAGGCCGCCATGCAAATGTTGAAGGAGACTATCAACG
AGGAGGCAGCCGAGTGGGACAGAGTGCATCCCGTCCACGCTGGCCCAATCGCGCCCGGACAGATGCGGGAG
CCTCGCGGCTCTGACATTGCCGGCACCACCTCTACACTGCAAGAGCAAATCGGATGGATGACCAACAATCC
TCCCATCCCAGTTGGAGAAATCTATAAACGGTGGATCATCCTGGGCCTGAACAAGATCGTGCGCATGTACT
CTCCGACATCCATCCTTGACATTAGACAGGGACCCAAAGAGCCTTTTAGGGATTACGTCGACCGGTTTTAT
AAGACCCTGCGAGCAGAGCAGGCCTCTCAGGAGGTCAAAAACTGGATGACGGAGACACTCCTGGTACAGAA
CGCTAACCCCGACTGCAAAACAATCTTGAAGGCACTAGGCCCGGCTGCCACCCTGGAAGAGATGATGACCG
CCTGTCAGGGAGTAGGCGGACCCGGACACAAAGCCAGAGTGTTGTAA [SEQ ID NO: 84]

Aminoacid sequence of insert

MAEQLWVTVYYGVPVWKEATTTLFCASDAKAYDTEVHNVWATHACVPTDPNPQEVVLGNVTEYFNMWKNNM
VDQMHEDIISLWDQSLKPCVKLTPLCVTLDCDDVNTTNSTTTTSNGWTGEIRKGEIKNCSFNITTSIRDKV
QKEYALFYNLDVVPIDDDNATTKNKTTRNFRLIHCNSSVMTQACPKVSFEPIPIHYCAPAGFAILKCNNKT
FDGKGLCTNVSTVQCTHGIRPVVSTQLLLNGSLAEEEVVIRSDNFMDNTKTIIVQLNESVAINCTRPNNNT
RKGIHIGPGRAFYAARKIIGDIRQAHCNLSRAQWNNTLKQIVIKLREHFGNKTIKFNQSSGGDPEIVRHSF
NCGGEFFYCDTTQLFNSTWNGTEGNNTEGNSTITLPCRIKQIINMWQEVGKAMYAPPIGGQIRCSSNITGL
LLTRDGGTEGNGTENETEIFRPGGGDMRDNWRSELYKYKVVKVEPLGVAPTRAKRRVVQRMGPISPIETVP
VKLKPGMDGPKVKQWPLTEEKIKALVEICTEMEKEGKISKIGPENPYNTPVFAIKKKDSTKWRKLVDFREL
NKRTQDFWEVQLGIPHPAGLKKKKSVTVLDVGDAYFSVPLDEDFRKYTAFTIPSINNETPGIRYQYNVLPQ
GWKGSPAIFQSSMTKILEPFRKQNPDIVIYQYMDDLYVGSDLEIGQHRTKIEELRQHLLRWGLTTPDKKHQ
KEPPFLKMGYELHPDKWTVQPIVLPEKDSWTVNDIQKLVGKLNWASQIYPGIKVRQLCKLLRGTKALTEVI
PLTEEAELELAENREILKEPVHGVYYDPSKDLIAEIQKQGQGQWTYQIYQEPFKNLKTGKYARMRGAHTND
VKQLTEAVQKITTESIVIWGKTPKFKLPIQKETWETWWTEYWQATWIPEWEFVNTPPLVKLWYQLEKEPIV
GAETFYVDGAANRETKLGKAGYVTNRGRQKVVTLTDTTNQKTELQAIYLALQDSGLEVNIVTDSQYALGII

# Fig.21 (Cont II).

QAQPDQSESELVNQIIEQLIKKEKVYLAWVPAHKGIGGNEQVDKLVSAGIRKVLMVGFPVTPQVPLRPMTY
KAAVDLSHFLKEKGGLEGLIHSQRRQDILDLWIYHTQGYFPDWQNYTPGPGVRYPLTFGWCYKLVPVEPDK
VEEANKGENTSLLHPVSLHGMDDPEREVLEWRFDSRLAFHHVARELHPEYFKNCMGARASVLSGGELDRWE
KIRLRPGGKKKYKLKHIVWASRELERFAVNPGLLETSEGCRQILGQLQPSLQTGSEELRSLYNTVATLYCV
HQRIEIKDTKEALDKIEEEQNKSKKKAQQAAADTGHSNQVSQNYPIVQNIQGQMVHQAISPRTLNAWVKVV
EEKAFSPEVIPMFSALSEGATPQDLNTMLNTVGGHQAAMQMLKETINEEAAEWDRVHPVHAGPIAPGQMRE
PRGSDIAGTTSTLQEQIGWMTNNPPIPVGEIYKRWIILGLNKIVRMYSPTSILDIRQGPKEPFRDYVDRFY
KTLRAEQASQEVKNWMTETLLVQNANPDCKTILKALGPAATLEEMMTACQGVGGPGHKARVL [SEQ ID
NO: 85]

# Fig.22.

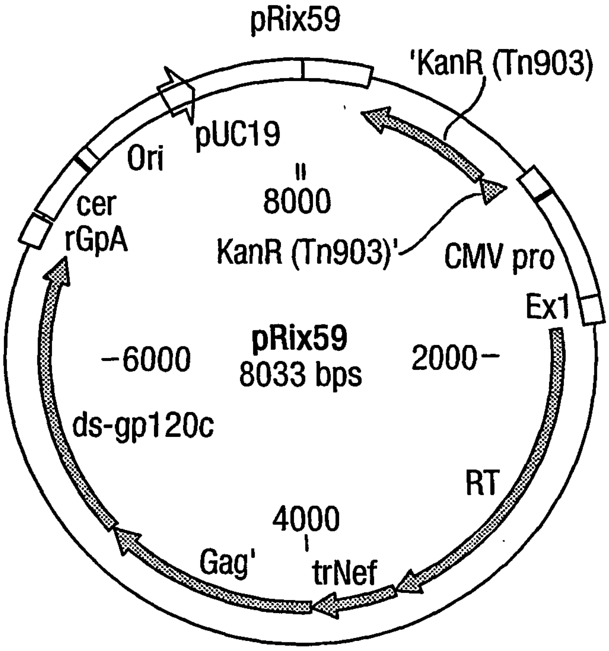


DNA sequence of insert

ATGGGCCCCATCAGTCCCATCGAGACCGTGCCGGTGAAGCTGAAACCCGGGATGGACGGCCCCAAGGTCAA
GCAGTGGCCACTCACCGAGGAGAAGATCAAGGCCCTGGTGGAGATCTGCACCGAGATGGAGAAAGAGGGCA
AGATCAGCAAGATCGGGCCGGAGAACCCATACAACACCCCCGTGTTTGCCATCAAGAAGAAGGACAGCACC
AAGTGGCGCAAGCTGGTGGATTTCCGGGAGCTGAATAAGCGGACCCAGGATTTCTGGGAGGTCCAGCTGGG
CATCCCCCATCCGGCCGGCCTGAAGAAGAAGAAGAGCGTGACCGTGCTGGACGTGGGCGACGCTTACTTCA
GCGTCCCTCTGGACGAGGACTTTAGAAAGTACACCGCCTTTACCATCCCATCTATCAACAACGAGACCCCT
GGCATCAGATATCAGTACAACGTCCTCCCCCAGGGCTGGAAGGGCTCTCCCGCCATTTTCCAGAGCTCCAT
GACCAAGATCCTGGAGCCGTTTCGGAAGCAGAACCCCGATATCGTCATCTACCAGTACATGGACGACCTGT
ACGTGGGCTCTGACCTGGAAATCGGGCAGCATCGCACGAAGATTGAGGAGCTGAGGCAGCATCTGCTGAGA
TGGGGCCTGACCACTCCGGACAAGAAGCATCAGAAGGAGCCGCCATTCCTGAAGATGGGCTACGAGCTCCA
TCCCGACAAGTGGACCGTGCAGCCTATCGTCCTCCCCGAGAAGGACAGCTGGACCGTGAACGACATCCAGA
AGCTGGTGGGCAAGCTCAACTGGGCTAGCCAGATCTATCCCGGGATCAAGGTGCGCCAGCTCTGCAAGCTG
CTGCGCGGCACCAAGGCCCTGACCGAGGTGATTCCCCTCACGGAGGAAGCCGAGCTCGAGCTGGCTGAGAA
CCGGGAGATCCTGAAGGAGCCCGTGCACGGCGTGTACTATGACCCCTCCAAGGACCTGATCGCCGAAATCC
AGAAGCAGGGCCAGGGGCAGTGGACATACCAGATTTACCAGGAGCCTTTCAAGAACCTCAAGACCGGCAAG
TACGCCCGCATGAGGGGCGCCCACACCAACGATGTCAAGCAGCTGACCGAGGCCGTCCAGAAGATCACGAC
CGAGTCCATCGTGATCTGGGGGAAGACACCCAAGTTCAAGCTGCCTATCCAGAAGGAGACCTGGGAGACGT
GGTGGACCGAATATTGGCAGGCCACCTGGATTCCCGAGTGGGAGTTCGTGAATACACCTCCTCTGGTGAAG
CTGTGGTACCAGCTCGAGAAGGAGCCCATCGTGGGCGCGGAGACATTCTACGTGGACGGCGCGGCCAACCG
CGAAACAAAGCTCGGGAAGGCCGGGTACGTCACCAACCGGGGCCGCCAGAAGGTCGTCACCCTGACCGACA
CCACCAACCAGAAGACGGAGCTGCAGGCCATCTATCTCGCTCTCCAGGACTCCGGCCTGGAGGTGAACATC
GTGACGGACAGCCAGTACGCGCTGGGCATTATTCAGGCCCAGCCGGACCAGTCCGAGAGCGAACTGGTGAA
CCAGATTATCGAGCAGCTGATCAAGAAAGAGAAGGTCTACCTCGCCTGGGTCCCGGCCCATAAGGGCATTG
GCGGCAACGAGCAGGTCGACAAGCTGGTGAGTGCGGGGATTAGAAAGGTGCTGATGGTGGGTTTTCCAGTC
ACACCTCAGGTACCTTTAAGACCAATGACTTACAAGGCAGCTGTAGATCTTAGCCACTTTTTAAAAGAAAA
GGGGGGACTGGAAGGGCTAATTCACTCCCAAAGAAGACAAGATATCCTTGATCTGTGGATCTACCACACAC
AAGGCTACTTCCCTGATTGGCAGAACTACACACCAGGGCCAGGGGTCAGATATCCACTGACCTTTGGATGG
TGCTACAAGCTAGTACCAGTTGAGCCAGATAAGGTAGAAGAGGCCAATAAAGGAGAGAACACCAGCTTGTT
ACACCCTGTGAGCCTGCATGGGATGGATGACCCGGAGAGAGAAGTGTTAGAGTGGAGGTTTGACAGCCGCC

# Fig.22 (Cont I).

TAGCATTTCATCACGTGGCCCGAGAGCTGCATCCGGAGTACTTCAAGAACTGCATGGGTGCCCGAGCTTCG
GTACTGTCTGGTGGAGAGCTGGACAGATGGGAGAAAATTAGGCTGCGCCCGGGAGGCAAAAAGAAATACAA
GCTCAAGCATATCGTGTGGGCCTCGAGGGAGCTTGAACGGTTTGCCGTGAACCCAGGCCTGCTGGAAACAT
CTGAGGGATGTCGCCAGATCCTGGGGCAATTGCAGCCATCCCTCCAGACCGGGAGTGAAGAGCTGAGGTCC
TTGTATAACACAGTGGCTACCCTCTACTGCGTACACCAGAGGATCGAGATTAAGGATACCAAGGAGGCCTT
GGACAAAATTGAGGAGGAGCAAAACAAGAGCAAGAAGAAGGCCCAGCAGGCAGCTGCTGACACTGGGCATA
GCAACCAGGTATCACAGAACTATCCTATTGTCCAAAACATTCAGGGCCAGATGGTTCATCAGGCCATCAGC
CCCCGGACGCTCAATGCCTGGGTGAAGGTTGTCGAAGAGAAGGCCTTTTCTCCTGAGGTTATCCCCATGTT
CTCCGCTTTGAGTGAGGGGGCCACTCCTCAGGACCTCAATACAATGCTTAATACCGTGGGCGGCCATCAGG
CCGCCATGCAAATGTTGAAGGAGACTATCAACGAGGAGGCAGCCGAGTGGGACAGAGTGCATCCCGTCCAC
GCTGGCCCAATCGCGCCCGGACAGATGCGGGAGCCTCGCGGCTCTGACATTGCCGGCACCACCTCTACACT
GCAAGAGCAAATCGGATGGATGACCAACAATCCTCCCATCCCAGTTGGAGAAATCTATAAACGGTGGATCA
TCCTGGGCCTGAACAAGATCGTGCGCATGTACTCTCCGACATCCATCCTTGACATTAGACAGGGACCCAAA
GAGCCTTTTAGGGATTACGTCGACCGGTTTTATAAGACCCTGCGAGCAGAGCAGGCCTCTCAGGAGGTCAA
AAACTGGATGACGGAGACACTCCTGGTACAGAACGCTAACCCCGACTGCAAAACAATCTTGAAGGCACTAG
GCCCGGCTGCCACCCTGGAAGAGATGATGACCGCCTGTCAGGGAGTAGGCGGACCCGGACACAAAGCCAGA
GTGTTGATGGCCGAGCAGCTGTGGGTCACCGTCTACTACGGCGTGCCTGTGTGGAAGGAGGCCACGACCAC
CCTCTTCTGCGCGAGCGACGCCAAGGCCTACGACACGGAAGTGCATAACGTGTGGGCGACGCATGCTTGCG
TGCCTACGGACCCCAACCCCCAGGAGGTGGTGCTGGGAAACGTGACCGAGTACTTCAACATGTGGAAGAAT
AACATGGTGGATCAGATGCACGAGGACATCATCTCTCTGTGGGACCAGTCCCTGAAGCCCTGCGTGAAGCT
GACGCCTCTCTGCGTGACACTGGACTGTGACGACGTCAACACCACCAACAGCACTACCACCACCAGCAACG
GCTGGACCGGAGAGATTCGGAAGGGCGAGATCAAGAACTGCTCCTTCAATATCACGACCTCGATCAGAGAC
AAGGTGCAGAAGGAATACGCGCTGTTTTATAATCTCGATGTGGTCCCCATCGACGACGACAATGCCACCAC
CAAGAACAAGACGACGCGTAATTTCAGACTCATTCACTGCAACAGCAGCGTCATGACGCAGGCCTGCCCCA
AGGTGTCCTTCGAACCAATCCCGATCCATTACTGTGCCCCTGCCGGATTCGCGATCCTCAAGTGTAACAAC
AAGACCTTCGACGGGAAGGGCCTGTGCACCAACGTCAGCACGGTGCAGTGCACCCATGGCATCCGCCCCGT
CGTGAGCACCCAGCTGCTGCTGAACGGGTCCCTGGCTGAGGAGGAGGTGGTGATCCGGTCGGACAACTTCA
TGGACAACACCAAGACAATCATCGTCCAGCTGAACGAGTCTGTGGCGATTAACTGTACCCGGCCTAACAAC
AACACCCGTAAGGGCATCCACATCGGGCCTGGACGGGCCTTCTATGCCGCCCGCAAGATCATCGGCGACAT
CCGGCAGGCCCATTGCAACCTCTCCCGCGCCCAGTGGAATAACACCCTGAAGCAGATCGTGATCAAGCTGA
GAGAGCACTTTGGAAACAAGACCATCAAGTTCAATCAGAGTTCTGGCGGAGACCCCGAGATCGTGCGGCAC
TCCTTCAACTGCGGGGGCGAGTTCTTCTACTGCGATACGACACAGCTCTTCAACTCCACCTGGAACGGCAC
CGAGGGCAACAACACAGAGGGAAACTCCACTATCACCCTCCCTTGCCGCATCAAGCAGATCATCAACATGT
GGCAGGAGGTGGGAAAGGCCATGTATGCCCCCCCCATCGGGGGCCAGATCCGCTGCTCCTCCAACATCACC
GGCCTGCTGCTCACCAGAGACGGGGGCACCGAGGGCAACGGCACGGAGAACGAGACGGAGATCTTCAGGCC
CGGCGGCGGCGACATGAGGGATAACTGGCGGAGCGAGCTGTACAAGTACAAGGTGGTGAAGGTGGAGCCGC
TCGGCGTGGCCCCCACCCGGGCCAAGCGCCGCGTCGTGCAGAGATGA [SEQ ID NO: 86]

**Aminoacid sequence of insert**

MGPISPIETVPVKLKPGMDGPKVKQWPLTEEKIKALVEICTEMEKEGKISKIGPENPYNTPVFAIKKKDST
KWRKLVDFRELNKRTQDFWEVQLGIPHPAGLKKKKSVTVLDVGDAYFSVPLDEDFRKYTAFTIPSINNETP
GIRYQYNVLPQGWKGSPAIFQSSMTKILEPFRKQNPDIVIYQYMDDLYVGSDLEIGQHRTKIEELRQHLLR
WGLTTPDKKHQKEPPFLKMGYELHPDKWTVQPIVLPEKDSWTVNDIQKLVGKLNWASQIYPGIKVRQLCKL
LRGTKALTEVIPLTEEAELELAENREILKEPVHGVYYDPSKDLIAEIQKQGQGQWTYQIYQEPFKNLKTGK
YARMRGAHTNDVKQLTEAVQKITTESIVIWGKTPKFKLPIQKETWETWWTEYWQATWIPEWEFVNTPPLVK
LWYQLEKEPIVGAETFYVDGAANRETKLGKAGYVTNRGRQKVVTLTDTTNQKTELQAIYLALQDSGLEVNI
VTDSQYALGIIQAQPDQSESELVNQIIEQLIKKEKVYLAWVPAHKGIGGNEQVDKLVSAGIRKVLMVGFPV
TPQVPLRPMTYKAAVDLSHFLKEKGGLEGLIHSQRRQDILDLWIYHTQGYFPDWQNYTPGPGVRYPLTFGW
CYKLVPVEPDKVEEANKGENTSLLHPVSLHGMDDPEREVLEWRFDSRLAFHHVARELHPEYFKNCMGARAS
VLSGGELDRWEKIRLRPGGKKKYKLKHIVWASRELERFAVNPGLLETSEGCRQILGQLQPSLQTGSEELRS
LYNTVATLYCVHQRIEIKDTKEALDKIEEEQNKSKKKAQQAAADTGHSNQVSQNYPIVQNIQGQMVHQAIS
PRTLNAWVKVVEEKAFSPEVIPMFSALSEGATPQDLNTMLNTVGGHQAAMQMLKETINEEAAEWDRVHPVH
AGPIAPGQMREPRGSDIAGTTSTLQEQIGWMTNNPPIPVGEIYKRWIILGLNKIVRMYSPTSILDIRQGPK
EPFRDYVDRFYKTLRAEQASQEVKNWMTETLLVQNANPDCKTILKALGPAATLEEMMTACQGVGGPGHKAR

# Fig.22 (Cont II).

VLMAEQLWVTVYYGVPVWKEATTTLFCASDAKAYDTEVHNVWATHACVPTDPNPQEVVLGNVTEYFNMWKN
NMVDQMHEDIISLWDQSLKPCVKLTPLCVTLDCDDVNTTNSTTTTSNGWTGEIRKGEIKNCSFNITTSIRD
KVQKEYALFYNLDVVPIDDDNATTKNKTTRNFRLIHCNSSVMTQACPKVSFEPIPIHYCAPAGFAILKCNN
KTFDGKGLCTNVSTVQCTHGIRPVVSTQLLLNGSLAEEEVVIRSDNFMDNTKTIIVQLNESVAINCTRPNN
NTRKGIHIGPGRAFYAARKIIGDIRQAHCNLSRAQWNNTLKQIVIKLREHFGNKTIKFNQSSGGDPEIVRH
SFNCGGEFFYCDTTQLFNSTWNGTEGNNTEGNSTITLPCRIKQIINMWQEVGKAMYAPPIGGQIRCSSNIT
GLLLTRDGGTEGNGTENETEIFRPGGGDMRDNWRSELYKYKVVKVEPLGVAPTRAKRRVVQR [SEQ ID NO: 87]

# Fig.23.

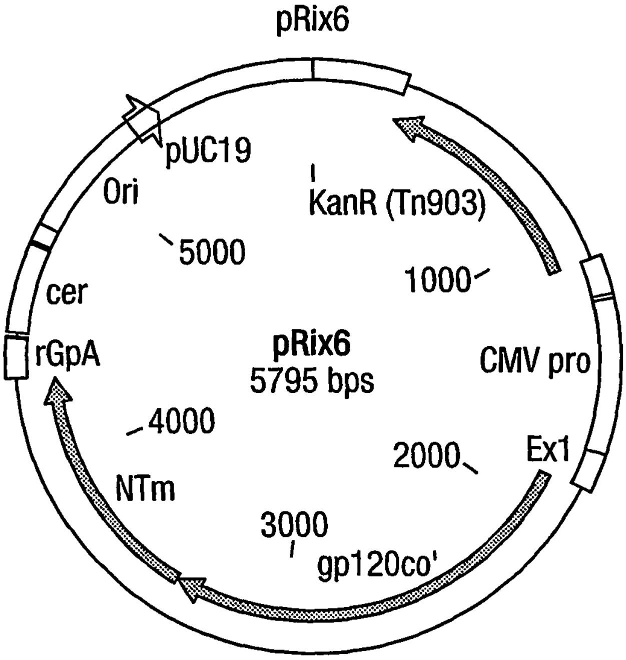


**DNA sequence of insert**

ATGAAGGTCAAGGAGACCAGAAAGAACTACCAGCATCTGTGGCGCTGGGGCACCATGCTCCTGGGAATGCT
GATGATCTGCTCCGCCGCCGAGCAGCTGTGGGTCACCGTCTACTACGGCGTGCCTGTGTGGAAGGAGGCCA
CGACCACCCTCTTCTGCGCGAGCGACGCCAAGGCCTACGACACGGAAGTGCATAACGTGTGGGCGACGCAT
GCTTGCGTGCCTACGGACCCCAACCCCCAGGAGGTGGTGCTGGGAAACGTGACCGAGTACTTCAACATGTG
GAAGAATAACATGGTGGATCAGATGCACGAGGACATCATCTCTCTGTGGGACCAGTCCCTGAAGCCCTGCG
TGAAGCTGACGCCTCTCTGCGTGACACTGGACTGTGACGACGTCAACACCACCAACAGCACTACCACCACC
AGCAACGGCTGGACCGGAGAGATTCGGAAGGGCGAGATCAAGAACTGCTCCTTCAATATCACGACCTCGAT
CAGAGACAAGGTGCAGAAGGAATACGCGCTGTTTTATAATCTCGATGTGGTCCCCATCGACGACGACAATG
CCACCACCAAGAACAAGACGACGCGTAATTTCAGACTCATTCACTGCAACAGCAGCGTCATGACGCAGGCC
TGCCCCAAGGTGTCCTTCGAACCAATCCCGATCCATTACTGTGCCCCTGCCGGATTCGCGATCCTCAAGTG
TAACAACAAGACCTTCGACGGGAAGGGCCTGTGCACCAACGTCAGCACGGTGCAGTGCACCCATGGCATCC
GCCCCGTCGTGAGCACCCAGCTGCTGCTGAACGGGTCCCTGGCTGAGGAGGAGGTGGTGATCCGGTCGGAC
AACTTCATGGACAACACCAAGACAATCATCGTCCAGCTGAACGAGTCTGTGGCGATTAACTGTACCCGGCC
TAACAACAACACCCGTAAGGGCATCCACATCGGGCCTGGACGGGCCTTCTATGCCGCCCGCAAGATCATCG
GCGACATCCGGCAGGCCCATTGCAACCTCTCCCGCGCCCAGTGGAATAACACCCTGAAGCAGATCGTGATC
AAGCTGAGAGAGCACTTTGGAAACAAGACCATCAAGTTCAATCAGAGTTCTGGCGGAGACCCCGAGATCGT
GCGGCACTCCTTCAACTGCGGGGGCGAGTTCTTCTACTGCGATACGACACAGCTCTTCAACTCCACCTGGA
ACGGCACCGAGGGCAACAACACAGAGGGAAACTCCACTATCACCCTCCCTTGCCGCATCAAGCAGATCATC
AACATGTGGCAGGAGGTGGGAAAGGCCATGTATGCCCCCCCCATCGGGGGCCAGATCCGCTGCTCCTCCAA
CATCACCGGCCTGCTGCTCACCAGAGACGGGGGCACCGAGGGCAACGGCACGGAGAACGAGACGGAGATCT
TCAGGCCCGGCGGCGGCGACATGAGGGATAACTGGCGGAGCGAGCTGTACAAGTACAAGGTGGTGAAGGTG
GAGCCGCTCGGCGTGGCCCCCACCCGGGCCAAGCGCCGCGTCGTGCAGAGAATGGGTGGCAAGTGGTCAAA
AAGTAGTGTGGTTGGATGGCCTACTGTAAGGGAAAGAATGAGACGAGCTGAGCCAGCAGCAGATGGGGTGG
GAGCAGCATCTCGAGACCTGGAAAAACATGGAGCAATCACAAGTAGCAATACAGCAGCTACCAATGCTGCT
TGTGCCTGGCTAGAAGCACAAGAGGAGGAGGAGGTGGGTTTTCCAGTCACACCTCAGGTACCTTTAAGACC
AATGACTTACAAGGCAGCTGTAGATCTTAGCCACTTTTTAAAAGAAAAGGGGGGACTGGAAGGGCTAATTC
ACTCCCAACGAAGACAAGATATCCTTGATCTGTGGATCTACCACACACAAGGCTACTTCCCTGATTGGCAG
AACTACACACCAGGGCCAGGGGTCAGATATCCACTGACCTTTGGATGGTGCTACAAGCTAGTACCAGTTGA
GCCAGATAAGGTAGAAGAGGCCAATAAAGGAGAGAACACCAGCTTGTTACACCCTGTGAGCCTGCATGGAA

# Fig.23 (Cont).

TGGATGACCCTGAGAGAGAAGTGTTAGAGTGGAGGTTTGACAGCCGCCTAGCATTTCATCACGTGGCCCGA
GAGCTGCATCCGGAGTACTTCAAGAACTGCACTAGTGAGCCAGTAGATCCTAGACTAGAGCCCTGGAAGCA
TCCAGGAAGTCAGCCTAAAACTGCTTGTACCAATTGCTATTGTAAAAAGTGTTGCTTTCATTGCCAAGTTT
GTTTCATAACAGCTGCCTTAGGCATCTCCTATGGCAGGAAGAAGCGGAGACAGCGACGAAGACCTCCTCAA
GGCAGTCAGACTCATCAAGTTTCTCTATCAAAGCAACCCACCTCCCAATCCAAAGGGGAGCCGACAGGCCC
GAAGGAATAA [SEQ ID NO: 88]

**Aminoacid sequence of insert**

MKVKETRKNYQHLWRWGTMLLGMLMICSAAEQLWVTVYYGVPVWKEATTTLFCASDAKAYDTEVHNVWATH
ACVPTDPNPQEVVLGNVTEYFNMWKNNMVDQMHEDIISLWDQSLKPCVKLTPLCVTLDCDDVNTTNSTTTT
SNGWTGEIRKGEIKNCSFNITTSIRDKVQKEYALFYNLDVVPIDDDNATTKNKTTRNFRLIHCNSSVMTQA
CPKVSFEPIPIHYCAPAGFAILKCNNKTFDGKGLCTNVSTVQCTHGIRPVVSTQLLLNGSLAEEEVVIRSD
NFMDNTKTIIVQLNESVAINCTRPNNNTRKGIHIGPGRAFYAARKIIGDIRQAHCNLSRAQWNNTLKQIVI
KLREHFGNKTIKFNQSSGGDPEIVRHSFNCGGEFFYCDTTQLFNSTWNGTEGNNTEGNSTITLPCRIKQII
NMWQEVGKAMYAPPIGGQIRCSSNITGLLLTRDGGTEGNGTENETEIFRPGGGDMRDNWRSELYKYKVVKV
EPLGVAPTRAKRRVVQRMGGKWSKSSVVGWPTVRERMRRAEPAADGVGAASRDLEKHGAITSSNTAATNAA
CAWLEAQEEEEVGFPVTPQVPLRPMTYKAAVDLSHFLKEKGGLEGLIHSQRRQDILDLWIYHTQGYFPDWQ
NYTPGPGVRYPLTFGWCYKLVPVEPDKVEEANKGENTSLLHPVSLHGMDDPEREVLEWRFDSRLAFHHVAR
ELHPEYFKNCTSEPVDPRLEPWKHPGSQPKTACTNCYCKKCCFHCQVCFITAALGISYGRKKRRQRRRPPQ
GSQTHQVSLSKQPTSQSKGEPTGPKE [SEQ ID NO: 89]

# Fig.24.

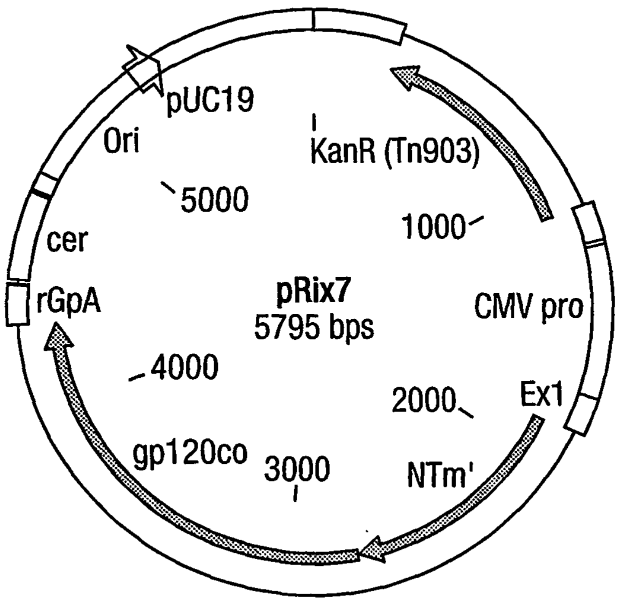


**DNA sequence of insert**

ATGGGTGGCAAGTGGTCAAAAAGTAGTGTGGTTGGATGGCCTACTGTAAGGGAAAGAATGAGACGAGCTGA
GCCAGCAGCAGATGGGGTGGAGCAGCATCTCGAGACCTGGAAAAACATGGAGCAATCACAAGTAGCAATA
CAGCAGCTACCAATGCTGCTTGTGCCTGGCTAGAAGCACAAGAGGAGGAGGAGGTGGGTTTTCCAGTCACA
CCTCAGGTACCTTTAAGACCAATGACTTACAAGGCAGCTGTAGATCTTAGCCACTTTTTAAAAGAAAAGGG
GGGACTGGAAGGGCTAATTCACTCCCAACGAAGACAAGATATCCTTGATCTGTGGATCTACCACACACAAG
GCTACTTCCCTGATTGGCAGAACTACACACCAGGGCCAGGGGTCAGATATCCACTGACCTTTGGATGGTGC
TACAAGCTAGTACCAGTTGAGCCAGATAAGGTAGAAGAGGCCAATAAAGGAGAGAACACCAGCTTGTTACA
CCCTGTGAGCCTGCATGGAATGGATGACCCTGAGAGAGAAGTGTTAGAGTGGAGGTTTGACAGCCGCCTAG
CATTTCATCACGTGGCCCGAGAGCTGCATCCGGAGTACTTCAAGAACTGCACTAGTGAGCCAGTAGATCCT
AGACTAGAGCCCTGGAAGCATCCAGGAAGTCAGCCTAAAACTGCTTGTACCAATTGCTATTGTAAAAAGTG
TTGCTTTCATTGCCAAGTTTGTTTCATAACAGCTGCCTTAGGCATCTCCTATGGCAGGAAGAAGCGGAGAC
AGCGACGAAGACCTCCTCAAGGCAGTCAGACTCATCAAGTTTCTCTATCAAAGCAACCCACCTCCCAATCC
AAAGGGGAGCCGACAGGCCCGAAGGAAATGAAGGTCAAGGAGACCAGAAAGAACTACCAGCATCTGTGGCG
CTGGGGCACCATGCTCCTGGGAATGCTGATGATCTGCTCCGCCGCCGAGCAGCTGTGGGTCACCGTCTACT
ACGGCGTGCCTGTGTGGAAGGAGGCCACGACCACCCTCTTCTGCGCGAGCGACGCCAAGGCCTACGACACG
GAAGTGCATAACGTGTGGGCGACGCATGCTTGCGTGCCTACGGACCCCAACCCCCAGGAGGTGGTGCTGGG
AAACGTGACCGAGTACTTCAACATGTGGAAGAATAACATGGTGGATCAGATGCACGAGGACATCATCTCTC
TGTGGGACCAGTCCCTGAAGCCCTGCGTGAAGCTGACGCCTCTCTGCGTGACACTGGACTGTGACGACGTC
AACACCACCAACAGCACTACCACCACCAGCAACGGCTGGACCGGAGAGATTCGGAAGGGCGAGATCAAGAA
CTGCTCCTTCAATATCACGACCTCGATCAGAGACAAGGTGCAGAAGGAATACGCGCTGTTTTATAATCTCG
ATGTGGTCCCCATCGACGACGACAATGCCACCACCAAGAACAAGACGACGCGTAATTTCAGACTCATTCAC
TGCAACAGCAGCGTCATGACGCAGGCCTGCCCCAAGGTGTCCTTCGAACCAATCCCGATCCATTACTGTGC
CCCTGCCGGATTCGCGATCCTCAAGTGTAACAACAAGACCTTCGACGGGAAGGGCCTGTGCACCAACGTCA
GCACGGTGCAGTGCACCCATGGCATCCGCCCCGTCGTGAGCACCCAGCTGCTGCTGAACGGGTCCCTGGCT
GAGGAGGAGGTGGTGATCCGGTCGGACAACTTCATGGACAACACCAAGACAATCATCGTCCAGCTGAACGA
GTCTGTGGCGATTAACTGTACCCGGCCTAACAACAACACCCGTAAGGGCATCCACATCGGGCCTGGACGGG
CCTTCTATGCCGCCCGCAAGATCATCGGCGACATCCGGCAGGCCCATTGCAACCTCTCCCGCGCCCAGTGG
AATAACACCCTGAAGCAGATCGTGATCAAGCTGAGAGAGCACTTTGGAAACAAGACCATCAAGTTCAATCA
GAGTTCTGGCGGAGACCCCGAGATCGTGCGGCACTCCTTCAACTGCGGGGGCGAGTTCTTCTACTGCGATA
CGACACAGCTCTTCAACTCCACCTGGAACGGCACCGAGGGCAACAACACAGAGGGAAACTCCACTATCACC

# Fig.24 (Cont).

CTCCCTTGCCGCATCAAGCAGATCATCAACATGTGGCAGGAGGTGGGAAAGGCCATGTATGCCCCCCCCAT
CGGGGGCCAGATCCGCTGCTCCTCCAACATCACCGGCCTGCTGCTCACCAGAGACGGGGGCACCGAGGGCA
ACGGCACGGAGAACGAGACGGAGATCTTCAGGCCCGGCGGCGGCGACATGAGGGATAACTGGCGGAGCGAG
CTGTACAAGTACAAGGTGGTGAAGGTGGAGCCGCTCGGCGTGGCCCCCACCCGGGCCAAGCGCCGCGTCGT
GCAGAGATGA [SEQ ID NO: 90]

**Aminoacid sequence of insert**

MGGKWSKSSVVGWPTVRERMRRAEPAADGVGAASRDLEKHGAITSSNTAATNAACAWLEAQEEEEVGFPVT
PQVPLRPMTYKAAVDLSHFLKEKGGLEGLIHSQRRQDILDLWIYHTQGYFPDWQNYTPGPGVRYPLTFGWC
YKLVPVEPDKVEEANKGENTSLLHPVSLHGMDDPEREVLEWRFDSRLAFHHVARELHPEYFKNCTSEPVDP
RLEPWKHPGSQPKTACTNCYCKKCCFHCQVCFITAALGISYGRKKRRQRRRPPQGSQTHQVSLSKQPTSQS
KGEPTGPKEMKVKETRKNYQHLWRWGTMLLGMLMICSAAEQLWVTVYYGVPVWKEATTTLFCASDAKAYDT
EVHNVWATHACVPTDPNPQEVVLGNVTEYFNMWKNNMVDQMHEDIISLWDQSLKPCVKLTPLCVTLDCDDV
NTTNSTTTTSNGWTGEIRKGEIKNCSFNITTSIRDKVQKEYALFYNLDVVPIDDDNATTKNKTTRNFRLIH
CNSSVMTQACPKVSFEPIPIHYCAPAGFAILKCNNKTFDGKGLCTNVSTVQCTHGIRPVVSTQLLLNGSLA
EEEVVIRSDNFMDNTKTIIVQLNESVAINCTRPNNNTRKGIHIGPGRAFYAARKIIGDIRQAHCNLSRAQW
NNTLKQIVIKLREHFGNKTIKFNQSSGGDPEIVRHSFNCGGEFFYCDTTQLFNSTWNGTEGNNTEGNSTIT
LPCRIKQIINMWQEVGKAMYAPPIGGQIRCSSNITGLLLTRDGGTEGNGTENETEIFRPGGGDMRDNWRSE
LYKYKVVKVEPLGVAPTRAKRRVVQR [SEQ ID NO: 91]

# Fig.25.

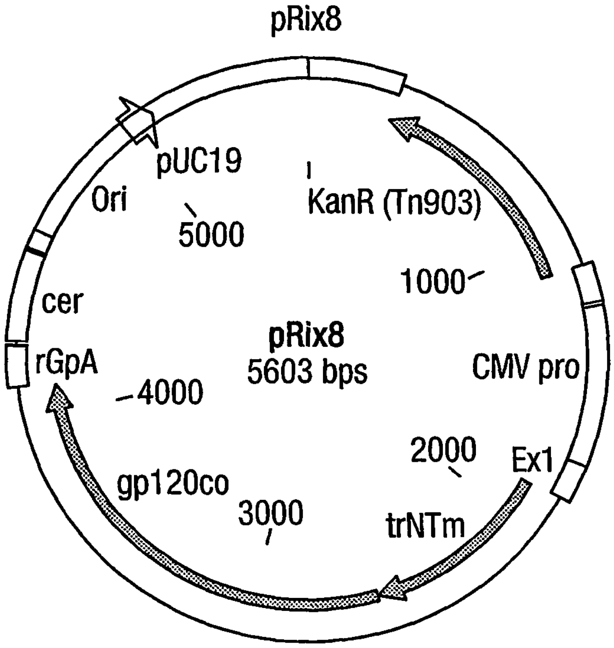


**DNA sequence of insert**

ATGGTGGGTTTTCCAGTCACACCTCAGGTACCTTTAAGACCAATGACTTACAAGGCAGCTGTAGATCTTAG
CCACTTTTTAAAAGAAAAGGGGGGACTGGAAGGGCTAATTCACTCCCAACGAAGACAAGATATCCTTGATC
TGTGGATCTACCACACACAAGGCTACTTCCCTGATTGGCAGAACTACACACCAGGGCCAGGGGTCAGATAT
CCACTGACCTTTGGATGGTGCTACAAGCTAGTACCAGTTGAGCCAGATAAGGTAGAAGAGGCCAATAAAGG
AGAGAACACCAGCTTGTTACACCCTGTGAGCCTGCATGGAATGGATGACCCTGAGAGAGAAGTGTTAGAGT
GGAGGTTTGACAGCCGCCTAGCATTTCATCACGTGGCCCGAGAGCTGCATCCGGAGTACTTCAAGAACTGC
ACTAGTGAGCCAGTAGATCCTAGACTAGAGCCCTGGAAGCATCCAGGAAGTCAGCCTAAAACTGCTTGTAC
CAATTGCTATTGTAAAAAGTGTTGCTTTCATTGCCAAGTTTGTTTCATAACAGCTGCCTTAGGCATCTCCT
ATGGCAGGAAGAAGCGGAGACAGCGACGAAGACCTCCTCAAGGCAGTCAGACTCATCAAGTTTCTCTATCA
AAGCAACCCACCTCCCAATCCAAAGGGGAGCCGACAGGCCCGAAGGAAATGAAGGTCAAGGAGACCAGAAA
GAACTACCAGCATCTGTGGCGCTGGGGCACCATGCTCCTGGGAATGCTGATGATCTGCTCCGCCGCCGAGC
AGCTGTGGGTCACCGTCTACTACGGCGTGCCTGTGTGGAAGGAGGCCACGACCACCCTCTTCTGCGCGAGC
GACGCCAAGGCCTACGACACGGAAGTGCATAACGTGTGGGCGACGCATGCTTGCGTGCCTACGGACCCCAA
CCCCCAGGAGGTGGTGCTGGGAAACGTGACCGAGTACTTCAACATGTGGAAGAATAACATGGTGGATCAGA
TGCACGAGGACATCATCTCTCTGTGGGACCAGTCCCTGAAGCCCTGCGTGAAGCTGACGCCTCTCTGCGTG
ACACTGGACTGTGACGACGTCAACACCACCAACAGCACTACCACCACCAGCAACGGCTGGACCGGAGAGAT
TCGGAAGGGCGAGATCAAGAACTGCTCCTTCAATATCACGACCTCGATCAGAGACAAGGTGCAGAAGGAAT
ACGCGCTGTTTTATAATCTCGATGTGGTCCCCATCGACGACGACAATGCCACCACCAAGAACAAGACGACG
CGTAATTTCAGACTCATTCACTGCAACAGCAGCGTCATGACGCAGGCCTGCCCCAAGGTGTCCTTCGAACC
AATCCCGATCCATTACTGTGCCCCTGCCGGATTCGCGATCCTCAAGTGTAACAACAAGACCTTCGACGGGA
AGGGCCTGTGCACCAACGTCAGCACGGTGCAGTGCACCCATGGCATCCGCCCCGTCGTGAGCACCCAGCTG
CTGCTGAACGGGTCCCTGGCTGAGGAGGAGGTGGTGATCCGGTCGGACAACTTCATGGACAACACCAAGAC
AATCATCGTCCAGCTGAACGAGTCTGTGGCGATTAACTGTACCCGGCCTAACAACAACACCCGTAAGGGCA
TCCACATCGGGCCTGGACGGGCCTTCTATGCCGCCCGCAAGATCATCGGCGACATCCGGCAGGCCCATTGC
AACCTCTCCCGCGCCCAGTGGAATAACACCCTGAAGCAGATCGTGATCAAGCTGAGAGAGCACTTTGGAAA
CAAGACCATCAAGTTCAATCAGAGTTCTGGCGGAGACCCCGAGATCGTGCGGCACTCCTTCAACTGCGGGG
GCGAGTTCTTCTACTGCGATACGACACAGCTCTTCAACTCCACCTGGAACGGCACCGAGGGCAACAACACA
GAGGGAAACTCCACTATCACCCTCCCTTGCCGCATCAAGCAGATCATCAACATGTGGCAGGAGGTGGGAAA
GGCCATGTATGCCCCCCCCATCGGGGGCCAGATCCGCTGCTCCTCCAACATCACCGGCCTGCTGCTCACCA
GAGACGGGGGCACCGAGGGCAACGGCACGGAGAACGAGACGGAGATCTTCAGGCCCGGCGGCGGCGACATG

# Fig.25 (Cont).

AGGGATAACTGGCGGAGCGAGCTGTACAAGTACAAGGTGGTGAAGGTGGAGCCGCTCGGCGTGGCCCCCAC
CCGGGCCAAGCGCCGCGTCGTGCAGAGATGA [SEQ ID NO: 92]

**Aminoacid sequence of insert**

MVGFPVTPQVPLRPMTYKAAVDLSHFLKEKGGLEGLIHSQRRQDILDLWIYHTQGYFPDWQNYTPGPGVRY
PLTFGWCYKLVPVEPDKVEEANKGENTSLLHPVSLHGMDDPEREVLEWRFDSRLAFHHVARELHPEYFKNC
TSEPVDPRLEPWKHPGSQPKTACTNCYCKKCCFHCQVCFITAALGISYGRKKRRQRRRPPQGSQTHQVSLS
KQPTSQSKGEPTGPKEMKVKETRKNYQHLWRWGTMLLGMLMICSAAEQLWVTVYYGVPVWKEATTTLFCAS
DAKAYDTEVHNVWATHACVPTDPNPQEVVLGNVTEYFNMWKNNMVDQMHEDIISLWDQSLKPCVKLTPLCV
TLDCDDVNTTNSTTTTSNGWTGEIRKGEIKNCSFNITTSIRDKVQKEYALFYNLDVVPIDDDNATTKNKTT
RNFRLIHCNSSVMTQACPKVSFEPIPIHYCAPAGFAILKCNNKTFDGKGLCTNVSTVQCTHGIRPVVSTQL
LLNGSLAEEEVVIRSDNFMDNTKTIIVQLNESVAINCTRPNNNTRKGIHIGPGRAFYAARKIIGDIRQAHC
NLSRAQWNNTLKQIVIKLREHFGNKTIKFNQSSGGDPEIVRHSFNCGGEFFYCDTTQLFNSTWNGTEGNNT
EGNSTITLPCRIKQIINMWQEVGKAMYAPPIGGQIRCSSNITGLLLTRDGGTEGNGTENETEIFRPGGGDM
RDNWRSELYKYKVVKVEPLGVAPTRAKRRVVQR [SEQ ID NO: 93]

Fig.26.

pRix11

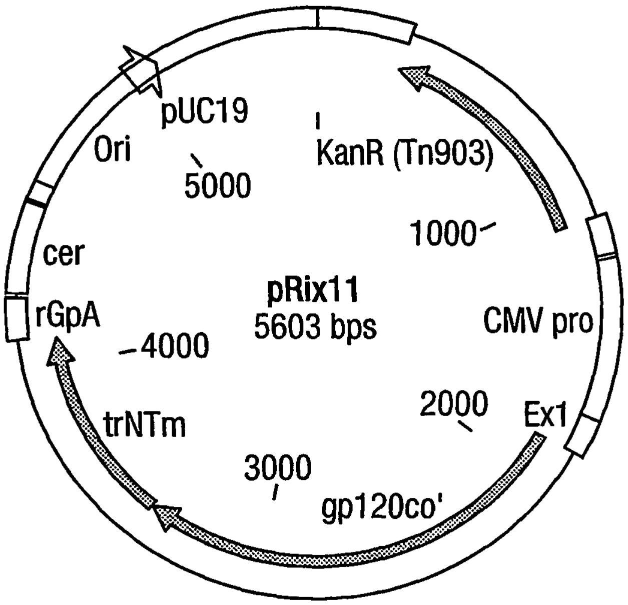


DNA sequence of insert

Aminoacid sequence of insert

MKVKETRKNYQHLWRWGTMLLGMLMICSAAEQLWVTVYYGVPVWKEATTTLFCASDAKAYDTEVHNVWATH
ACVPTDPNPQEVVLGNVTEYFNMWKNNMVDQMHEDIISLWDQSLKPCVKLTPLCVTLDCDDVNTTNSTTTT
SNGWTGEIRKGEIKNCSFNITTSIRDKVQKEYALFYNLDVVPIDDDNATTKNKTTRNFRLIHCNSSVMTQA
CPKVSFEPIPIHYCAPAGFAILKCNNKTFDGKGLCTNVSTVQCTHGIRPVVSTQLLLNGSLAEEEVVIRSD
NFMDNTKTIIVQLNESVAINCTRPNNNTRKGIHIGPGRAFYAARKIIGDIRQAHCNLSRAQWNNTLKQIVI
KLREHFGNKTIKFNQSSGGDPEIVRHSFNCGGEFFYCDTTQLFNSTWNGTEGNNTEGNSTITLPCRIKQII
NMWQEVGKAMYAPPIGGQIRCSSNITGLLLTRDGGTEGNGTENETEIFRPGGGDMRDNWRSELYKYKVVKV
EPLGVAPTRAKRRVVQRMVGFPVTPQVPLRPMTYKAAVDLSHFLKEKGGLEGLIHSQRRQDILDLWIYHTQ
GYFPDWQNYTPGPGVRYPLTFGWCYKLVPVEPDKVEEANKGENTSLLHPVSLHGMDDPEREVLEWRFDSRL
AFHHVARELHPEYFKNCTSEPVDPRLEPWKHPGSQPKTACTNCYCKKCCFHCQVCFITAALGISYGRKKRR
QRRRPPQGSQTHQVSLSKQPTSQSKGEPTGPKE [SEQ ID NO: 94]

# Fig.27.

pRix30

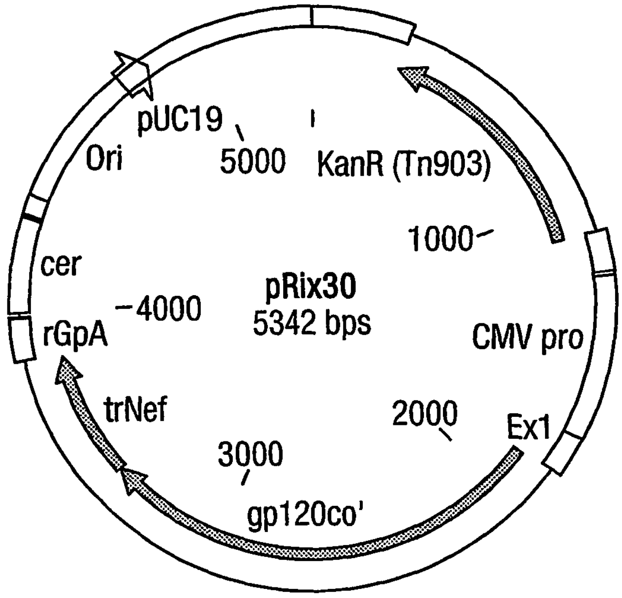


DNA sequence of insert

ATGAAGGTCAAGGAGACCAGAAAGAACTACCAGCATCTGTGGCGCTGGGGCACCATGCTCCTGGGAATGCT
GATGATCTGCTCCGCCGCCGAGCAGCTGTGGGTCACCGTCTACTACGGCGTGCCTGTGTGGAAGGAGGCCA
CGACCACCCTCTTCTGCGCGAGCGACGCCAAGGCCTACGACACGGAAGTGCATAACGTGTGGGCGACGCAT
GCTTGCGTGCCTACGGACCCCAACCCCCAGGAGGTGGTGCTGGGAAACGTGACCGAGTACTTCAACATGTG
GAAGAATAACATGGTGGATCAGATGCACGAGGACATCATCTCTCTGTGGGACCAGTCCCTGAAGCCCTGCG
TGAAGCTGACGCCTCTCTGCGTGACACTGGACTGTGACGACGTCAACACCACCAACAGCACTACCACCACC
AGCAACGGCTGGACCGGAGAGATTCGGAAGGGCGAGATCAAGAACTGCTCCTTCAATATCACGACCTCGAT
CAGAGACAAGGTGCAGAAGGAATACGCGCTGTTTTATAATCTCGATGTGGTCCCCATCGACGACGACAATG
CCACCACCAAGAACAAGACGACGCGTAATTTCAGACTCATTCACTGCAACAGCAGCGTCATGACGCAGGCC
TGCCCCAAGGTGTCCTTCGAACCAATCCCGATCCATTACTGTGCCCCTGCCGGATTCGCGATCCTCAAGTG
TAACAACAAGACCTTCGACGGGAAGGGCCTGTGCACCAACGTCAGCACGGTGCAGTGCACCCATGGCATCC
GCCCCGTCGTGAGCACCCAGCTGCTGCTGAACGGGTCCCTGGCTGAGGAGGAGGTGGTGATCCGGTCGGAC
AACTTCATGGACAACACCAAGACAATCATCGTCCAGCTGAACGAGTCTGTGGCGATTAACTGTACCCGGCC
TAACAACAACACCCGTAAGGGCATCCACATCGGGCCTGGACGGGCCTTCTATGCCGCCCGCAAGATCATCG
GCGACATCCGGCAGGCCCATTGCAACCTCTCCCGCGCCCAGTGGAATAACACCCTGAAGCAGATCGTGATC
AAGCTGAGAGAGCACTTTGGAAACAAGACCATCAAGTTCAATCAGAGTTCTGGCGGAGACCCCGAGATCGT
GCGGCACTCCTTCAACTGCGGGGGCGAGTTCTTCTACTGCGATACGACACAGCTCTTCAACTCCACCTGGA
ACGGCACCGAGGGCAACAACACAGAGGGAAACTCCACTATCACCCTCCCTTGCCGCATCAAGCAGATCATC
AACATGTGGCAGGAGGTGGGAAAGGCCATGTATGCCCCCCCCATCGGGGGCCAGATCCGCTGCTCCTCCAA
CATCACCGGCCTGCTGCTCACCAGAGACGGGGGCACCGAGGGCAACGGCACGGAGAACGAGACGGAGATCT
TCAGGCCCGGCGGCGGCGACATGAGGGATAACTGGCGGAGCGAGCTGTACAAGTACAAGGTGGTGAAGGTG
GAGCCGCTCGGCGTGGCCCCCACCCGGGCCAAGCGCCGCGTCGTGCAGAGAATGGTGGGTTTTCCAGTCAC
ACCTCAGGTACCTTTAAGACCAATGACTTACAAGGCAGCTGTAGATCTTAGCCACTTTTTAAAAGAAAAGG
GGGGACTGGAAGGGCTAATTCACTCCCAACGAAGACAAGATATCCTTGATCTGTGGATCTACCACACACAA
GGCTACTTCCCTGATTGGCAGAACTACACACCAGGGCCAGGGGTCAGATATCCACTGACCTTTGGATGGTG
CTACAAGCTAGTACCAGTTGAGCCAGATAAGGTAGAAGAGGCCAATAAAGGAGAGAACACCAGCTTGTTAC
ACCCTGTGAGCCTGCATGGAATGGATGACCCTGAGAGAGAAGTGTTAGAGTGGAGGTTTGACAGCCGCCTA
GCATTTCATCACGTGGCCCGAGAGCTGCATCCGGAGTACTTCAAGAACTGCTAA [SEQ ID NO: 95]

# Fig.27 (Cont).

**Aminoacid sequence of insert**

MKVKETRKNYQHLWRWGTMLLGMLMICSAAEQLWVTVYYGVPVWKEATTTLFCASDAKAYDTEVHNVWATH
ACVPTDPNPQEVVLGNVTEYFNMWKNNMVDQMHEDIISLWDQSLKPCVKLTPLCVTLDCDDVNTTNSTTTT
SNGWTGEIRKGEIKNCSFNITTSIRDKVQKEYALFYNLDVVPIDDDNATTKNKTTRNFRLIHCNSSVMTQA
CPKVSFEPIPIHYCAPAGFAILKCNNKTFDGKGLCTNVSTVQCTHGIRPVVSTQLLLNGSLAEEEVVIRSD
NFMDNTKTIIVQLNESVAINCTRPNNNTRKGIHIGPGRAFYAARKIIGDIRQAHCNLSRAQWNNTLKQIVI
KLREHFGNKTIKFNQSSGGDPEIVRHSFNCGGEFFYCDTTQLFNSTWNGTEGNNTEGNSTITLPCRIKQII
NMWQEVGKAMYAPPIGGQIRCSSNITGLLLTRDGGTEGNGTENETEIFRPGGGDMRDNWRSELYKYKVVKV
EPLGVAPTRAKRRVVQRMVGFPVTPQVPLRPMTYKAAVDLSHFLKEKGGLEGLIHSQRRQDILDLWIYHTQ
GYFPDWQNYTPGPGVRYPLTFGWCYKLVPVEPDKVEEANKGENTSLLHPVSLHGMDDPEREVLEWRFDSRL
AFHHVARELHPEYFKNC [SEQ ID NO: 96]

# Fig.28.

pRix32

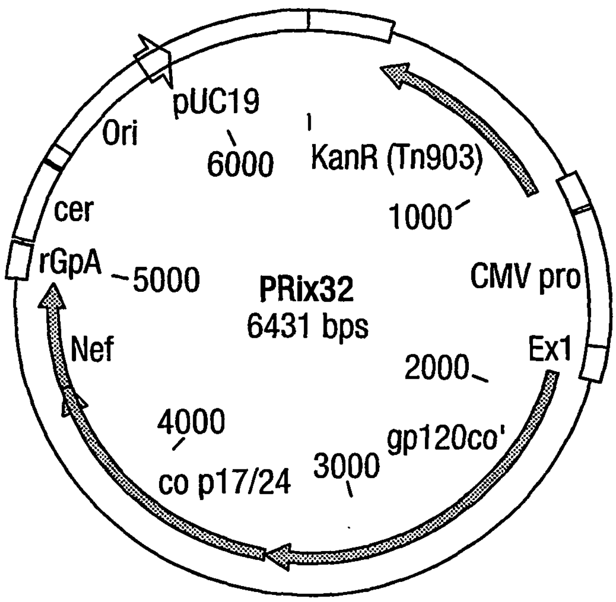


DNA sequence of insert

ATGAAGGTCAAGGAGACCAGAAAGAACTACCAGCATCTGTGGCGCTGGGGCACCATGCTCCTGGGAATGCT
GATGATCTGCTCCGCCGCCGAGCAGCTGTGGGTCACCGTCTACTACGGCGTGCCTGTGTGGAAGGAGGCCA
CGACCACCCTCTTCTGCGCGAGCGACGCCAAGGCCTACGACACGGAAGTGCATAACGTGTGGGCGACGCAT
GCTTGCGTGCCTACGGACCCCAACCCCCAGGAGGTGGTGCTGGGAAACGTGACCGAGTACTTCAACATGTG
GAAGAATAACATGGTGGATCAGATGCACGAGGACATCATCTCTCTGTGGGACCAGTCCCTGAAGCCCTGCG
TGAAGCTGACGCCTCTCTGCGTGACACTGGACTGTGACGACGTCAACACCACCAACAGCACTACCACCACC
AGCAACGGCTGGACCGGAGAGATTCGGAAGGGCGAGATCAAGAACTGCTCCTTCAATATCACGACCTCGAT
CAGAGACAAGGTGCAGAAGGAATACGCGCTGTTTTATAATCTCGATGTGGTCCCCATCGACGACGACAATG
CCACCACCAAGAACAAGACGACGCGTAATTTCAGACTCATTCACTGCAACAGCAGCGTCATGACGCAGGCC
TGCCCCAAGGTGTCCTTCGAACCAATCCCGATCCATTACTGTGCCCCTGCCGGATTCGCGATCCTCAAGTG
TAACAACAAGACCTTCGACGGAAGGGCCTGTGCACCAACGTCAGCACGGTGCAGTGCACCCATGGCATCC
GCCCCGTCGTGAGCACCCAGCTGCTGCTGAACGGGTCCCTGGCTGAGGAGGAGGTGGTGATCCGGTCGGAC
AACTTCATGGACAACACCAAGACAATCATCGTCCAGCTGAACGAGTCTGTGGCGATTAACTGTACCCGGCC
TAACAACAACACCCGTAAGGGCATCCACATCGGGCCTGGACGGGCCTTCTATGCCGCCCGCAAGATCATCG
GCGACATCCGGCAGGCCCATTGCAACCTCTCCCGCGCCCAGTGGAATAACACCCTGAAGCAGATCGTGATC
AAGCTGAGAGAGCACTTTGGAAACAAGACCATCAAGTTCAATCAGAGTTCTGGCGGAGACCCCGAGATCGT
GCGGCACTCCTTCAACTGCGGGGGCGAGTTCTTCTACTGCGATACGACACAGCTCTTCAACTCCACCTGGA
ACGGCACCGAGGGCAACAACACAGAGGGAAACTCCACTATCACCCTCCCTTGCCGCATCAAGCAGATCATC
AACATGTGGCAGGAGGTGGGAAAGGCCATGTATGCCCCCCCCATCGGGGGCCAGATCCGCTGCTCCTCCAA
CATCACCGGCCTGCTGCTCACCAGAGACGGGGGCACCGAGGGCAACGGCACGGAGAACGAGACGGAGATCT
TCAGGCCCGGCGGCGGCGACATGAGGGATAACTGGCGGAGCGAGCTGTACAAGTACAAGGTGGTGAAGGTG
GAGCCGCTCGGCGTGGCCCCCACCCGGGCCAAGCGCCGCGTCGTGCAGAGAATGGGTGCCCGAGCTTCGGT
ACTGTCTGGTGGAGAGCTGGACAGATGGGAGAAAATTAGGCTGCGCCCGGGAGGCAAAAAGAAATACAAGC
TCAAGCATATCGTGTGGGCCTCGAGGGAGCTTGAACGGTTTGCCGTGAACCCAGGCCTGCTGGAAACATCT
GAGGGATGTCGCCAGATCCTGGGGCAATTGCAGCCATCCCTCCAGACCGGGAGTGAAGAGCTGAGGTCCTT
GTATAACACAGTGGCTACCCTCTACTGCGTACACCAGAGGATCGAGATTAAGGATACCAAGGAGGCCTTGG
ACAAAATTGAGGAGGAGCAAAACAAGAGCAAGAAGAAGGCCCAGCAGGCAGCTGCTGACACTGGGCATAGC
AACCAGGTATCACAGAACTATCCTATTGTCCAAAACATTCAGGGCCAGATGGTTCATCAGGCCATCAGCCC
CCGGACGCTCAATGCCTGGGTGAAGGTTGTCGAAGAGAAGGCCTTTTCTCCTGAGGTTATCCCCATGTTCT

# Fig.28 (Cont).

CCGCTTTGAGTGAGGGGGCCACTCCTCAGGACCTCAATACAATGCTTAATACCGTGGGCGGCCATCAGGCC
GCCATGCAAATGTTGAAGGAGACTATCAACGAGGAGGCAGCCGAGTGGGACAGAGTGCATCCCGTCCACGC
TGGCCCAATCGCGCCCGGACAGATGCGGGAGCCTCGCGGCTCTGACATTGCCGGCACCACCTCTACACTGC
AAGAGCAAATCGGATGGATGACCAACAATCCTCCCATCCCAGTTGGAGAAATCTATAAACGGTGGATCATT
CTCGGTCTCAATAAAATTGTTAGAATGTACTCTCCGACATCCATCCTTGACATTAGACAGGGACCCAAAGA
GCCTTTTAGGGATTACGTCGACCGGTTTTATAAGACCCTGCGAGCAGAGCAGGCCTCTCAGGAGGTCAAAA
ACTGGATGACGGAGACACTCCTGGTACAGAACGCTAACCCCGACTGCAAAACAATCTTGAAGGCACTAGGC
CCGGCTGCCACCCTGGAAGAGATGATGACCGCCTGTCAGGGAGTAGGCGGACCCGGACACAAAGCCAGAGT
GTTGATGGTGGGTTTTCCAGTCACACCTCAGGTACCTTTAAGACCAATGACTTACAAGGCAGCTGTAGATC
TTAGCCACTTTTTAAAAGAAAAGGGGGGACTGGAAGGGCTAATTCACTCCCAAAGAAGACAAGATATCCTT
GATCTGTGGATCTACCACACACAAGGCTACTTCCCTGATTGGCAGAACTACACACCAGGGCCAGGGGTCAG
ATATCCACTGACCTTTGGATGGTGCTACAAGCTAGTACCAGTTGAGCCAGATAAGGTAGAAGAGGCCAATA
AAGGAGAGAACACCAGCTTGTTACACCCTGTGAGCCTGCATGGGATGGATGACCCGGAGAGAGAAGTGTTA
GAGTGGAGGTTTGACAGCCGCCTAGCATTTCATCACGTGGCCCGAGAGCTGCATCCGGAGTACTTCAAGAA
CTGCTGA [SEQ ID NO: 97]

Aminoacid sequence of insert

MKVKETRKNYQHLWRWGTMLLGMLMICSAAEQLWVTVYYGVPVWKEATTTLFCASDAKAYDTEVHNVWATH
ACVPTDPNPQEVVLGNVTEYFNMWKNNMVDQMHEDIISLWDQSLKPCVKLTPLCVTLDCDDVNTTNSTTTT
SNGWTGEIRKGEIKNCSFNITTSIRDKVQKEYALFYNLDVVPIDDDNATTKNKTTRNFRLIHCNSSVMTQA
CPKVSFEPIPIHYCAPAGFAILKCNNKTFDGKGLCTNVSTVQCTHGIRPVVSTQLLLNGSLAEEEVVIRSD
NFMDNTKTIIVQLNESVAINCTRPNNNTRKGIHIGPGRAFYAARKIIGDIRQAHCNLSRAQWNNTLKQIVI
KLREHFGNKTIKFNQSSGGDPEIVRHSFNCGGEFFYCDTTQLFNSTWNGTEGNNTEGNSTITLPCRIKQII
NMWQEVGKAMYAPPIGGQIRCSSNITGLLLTRDGGTEGNGTENETEIFRPGGGDMRDNWRSELYKYKVVKV
EPLGVAPTRAKRRVVQRMGARASVLSGGELDRWEKIRLRPGGKKKYKLKHIVWASRELERFAVNPGLLETS
EGCRQILGQLQPSLQTGSEELRSLYNTVATLYCVHQRIEIKDTKEALDKIEEEQNKSKKKAQQAAADTGHS
NQVSQNYPIVQNIQGQMVHQAISPRTLNAWVKVVEEKAFSPEVIPMFSALSEGATPQDLNTMLNTVGGHQA
AMQMLKETINEEAAEWDRVHPVHAGPIAPGQMREPRGSDIAGTTSTLQEQIGWMTNNPPIPVGEIYKRWII
LGLNKIVRMYSPTSILDIRQGPKEPFRDYVDRFYKTLRAEQASQEVKNWMTETLLVQNANPDCKTILKALG
PAATLEEMMTACQGVGGPGHKARVLMVGFPVTPQVPLRPMTYKAAVDLSHFLKEKGGLEGLIHSQRRQDIL
DLWIYHTQGYFPDWQNYTPGPGVRYPLTFGWCYKLVPVEPDKVEEANKGENTSLLHPVSLHGMDDPEREVL
EWRFDSRLAFHHVARELHPEYFKNC [SEQ ID NO: 98]

Fig.29.

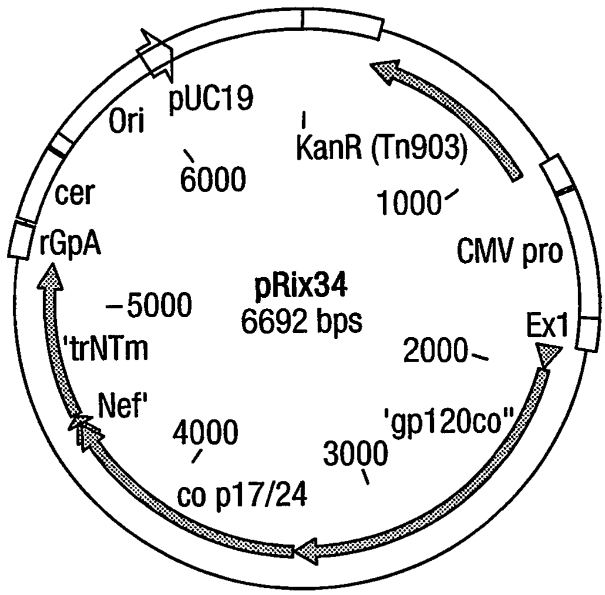


DNA sequence of insert

ATGAAGGTCAAGGAGACCAGAAAGAACTACCAGCATCTGTGGCGCTGGGGCACCATGCTCCTGGGAATGCT
GATGATCTGCTCCGCCGCCGAGCAGCTGTGGGTCACCGTCTACTACGGCGTGCCTGTGTGGAAGGAGGCCA
CGACCACCCTCTTCTGCGCGAGCGACGCCAAGGCCTACGACACGGAAGTGCATAACGTGTGGGCGACGCAT
GCTTGCGTGCCTACGGACCCCAACCCCCAGGAGGTGGTGCTGGGAAACGTGACCGAGTACTTCAACATGTG
GAAGAATAACATGGTGGATCAGATGCACGAGGACATCATCTCTCTGTGGGACCAGTCCCTGAAGCCCTGCG
TGAAGCTGACGCCTCTCTGCGTGACACTGGACTGTGACGACGTCAACACCACCAACAGCACTACCACCACC
AGCAACGGCTGGACCGGAGAGATTCGGAAGGGCGAGATCAAGAACTGCTCCTTCAATATCACGACCTCGAT
CAGAGACAAGGTGCAGAAGGAATACGCGCTGTTTTATAATCTCGATGTGGTCCCCATCGACGACGACAATG
CCACCACCAAGAACAAGACGACGCGTAATTTCAGACTCATTCACTGCAACAGCAGCGTCATGACGCAGGCC
TGCCCCAAGGTGTCCTTCGAACCAATCCCGATCCATTACTGTGCCCCTGCCGGATTCGCGATCCTCAAGTG
TAACAACAAGACCTTCGACGGGAAGGGCCTGTGCACCAACGTCAGCACGGTGCAGTGCACCCATGGCATCC
GCCCCGTCGTGAGCACCCAGCTGCTGCTGAACGGGTCCCTGGCTGAGGAGGAGGTGGTGATCCGGTCGGAC
AACTTCATGGACAACACCAAGACAATCATCGTCCAGCTGAACGAGTCTGTGGCGATTAACTGTACCCGGCC
TAACAACAACACCCGTAAGGGCATCCACATCGGGCCTGGACGGGCCTTCTATGCCGCCCGCAAGATCATCG
GCGACATCCGGCAGGCCCATTGCAACCTCTCCCGCGCCCAGTGGAATAACACCCTGAAGCAGATCGTGATC
AAGCTGAGAGAGCACTTTGGAAACAAGACCATCAAGTTCAATCAGAGTTCTGGCGGAGACCCCGAGATCGT
GCGGCACTCCTTCAACTGCGGGGGCGAGTTCTTCTACTGCGATACGACACAGCTCTTCAACTCCACCTGGA
ACGGCACCGAGGGCAACAACACAGAGGGAAACTCCACTATCACCCTCCCTTGCCGCATCAAGCAGATCATC
AACATGTGGCAGGAGGTGGGAAAGGCCATGTATGCCCCCCCCATCGGGGGCCAGATCCGCTGCTCCTCCAA
CATCACCGGCCTGCTGCTCACCAGAGACGGGGGCACCGAGGGCAACGGCACGGAGAACGAGACGGAGATCT
TCAGGCCCGGCGGCGGCGACATGAGGGATAACTGGCGGAGCGAGCTGTACAAGTACAAGGTGGTGAAGGTG
GAGCCGCTCGGCGTGGCCCCCACCCGGGCCAAGCGCCGCGTCGTGCAGAGAATGGGTGCCCGAGCTTCGGT
ACTGTCTGGTGGAGAGCTGGACAGATGGGAGAAAATTAGGCTGCGCCCGGGAGGCAAAAAGAAATACAAGC
TCAAGCATATCGTGTGGGCCTCGAGGGAGCTTGAACGGTTTGCCGTGAACCCAGGCCTGCTGGAAACATCT
GAGGGATGTCGCCAGATCCTGGGGCAATTGCAGCCATCCCTCCAGACCGGGAGTGAAGAGCTGAGGTCCTT
GTATAACACAGTGGCTACCCTCTACTGCGTACACCAGAGGATCGAGATTAAGGATACCAAGGAGGCCTTGG
ACAAAATTGAGGAGGAGCAAAACAAGAGCAAGAAGAAGGCCCAGCAGGCAGCTGCTGACACTGGGCATAGC
AACCAGGTATCACAGAACTATCCTATTGTCCAAAACATTCAGGGCCAGATGGTTCATCAGGCCATCAGCCC
CCGGACGCTCAATGCCTGGGTGAAGGTTGTCGAAGAGAAGGCCTTTTCTCCTGAGGTTATCCCCATGTTCT
CCGCTTTGAGTGAGGGGGCCACTCCTCAGGACCTCAATACAATGCTTAATACCGTGGGCGGCCATCAGGCC

# Fig.29 (Cont).

GCCATGCAAATGTTGAAGGAGACTATCAACGAGGAGGCAGCCGAGTGGGACAGAGTGCATCCCGTCCACGC
TGGCCCAATCGCGCCCGGACAGATGCGGGAGCCTCGCGGCTCTGACATTGCCGGCACCACCTCTACACTGC
AAGAGCAAATCGGATGGATGACCAACAATCCTCCCATCCCAGTTGGAGAAATCTATAAACGGTGGATCATT
CTCGGTCTCAATAAAATTGTTAGAATGTACTCTCCGACATCCATCCTTGACATTAGACAGGGACCCAAAGA
GCCTTTTAGGGATTACGTCGACCGGTTTTATAAGACCCTGCGAGCAGAGCAGGCCTCTCAGGAGGTCAAAA
ACTGGATGACGGAGACACTCCTGGTACAGAACGCTAACCCCGACTGCAAAACAATCTTGAAGGCACTAGGC
CCGGCTGCCACCCTGGAAGAGATGATGACCGCCTGTCAGGGAGTAGGCGGACCCGGACACAAAGCCAGAGT
GTTGATGGTGGGTTTTCCAGTCACACCTCAGGTACCTTTAAGACCAATGACTTACAAGGCAGCTGTAGATC
TTAGCCACTTTTTAAAAGAAAAGGGGGGACTGGAAGGGCTAATTCACTCCCAACGAAGACAAGATATCCTT
GATCTGTGGATCTACCACACACAAGGCTACTTCCCTGATTGGCAGAACTACACACCAGGGCCAGGGGTCAG
ATATCCACTGACCTTTGGATGGTGCTACAAGCTAGTACCAGTTGAGCCAGATAAGGTAGAAGAGGCCAATA
AAGGAGAGAACACCAGCTTGTTACACCCTGTGAGCCTGCATGGAATGGATGACCCTGAGAGAGAAGTGTTA
GAGTGGAGGTTTGACAGCCGCCTAGCATTTCATCACGTGGCCCGAGAGCTGCATCCGGAGTACTTCAAGAA
CTGCACTAGTGAGCCAGTAGATCCTAGACTAGAGCCCTGGAAGCATCCAGGAAGTCAGCCTAAAACTGCTT
GTACCAATTGCTATTGTAAAAAGTGTTGCTTTCATTGCCAAGTTTGTTTCATAACAGCTGCCTTAGGCATC
TCCTATGGCAGGAAGAAGCGGAGACAGCGACGAAGACCTCCTCAAGGCAGTCAGACTCATCAAGTTTCTCT
ATCAAAGCAACCCACCTCCCAATCCAAAGGGGAGCCGACAGGCCCGAAGGAATAA [SEQ ID NO: 99]

**Aminoacid sequence of insert**

MKVKETRKNYQHLWRWGTMLLGMLMICSAAEQLWVTVYYGVPVWKEATTTLFCASDAKAYDTEVHNVWATH
ACVPTDPNPQEVVLGNVTEYFNMWKNNMVDQMHEDIISLWDQSLKPCVKLTPLCVTLDCDDVNTTNSTTTT
SNGWTGEIRKGEIKNCSFNITTSIRDKVQKEYALFYNLDVVPIDDDNATTKNKTTRNFRLIHCNSSVMTQA
CPKVSFEPIPIHYCAPAGFAILKCNNKTFDGKGLCTNVSTVQCTHGIRPVVSTQLLLNGSLAEEEVVIRSD
NFMDNTKTIIVQLNESVAINCTRPNNNTRKGIHIGPGRAFYAARKIIGDIRQAHCNLSRAQWNNTLKQIVI
KLREHFGNKTIKFNQSSGGDPEIVRHSFNCGGEFFYCDTTQLFNSTWNGTEGNNTEGNSTITLPCRIKQII
NMWQEVGKAMYAPPIGGQIRCSSNITGLLLTRDGGTEGNGTENETEIFRPGGGDMRDNWRSELYKYKVVKV
EPLGVAPTRAKRRVVQRMGARASVLSGGELDRWEKIRLRPGGKKKYKLKHIVWASRELERFAVNPGLLETS
EGCRQILGQLQPSLQTGSEELRSLYNTVATLYCVHQRIEIKDTKEALDKIEEEQNKSKKKAQQAAADTGHS
NQVSQNYPIVQNIQGQMVHQAISPRTLNAWVKVVEEKAFSPEVIPMFSALSEGATPQDLNTMLNTVGGHQA
AMQMLKETINEEAAEWDRVHPVHAGPIAPGQMREPRGSDIAGTTSTLQEQIGWMTNNPPIPVGEIYKRWII
LGLNKIVRMYSPTSILDIRQGPKEPFRDYVDRFYKTLRAEQASQEVKNWMTETLLVQNANPDCKTILKALG
PAATLEEMMTACQGVGGPGHKARVLMVGFPVTPQVPLRPMTYKAAVDLSHFLKEKGGLEGLIHSQRRQDIL
DLWIYHTQGYFPDWQNYTPGPGVRYPLTFGWCYKLVPVEPDKVEEANKGENTSLLHPVSLHGMDDPEREVL
EWRFDSRLAFHHVARELHPEYFKNCTSEPVDPRLEPWKHPGSQPKTACTNCYCKKCCFHCQVCFITAALGI
SYGRKKRRQRRRPPQGSQTHQVSLSKQPTSQSKGEPTGPKE [SEQ ID NO: 100]

# Fig.30.

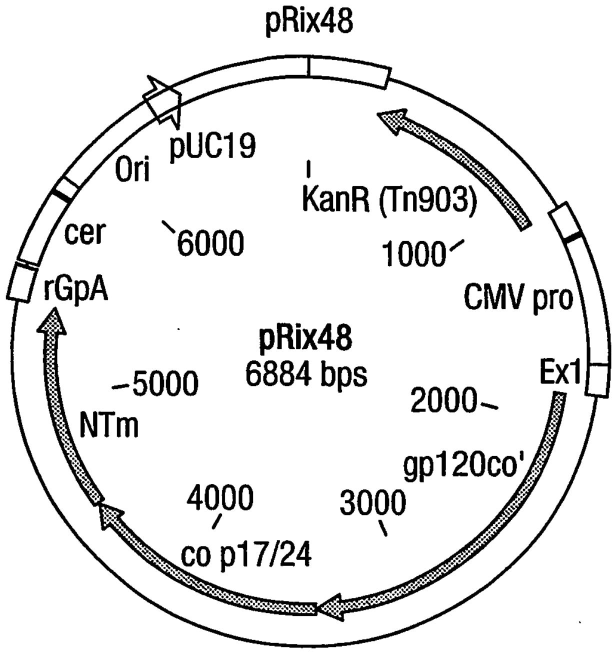


DNA sequence of insert

ATGAAGGTCAAGGAGACCAGAAAGAACTACCAGCATCTGTGGCGCTGGGGCACCATGCTCCTGGGAATGCT
GATGATCTGCTCCGCCGCCGAGCAGCTGTGGGTCACCGTCTACTACGGCGTGCCTGTGTGGAAGGAGGCCA
CGACCACCCTCTTCTGCGCGAGCGACGCCAAGGCCTACGACACGGAAGTGCATAACGTGTGGGCGACGCAT
GCTTGCGTGCCTACGGACCCCAACCCCCAGGAGGTGGTGCTGGGAAACGTGACCGAGTACTTCAACATGTG
GAAGAATAACATGGTGGATCAGATGCACGAGGACATCATCTCTCTGTGGGACCAGTCCCTGAAGCCCTGCG
TGAAGCTGACGCCTCTCTGCGTGACACTGGACTGTGACGACGTCAACACCACCAACAGCACTACCACCACC
AGCAACGGCTGGACCGGAGAGATTCGGAAGGGCGAGATCAAGAACTGCTCCTTCAATATCACGACCTCGAT
CAGAGACAAGGTGCAGAAGGAATACGCGCTGTTTTATAATCTCGATGTGGTCCCCATCGACGACGACAATG
CCACCACCAAGAACAAGACGACGCGTAATTTCAGACTCATTCACTGCAACAGCAGCGTCATGACGCAGGCC
TGCCCCAAGGTGTCCTTCGAACCAATCCCGATCCATTACTGTGCCCCTGCCGGATTCGCGATCCTCAAGTG
TAACAACAAGACCTTCGACGGGAAGGGCCTGTGCACCAACGTCAGCACGGTGCAGTGCACCCATGGCATCC
GCCCCGTCGTGAGCACCCAGCTGCTGCTGAACGGGTCCCTGGCTGAGGAGGAGGTGGTGATCCGGTCGGAC
AACTTCATGGACAACACCAAGACAATCATCGTCCAGCTGAACGAGTCTGTGGCGATTAACTGTACCCGGCC
TAACAACAACACCCGTAAGGGCATCCACATCGGGCCTGGACGGGCCTTCTATGCCGCCCGCAAGATCATCG
GCGACATCCGGCAGGCCCATTGCAACCTCTCCCGCGCCCAGTGGAATAACACCCTGAAGCAGATCGTGATC
AAGCTGAGAGAGCACTTTGGAAACAAGACCATCAAGTTCAATCAGAGTTCTGGCGGAGACCCCGAGATCGT
GCGGCACTCCTTCAACTGCGGGGGCGAGTTCTTCTACTGCGATACGACACAGCTCTTCAACTCCACCTGGA
ACGGCACCGAGGGCAACAACACAGAGGGAAACTCCACTATCACCCTCCCTTGCCGCATCAAGCAGATCATC
AACATGTGGCAGGAGGTGGGAAAGGCCATGTATGCCCCCCCCATCGGGGGCCAGATCCGCTGCTCCTCCAA
CATCACCGGCCTGCTGCTCACCAGAGACGGGGGCACCGAGGGCAACGGCACGGAGAACGAGACGGAGATCT
TCAGGCCCGGCGGCGGCGACATGAGGGATAACTGGCGGAGCGAGCTGTACAAGTACAAGGTGGTGAAGGTG
GAGCCGCTCGGCGTGGCCCCCACCCGGGCCAAGCGCCGCGTCGTGCAGAGAATGGGTGCCCGAGCTTCGGT
ACTGTCTGGTGGAGAGCTGGACAGATGGGAGAAAATTAGGCTGCGCCCGGGAGGCAAAAAGAAATACAAGC
TCAAGCATATCGTGTGGGCCTCGAGGGAGCTTGAACGGTTTGCCGTGAACCCAGGCCTGCTGGAAACATCT
GAGGGATGTCGCCAGATCCTGGGGCAATTGCAGCCATCCCTCCAGACCGGGAGTGAAGAGCTGAGGTCCTT
GTATAACACAGTGGCTACCCTCTACTGCGTACACCAGAGGATCGAGATTAAGGATACCAAGGAGGCCTTGG
ACAAAATTGAGGAGGAGCAAAACAAGAGCAAGAAGAAGGCCCAGCAGGCAGCTGCTGACACTGGGCATAGC
AACCAGGTATCACAGAACTATCCTATTGTCCAAAACATTCAGGGCCAGATGGTTCATCAGGCCATCAGCCC
CCGGACGCTCAATGCCTGGGTGAAGGTTGTCGAAGAGAAGGCCTTTTCTCCTGAGGTTATCCCCATGTTCT
CCGCTTTGAGTGAGGGGGCCACTCCTCAGGACCTCAATACAATGCTTAATACCGTGGCGGCCATCAGGCC
GCCATGCAAATGTTGAAGGAGACTATCAACGAGGAGGCAGCCGAGTGGGACAGAGTGCATCCCGTCCACGC

# Fig.30 (Cont).

TGGCCCAATCGCGCCCGGACAGATGCGGGAGCCTCGCGGCTCTGACATTGCCGGCACCACCTCTACACTGC
AAGAGCAAATCGGATGGATGACCAACAATCCTCCCATCCCAGTTGGAGAAATCTATAAACGGTGGATCATT
CTCGGTCTCAATAAAATTGTTAGAATGTACTCTCCGACATCCATCCTTGACATTAGACAGGGACCCAAAGA
GCCTTTTAGGGATTACGTCGACCGGTTTTATAAGACCCTGCGAGCAGAGCAGGCCTCTCAGGAGGTCAAAA
ACTGGATGACGGAGACACTCCTGGTACAGAACGCTAACCCCGACTGCAAAACAATCTTGAAGGCACTAGGC
CCGGCTGCCACCCTGGAAGAGATGATGACCGCCTGTCAGGGAGTAGGCGGACCCGGACACAAAGCCAGAGT
GTTGATGGGTGGCAAGTGGTCAAAAAGTAGTGTGGTTGGATGGCCTACTGTAAGGGAAAGAATGAGACGAG
CTGAGCCAGCAGCAGATGGGGTGGGAGCAGCATCTCGAGACCTGGAAAAACATGGAGCAATCACAAGTAGC
AATACAGCAGCTACCAATGCTGCTTGTGCCTGGCTAGAAGCACAAGAGGAGGAGGAGGTGGGTTTTCCAGT
CACACCTCAGGTACCTTTAAGACCAATGACTTACAAGGCAGCTGTAGATCTTAGCCACTTTTTAAAAGAAA
AGGGGGGACTGGAAGGGCTAATTCACTCCCAACGAAGACAAGATATCCTTGATCTGTGGATCTACCACACA
CAAGGCTACTTCCCTGATTGGCAGAACTACACACCAGGGCCAGGGGTCAGATATCCACTGACCTTTGGATG
GTGCTACAAGCTAGTACCAGTTGAGCCAGATAAGGTAGAAGAGGCCAATAAAGGAGAGAACACCAGCTTGT
TACACCCTGTGAGCCTGCATGGAATGGATGACCCTGAGAGAGAAGTGTTAGAGTGGAGGTTTGACAGCCGC
CTAGCATTTCATCACGTGGCCCGAGAGCTGCATCCGGAGTACTTCAAGAACTGCACTAGTGAGCCAGTAGA
TCCTAGACTAGAGCCCTGGAAGCATCCAGGAAGTCAGCCTAAAACTGCTTGTACCAATTGCTATTGTAAAA
AGTGTTGCTTTCATTGCCAAGTTTGTTTCATAACAGCTGCCTTAGGCATCTCCTATGGCAGGAAGAAGCGG
AGACAGCGACGAAGACCTCCTCAAGGCAGTCAGACTCATCAAGTTTCTCTATCAAAGCAACCCACCTCCCA
ATCCAAAGGGGAGCCGACAGGCCCGAAGGAATAA [SEQ ID NO: 101]

**Aminoacid sequence of insert**

MKVKETRKNYQHLWRWGTMLLGMLMICSAAEQLWVTVYYGVPVWKEATTTLFCASDAKAYDTEVHNVWATH
ACVPTDPNPQEVVLGNVTEYFNMWKNNMVDQMHEDIISLWDQSLKPCVKLTPLCVTLDCDDVNTTNSTTTT
SNGWTGEIRKGEIKNCSFNITTSIRDKVQKEYALFYNLDVVPIDDDNATTKNKTTRNFRLIHCNSSVMTQA
CPKVSFEPIPIHYCAPAGFAILKCNNKTFDGKGLCTNVSTVQCTHGIRPVVSTQLLLNGSLAEEEVVIRSD
NFMDNTKTIIVQLNESVAINCTRPNNNTRKGIHIGPGRAFYAARKIIGDIRQAHCNLSRAQWNNTLKQIVI
KLREHFGNKTIKFNQSSGGDPEIVRHSFNCGGEFFYCDTTQLFNSTWNGTEGNNTEGNSTITLPCRIKQII
NMWQEVGKAMYAPPIGGQIRCSSNITGLLLTRDGGTEGNGTENETEIFRPGGGDMRDNWRSELYKYKVVKV
EPLGVAPTRAKRRVVQRMGARASVLSGGELDRWEKIRLRPGGKKKYKLKHIVWASRELERFAVNPGLLETS
EGCRQILGQLQPSLQTGSEELRSLYNTVATLYCVHQRIEIKDTKEALDKIEEEQNKSKKKAQQAAADTGHS
NQVSQNYPIVQNIQGQMVHQAISPRTLNAWVKVVEEKAFSPEVIPMFSALSEGATPQDLNTMLNTVGGHQA
AMQMLKETINEEAAEWDRVHPVHAGPIAPGQMREPRGSDIAGTTSTLQEQIGWMTNNPPIPVGEIYKRWII
LGLNKIVRMYSPTSILDIRQGPKEPFRDYVDRFYKTLRAEQASQEVKNWMTETLLVQNANPDCKTILKALG
PAATLEEMMTACQGVGGPGHKARVLMGGKWSKSSVVGWPTVRERMRRAEPAADGVGAASRDLEKHGAITSS
NTAATNAACAWLEAQEEEEVGFPVTPQVPLRPMTYKAAVDLSHFLKEKGGLEGLIHSQRRQDILDLWIYHT
QGYFPDWQNYTPGPGVRYPLTFGWCYKLVPVEPDKVEEANKGENTSLLHPVSLHGMDDPEREVLEWRFDSR
LAFHHVARELHPEYFKNCTSEPVDPRLEPWKHPGSQPKTACTNCYCKKCCFHCQVCFITAALGISYGRKKR
RQRRRPPQGSQTHQVSLSKQPTSQSKGEPTGPKE [SEQ ID NO: 102]

# Fig.31.

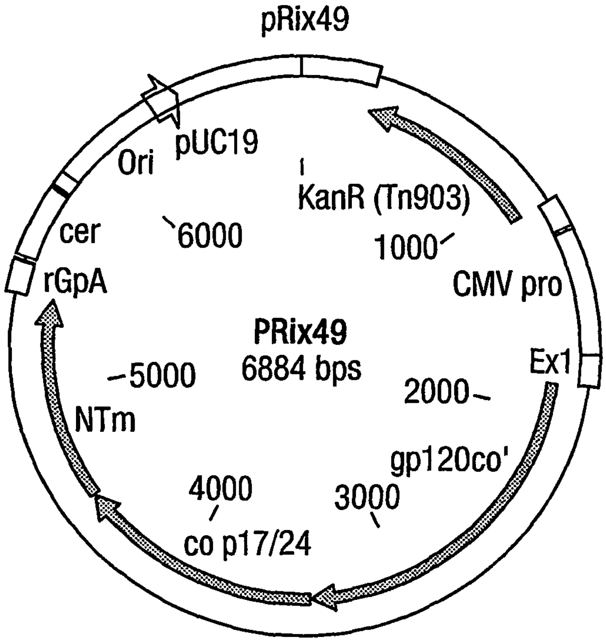


DNA sequence of insert

ATGAAGGTCAAGGAGACCAGAAAGAACTACCAGCATCTGTGGCGCTGGGGCACCATGCTCCTGGGAATGCT
GATGATCTGCTCCGCCGCCGAGCAGCTGTGGGTCACCGTCTACTACGGCGTGCCTGTGTGGAAGGAGGCCA
CGACCACCCTCTTCTGCGCGAGCGACGCCAAGGCCTACGACACGGAAGTGCATAACGTGTGGGCGACGCAT
GCTTGCGTGCCTACGGACCCCAACCCCCAGGAGGTGGTGCTGGGAAACGTGACCGAGTACTTCAACATGTG
GAAGAATAACATGGTGGATCAGATGCACGAGGACATCATCTCTCTGTGGGACCAGTCCCTGAAGCCCTGCG
TGAAGCTGACGCCTCTCTGCGTGACACTGGACTGTGACGACGTCAACACCACCAACAGCACTACCACCACC
AGCAACGGCTGGACCGGAGAGATTCGGAAGGGCGAGATCAAGAACTGCTCCTTCAATATCACGACCTCGAT
CAGAGACAAGGTGCAGAAGGAATACGCGCTGTTTTATAATCTCGATGTGGTCCCCATCGACGACGACAATG
CCACCACCAAGAACAAGACGACGCGTAATTTCAGACTCATTCACTGCAACAGCAGCGTCATGACGCAGGCC
TGCCCCAAGGTGTCCTTCGAACCAATCCCGATCCATTACTGTGCCCCTGCCGGATTCGCGATCCTCAAGTG
TAACAACAAGACCTTCGACGGGAAGGGCCTGTGCACCAACGTCAGCACGGTGCAGTGCACCCATGGCATCC
GCCCCGTCGTGAGCACCCAGCTGCTGCTGAACGGGTCCCTGGCTGAGGAGGAGGTGGTGATCCGGTCGGAC
AACTTCATGGACAACACCAAGACAATCATCGTCCAGCTGAACGAGTCTGTGGCGATTAACTGTACCCGGCC
TAACAACAACACCCGTAAGGGCATCCACATCGGGCCTGGACGGGCCTTCTATGCCGCCCGCAAGATCATCG
GCGACATCCGGCAGGCCCATTGCAACCTCTCCCGCGCCCAGTGGAATAACACCCTGAAGCAGATCGTGATC
AAGCTGAGAGAGCACTTTGGAAACAAGACCATCAAGTTCAATCAGAGTTCTGGCGGAGACCCCGAGATCGT
GCGGCACTCCTTCAACTGCGGGGGCGAGTTCTTCTACTGCGATACGACACAGCTCTTCAACTCCACCTGGA
ACGGCACCGAGGGCAACAACACAGAGGGAAACTCCACTATCACCCTCCCTTGCCGCATCAAGCAGATCATC
AACATGTGGCAGGAGGTGGGAAAGGCCATGTATGCCCCCCCCATCGGGGGCCAGATCCGCTGCTCCTCCAA
CATCACCGGCCTGCTGCTCACCAGAGACGGGGGCACCGAGGGCAACGGCACGGAGAACGAGACGGAGATCT
TCAGGCCCGGCGGCGGCGACATGAGGGATAACTGGCGGAGCGAGCTGTACAAGTACAAGGTGGTGAAGGTG
GAGCCGCTCGGCGTGGCCCCCACCCGGGCCAAGCGCCGCGTCGTGCAGAGAATGGGTGCCCGAGCTTCGGT
ACTGTCTGGTGGAGAGCTGGACAGATGGGAGAAAATTAGGCTGCGCCCGGGAGGCAAAAAGAAATACAAGC
TCAAGCATATCGTGTGGGCCTCGAGGGAGCTTGAACGGTTTGCCGTGAACCCAGGCCTGCTGGAAACATCT
GAGGGATGTCGCCAGATCCTGGGGCAATTGCAGCCATCCCTCCAGACCGGGAGTGAAGAGCTGAGGTCCTT
GTATAACACAGTGGCTACCCTCTACTGCGTACACCAGAGGATCGAGATTAAGGATACCAAGGAGGCCTTGG
ACAAAATTGAGGAGGAGCAAAACAAGAGCAAGAAGAAGGCCCAGCAGGCAGCTGCTGACACTGGGCATAGC
AACCAGGTATCACAGAACTATCCTATTGTCCAAAACATTCAGGGCCAGATGGTTCATCAGGCCATCAGCCC
CCGGACGCTCAATGCCTGGGTGAAGGTTGTCGAAGAGAAGGCCTTTTCTCCTGAGGTTATCCCCATGTTCT

# Fig.31 (Cont).

CCGCTTTGAGTGAGGGGGCCACTCCTCAGGACCTCAATACAATGCTTAATACCGTGGGCGGCCATCAGGCC
GCCATGCAAATGTTGAAGGAGACTATCAACGAGGAGGCAGCCGAGTGGGACAGAGTGCATCCCGTCCACGC
TGGCCCAATCGCGCCCGGACAGATGCGGGAGCCTCGCGGCTCTGACATTGCCGGCACCACCTCTACACTGC
AAGAGCAAATCGGATGGATGACCAACAATCCTCCCATCCCAGTTGGAGAAATCTATAAACGGTGGATCATT
CTCGGTCTCAATAAAATTGTTAGAATGTACTCTCCGACATCCATCCTTGACATTAGACAGGGACCCAAAGA
GCCTTTTAGGGATTACGTCGACCGGTTTTATAAGACCCTGCGAGCAGAGCAGGCCTCTCAGGAGGTCAAAA
ACTGGATGACGGAGACACTCCTGGTACAGAACGCTAACCCCGACTGCAAAACAATCTTGAAGGCACTAGGC
CCGGCTGCCACCCTGGAAGAGATGATGACCGCCTGTCAGGGAGTAGGCGGACCCGGACACAAAGCCAGAGT
GTTGATGGGTGGCAAGTGGTCAAAAAGTAGTGTGGTTGGATGGCCTACTGTAAGGGAAAGAATGAGACGAG
CTGAGCCAGCAGCAGATGGGGTGGGAGCAGCATCTCGAGACCTGGAAAAACATGGAGCAATCACAAGTAGC
AATACAGCAGCTACCAATGCTGCTTGTGCCTGGCTAGAAGCACAAGAGGAGGAGGAGGTGGGTTTTCCAGT
CACACCTCAGGTACCTTTAAGACCAATGACTTACAAGGCAGCTGTAGATCTTAGCCACTTTTTAAAAGAAA
AGGGGGGACTGGAAGGGCTAATTCACTCCCAACGAAGACAAGATATCCTTGATCTGTGGATCTACCACACA
CAAGGCTACTTCCCTGATTGGCAGAACTACACACCAGGGCCAGGGGTCAGATATCCACTGACCTTTGGATG
GTGCTACAAGCTAGTACCAGTTGAGCCAGATAAGGTAGAAGAGGCCAATAAAGGAGAGAACACCAGCGCCT
TACACCCTGTGAGCCTGCATGGAATGGATGACCCTGAGAGAGAAGTGTTAGAGTGGAGGTTTGACAGCCGC
CTAGCATTTCATCACGTGGCCCGAGAGCTGCATCCGGAGTACTTCAAGAACTGCACTAGTGAGCCAGTAGA
TCCTAGACTAGAGCCCTGGAAGCATCCAGGAAGTCAGCCTAAAACTGCTTGTACCAATTGCTATTGTAAAA
AGTGTTGCTTTCATTGCCAAGTTTGTTTCATAACAGCTGCCTTAGGCATCTCCTATGGCAGGAAGAAGCGG
AGACAGCGACGAAGACCTCCTCAAGGCAGTCAGACTCATCAAGTTTCTCTATCAAAGCAACCCACCTCCCA
ATCCAAAGGGGAGCCGACAGGCCCGAAGGAATAA [SEQ ID NO: 103]

**Aminoacid sequence of insert**

MKVKETRKNYQHLWRWGTMLLGMLMICSAAEQLWVTVYYGVPVWKEATTTLFCASDAKAYDTEVHNVWATH
ACVPTDPNPQEVVLGNVTEYFNMWKNNMVDQMHEDIISLWDQSLKPCVKLTPLCVTLDCDDVNTTNSTTTT
SNGWTGEIRKGEIKNCSFNITTSIRDKVQKEYALFYNLDVVPIDDDNATTKNKTTRNFRLIHCNSSVMTQA
CPKVSFEPIPIHYCAPAGFAILKCNNKTFDGKGLCTNVSTVQCTHGIRPVVSTQLLLNGSLAEEEVVIRSD
NFMDNTKTIIVQLNESVAINCTRPNNNTRKGIHIGPGRAFYAARKIIGDIRQAHCNLSRAQWNNTLKQIVI
KLREHFGNKTIKFNQSSGGDPEIVRHSFNCGGEFFYCDTTQLFNSTWNGTEGNNTEGNSTITLPCRIKQII
NMWQEVGKAMYAPPIGGQIRCSSNITGLLLTRDGGTEGNGTENETEIFRPGGGDMRDNWRSELYKYKVVKV
EPLGVAPTRAKRRVVQRMGARASVLSGGELDRWEKIRLRPGGKKKYKLKHIVWASRELERFAVNPGLLETS
EGCRQILGQLQPSLQTGSEELRSLYNTVATLYCVHQRIEIKDTKEALDKIEEEQNKSKKKAQQAAADTGHS
NQVSQNYPIVQNIQGQMVHQAISPRTLNAWVKVVEEKAFSPEVIPMFSALSEGATPQDLNTMLNTVGGHQA
AMQMLKETINEEAAEWDRVHPVHAGPIAPGQMREPRGSDIAGTTSTLQEQIGWMTNNPPIPVGEIYKRWII
LGLNKIVRMYSPTSILDIRQGPKEPFRDYVDRFYKTLRAEQASQEVKNWMTETLLVQNANPDCKTILKALG
PAATLEEMMTACQGVGGPGHKARVLMGGKWSKSSVVGWPTVRERMRRAEPAADGVGAASRDLEKHGAITSS
NTAATNAACAWLEAQEEEEVGFPVTPQVPLRPMTYKAAVDLSHFLKEKGGLEGLIHSQRRQDILDLWIYHT
QGYFPDWQNYTPGPGVRYPLTFGWCYKLVPVEPDKVEEANKGENTSALHPVSLHGMDDPEREVLEWRFDSR
LAFHHVARELHPEYFKNCTSEPVDPRLEPWKHPGSQPKTACTNCYCKKCFHCQVCFITAALGISYGRKKR
RQRRRPPQGSQTHQVSLSKQPTSQSKGEPTGPKE [SEQ ID NO: 104]

Fig.32.

pT-RNG

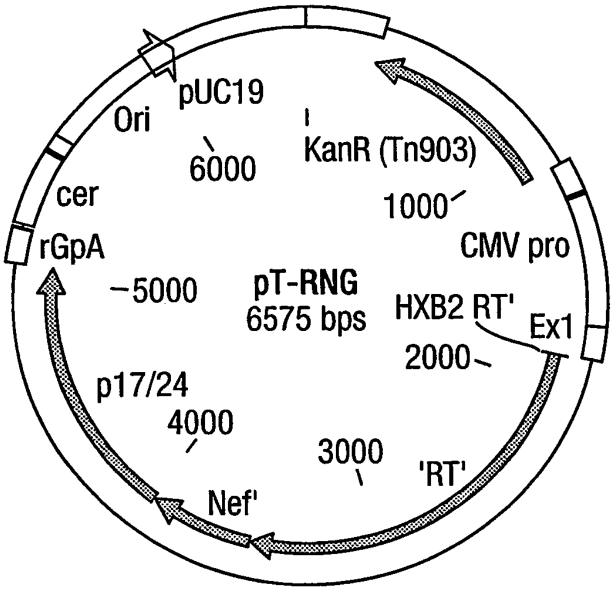


DNA sequence of insert:

ATGGGCCCCATCAGTCCCATCGAGACCGTGCCGGTGAAGCTGAAACCCGGGATGGACGGCCCCAAGGTCAA
GCAGTGGCCACTCACCGAGGAGAAGATCAAGGCCCTGGTGGAGATCTGCACCGAGATGGAGAAAGAGGGCA
AGATCAGCAAGATCGGGCCTGAGAACCCATACAACACCCCCGTGTTTGCCATCAAGAAGAAGGACAGCACC
AAGTGGCGCAAGCTGGTGGATTTCCGGGAGCTGAATAAGCGGACCCAGGATTTCTGGGAGGTCCAGCTGGG
CATCCCCCATCCGGCCGGCCTGAAGAAGAAGAAGAGCGTGACCGTGCTGGACGTGGGCGACGCTTACTTCA
GCGTCCCTCTGGACGAGGACTTTAGAAAGTACACCGCCTTTACCATCCCATCTATCAACAACGAGACCCCT
GGCATCAGATATCAGTACAACGTCCTCCCCCAGGGCTGGAAGGGCTCTCCCGCCATTTTCCAGAGCTCCAT
GACCAAGATCCTGGAGCCGTTTCGGAAGCAGAACCCCGATATCGTCATCTACCAGTACATGGACGACCTGT
ACGTGGGCTCTGACCTGGAAATCGGGCAGCATCGCACGAAGATTGAGGAGCTGAGGCAGCATCTGCTGAGA
TGGGGCCTGACCACTCCGGACAAGAAGCATCAGAAGGAGCCGCCATTCCTGAAGATGGGCTACGAGCTCCA
TCCCGACAAGTGGACCGTGCAGCCTATCGTCCTCCCCGAGAAGGACAGCTGGACCGTGAACGACATCCAGA
AGCTGGTGGGCAAGCTCAACTGGGCTAGCCAGATCTATCCCGGGATCAAGGTGCGCCAGCTCTGCAAGCTG
CTGCGCGGCACCAAGGCCCTGACCGAGGTGATTCCCCTCACGGAGGAAGCCGAGCTCGAGCTGGCTGAGAA
CCGGGAGATCCTGAAGGAGCCCGTGCACGGCGTGTACTATGACCCCTCCAAGGACCTGATCGCCGAAATCC
AGAAGCAGGGCCAGGGGCAGTGGACATACCAGATTTACCAGGAGCCTTTCAAGAACCTCAAGACCGGCAAG
TACGCCCGCATGAGGGGCGCCCACACCAACGATGTCAAGCAGCTGACCGAGGCCGTCCAGAAGATCACGAC
CGAGTCCATCGTGATCTGGGGGAAGACACCCAAGTTCAAGCTGCCTATCCAGAAGGAGACCTGGGAGACGT
GGTGGACCGAATATTGGCAGGCCACCTGGATTCCCGAGTGGGAGTTCGTGAATACACCTCCTCTGGTGAAG
CTGTGGTACCAGCTCGAGAAGGAGCCCATCGTGGGCGCGGAGACATTCTACGTGGACGGCGCGGCCAACCG
CGAAACAAAGCTCGGGAAGGCCGGGTACGTCACCAACCGGGGCCGCCAGAAGGTCGTCACCCTGACCGACA
CCACCAACCAGAAGACGGAGCTGCAGGCCATCTATCTCGCTCTCCAGGACTCCGGCCTGGAGGTGAACATC
GTGACGGACAGCCAGTACGCGCTGGGCATTATTCAGGCCCAGCCGGACCAGTCCGAGAGCGAACTGGTGAA
CCAGATTATCGAGCAGCTGATCAAGAAAGAGAAGGTCTACCTCGCCTGGGTCCCGGCCCATAAGGGCATTG
GCGGCAACGAGCAGGTCGACAAGCTGGTGAGTGCGGGGATTAGAAAGGTGCTGATGGTGGGTTTTCCAGTC
ACACCTCAGGTACCTTTAAGACCAATGACTTACAAGGCAGCTGTAGATCTTAGCCACTTTTTAAAAGAAAA
GGGGGGACTGGAAGGGCTAATTCACTCCCAAAGAAGACAAGATATCCTTGATCTGTGGATCTACCACACAC
AAGGCTACTTCCCTGATTGGCAGAACTACACACCAGGGCCAGGGGTCAGATATCCACTGACCTTTGGATGG
TGCTACAAGCTAGTACCAGTTGAGCCAGATAAGGTAGAAGAGGCCAATAAAGGAGAGAACACCAGCTTGTT

# Fig.32 (Cont).

ACACCCTGTGAGCCTGCATGGGATGGATGACCCGGAGAGAGAAGTGTTAGAGTGGAGGTTTGACAGCCGCC
TAGCATTTCATCACGTGGCCCGAGAGCTGCATCCGGAGTACTTCAAGAACTGCATGGGTGCCCGAGCTTCG
GTACTGTCTGGTGGAGAGCTGGACAGATGGGAGAAAATTAGGCTGCGCCCGGGAGGCAAAAAGAAATACAA
GCTCAAGCATATCGTGTGGGCCTCGAGGGAGCTTGAACGGTTTGCCGTGAACCCAGGCCTGCTGGAAACAT
CTGAGGGATGTCGCCAGATCCTGGGGCAATTGCAGCCATCCCTCCAGACCGGGAGTGAAGAGCTGAGGTCC
TTGTATAACACAGTGGCTACCCTCTACTGCGTACACCAGAGGATCGAGATTAAGGATACCAAGGAGGCCTT
GGACAAAATTGAGGAGGAGCAAAACAAGAGCAAGAAGAAGGCCCAGCAGGCAGCTGCTGACACTGGGCATA
GCAACCAGGTATCACAGAACTATCCTATTGTCCAAAACATTCAGGGCCAGATGGTTCATCAGGCCATCAGC
CCCCGGACGCTCAATGCCTGGGTGAAGGTTGTCGAAGAGAAGGCCTTTTCTCCTGAGGTTATCCCCATGTT
CTCCGCTTTGAGTGAGGGGGCCACTCCTCAGGACCTCAATACAATGCTTAATACCGTGGGCGGCCATCAGG
CCGCCATGCAAATGTTGAAGGAGACTATCAACGAGGAGGCAGCCGAGTGGGACAGAGTGCATCCCGTCCAC
GCTGGCCCAATCGCGCCCGGACAGATGCGGGAGCCTCGCGGCTCTGACATTGCCGGCACCACCTCTACACT
GCAAGAGCAAATCGGATGGATGACCAACAATCCTCCCATCCCAGTTGGAGAAATCTATAAACGGTGGATCA
TCCTGGGCCTGAACAAGATCGTGCGCATGTACTCTCCGACATCCATCCTTGACATTAGACAGGGACCCAAA
GAGCCTTTTAGGGATTACGTCGACCGGTTTTATAAGACCCTGCGAGCAGAGCAGGCCTCTCAGGAGGTCAA
AAACTGGATGACGGAGACACTCCTGGTACAGAACGCTAACCCCGACTGCAAAACAATCTTGAAGGCACTAG
GCCCGGCTGCCACCCTGGAAGAGATGATGACCGCCTGTCAGGGAGTAGGCGGACCCGGACACAAAGCCAGA
GTGTTGTAA [SEQ ID NO: 88]

Amino acid sequence of insert:

MGPISPIETVPVKLKPGMDGPKVKQWPLTEEKIKALVEICTEMEKEGKISKIGPENPYNTPVFAIKKKDST
KWRKLVDFRELNKRTQDFWEVQLGIPHPAGLKKKKSVTVLDVGDAYFSVPLDEDFRKYTAFTIPSINNETP
GIRYQYNVLPQGWKGSPAIFQSSMTKILEPFRKQNPDIVIYQYMDDLYVGSDLEIGQHRTKIEELRQHLLR
WGLTTPDKKHQKEPPFLKMGYELHPDKWTVQPIVLPEKDSWTVNDIQKLVGKLNWASQIYPGIKVRQLCKL
LRGTKALTEVIPLTEEAELELAENREILKEPVHGVYYDPSKDLIAEIQKQGQGQWTYQIYQEPFKNLKTGK
YARMRGAHTNDVKQLTEAVQKITTESIVIWGKTPKFKLPIQKETWETWWTEYWQATWIPEWEFVNTPPLVK
LWYQLEKEPIVGAETFYVDGAANRETKLGKAGYVTNRGRQKVVTLTDTTNQKTELQAIYLALQDSGLEVNI
VTDSQYALGIIQAQPDQSESELVNQIIEQLIKKEKVYLAWVPAHKGIGGNEQVDKLVSAGIRKVLMVGFPV
TPQVPLRPMTYKAAVDLSHFLKEKGGLEGLIHSQRRQDILDLWIYHTQGYFPDWQNYTPGPGVRYPLTFGW
CYKLVPVEPDKVEEANKGENTSLLHPVSLHGMDDPEREVLEWRFDSRLAFHHVARELHPEYFKNCMGARAS
VLSGGELDRWEKIRLRPGGKKKYKLKHIVWASRELERFAVNPGLLETSEGCRQILGQLQPSLQTGSEELRS
LYNTVATLYCVHQRIEIKDTKEALDKIEEEQNKSKKKAQQAAADTGHSNQVSQNYPIVQNIQGQMVHQAIS
PRTLNAWVKVVEEKAFSPEVIPMFSALSEGATPQDLNTMLNTVGGHQAAMQMLKETINEEAAEWDRVHPVH
AGPIAPGQMREPRGSDIAGTTSTLQEQIGWMTNNPPIPVGEIYKRWIILGLNKIVRMYSPTSILDIRQGPK
EPFRDYVDRFYKTLRAEQASQEVKNWMTETLLVQNANPDCKTILKALGPAATLEEMMTACQGVGGPGHKAR
VL [SEQ ID NO: 89]

Fig.33.

A schematic representation of the constructs and associated expression data is shown below

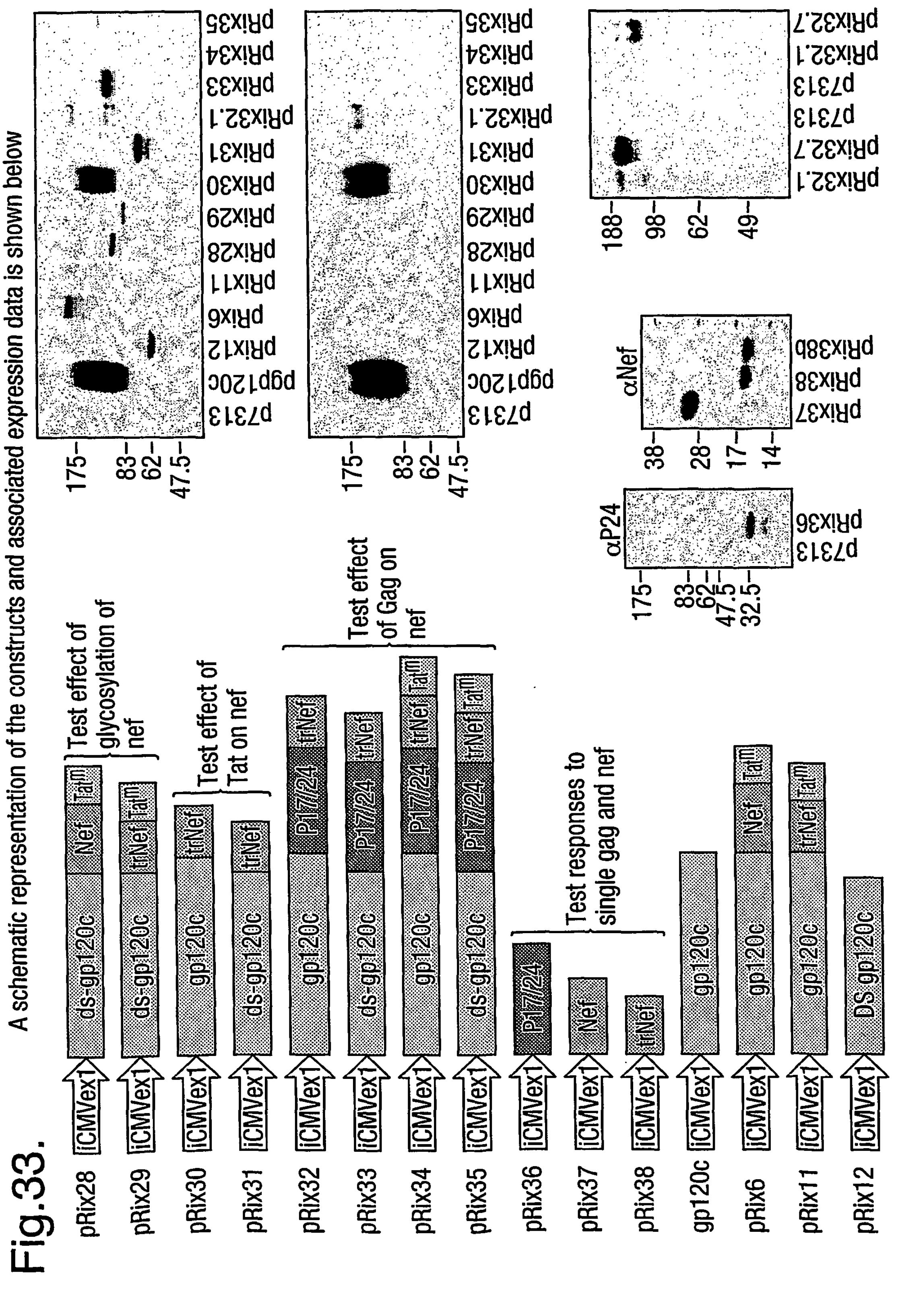

Fig.34.

A Schematic representation of further constructs

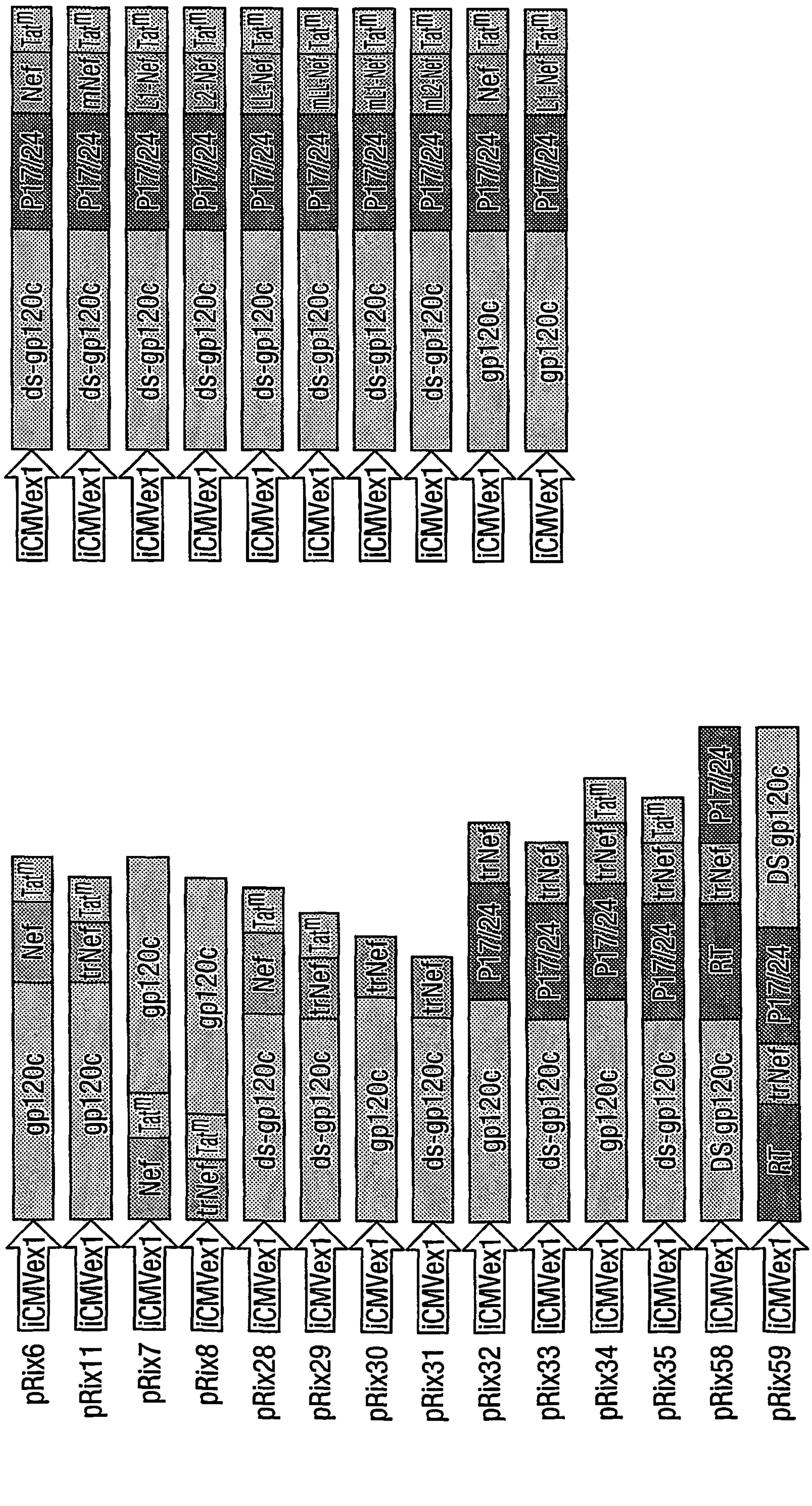

# Fig.35.

Expression data (anti-Nef) for dsgp120/Gag/Nef/Tat fusions with mutations in Nef (pRix40-47)

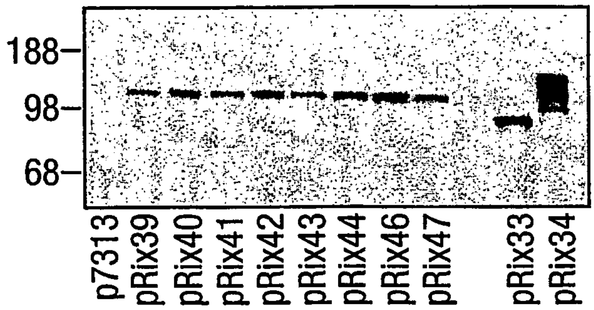


Expression data (anti-Nef) for dsgp120/Gag/Nef/Tat fusions with glycosylated gp120 (pRix48 and pRix49)

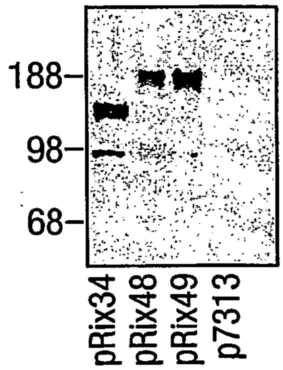


Expression data (anti-Nef) for the quadrivalent fusion proteins containing RT, Nef, Gag and dsgp120, compared to expression of the RT,Nef,Gag fusion alone

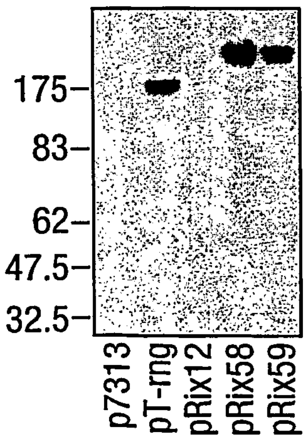

Fig.36.

Responses to Gp120 peptide by IFN-gamma ELIspot at 7 days post-boost (Day 35)

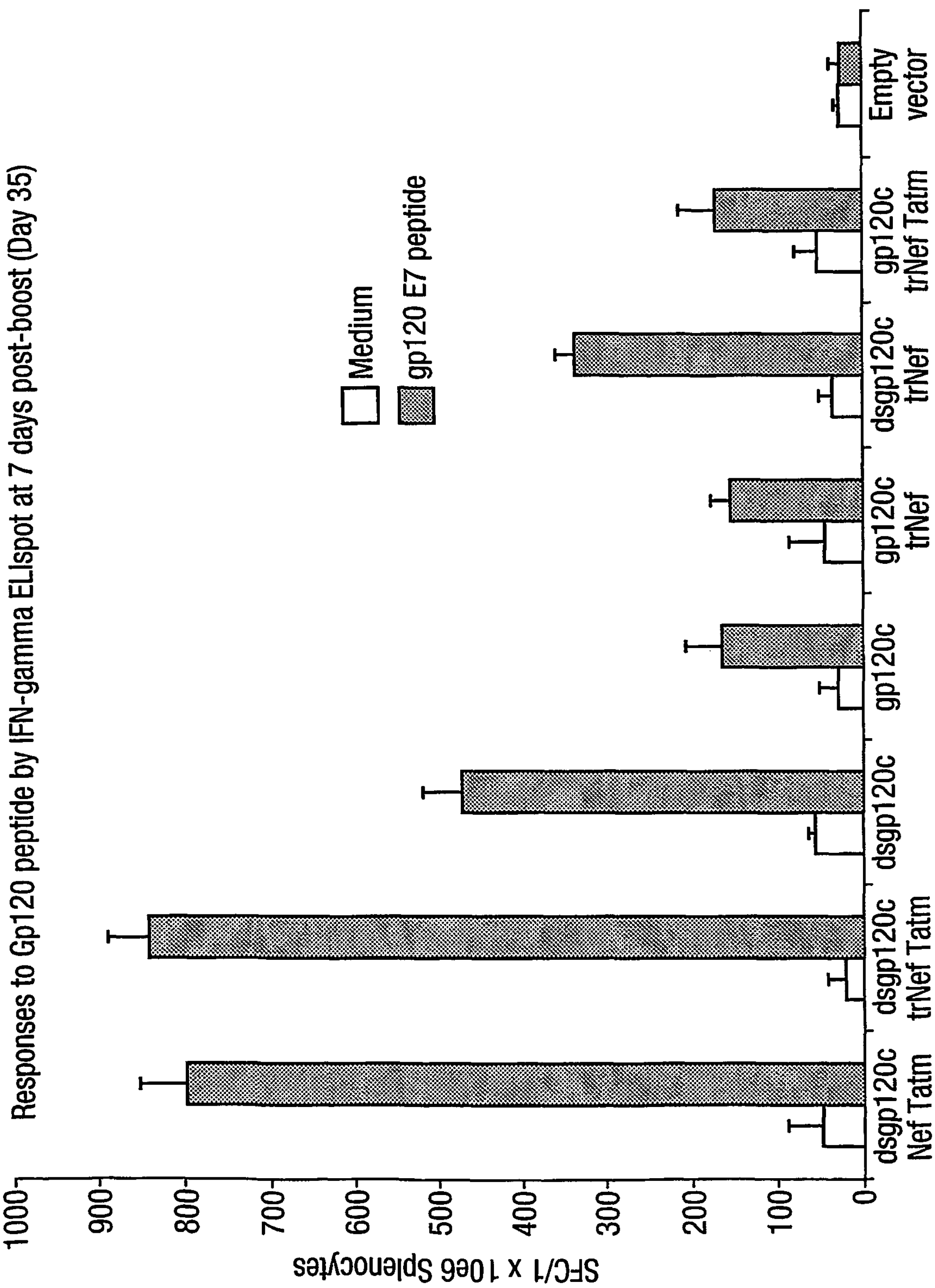

Fig.37.

Responses to gp120 peptide by IFN-gamma ELIspot at 7 days-boost (Day 35)

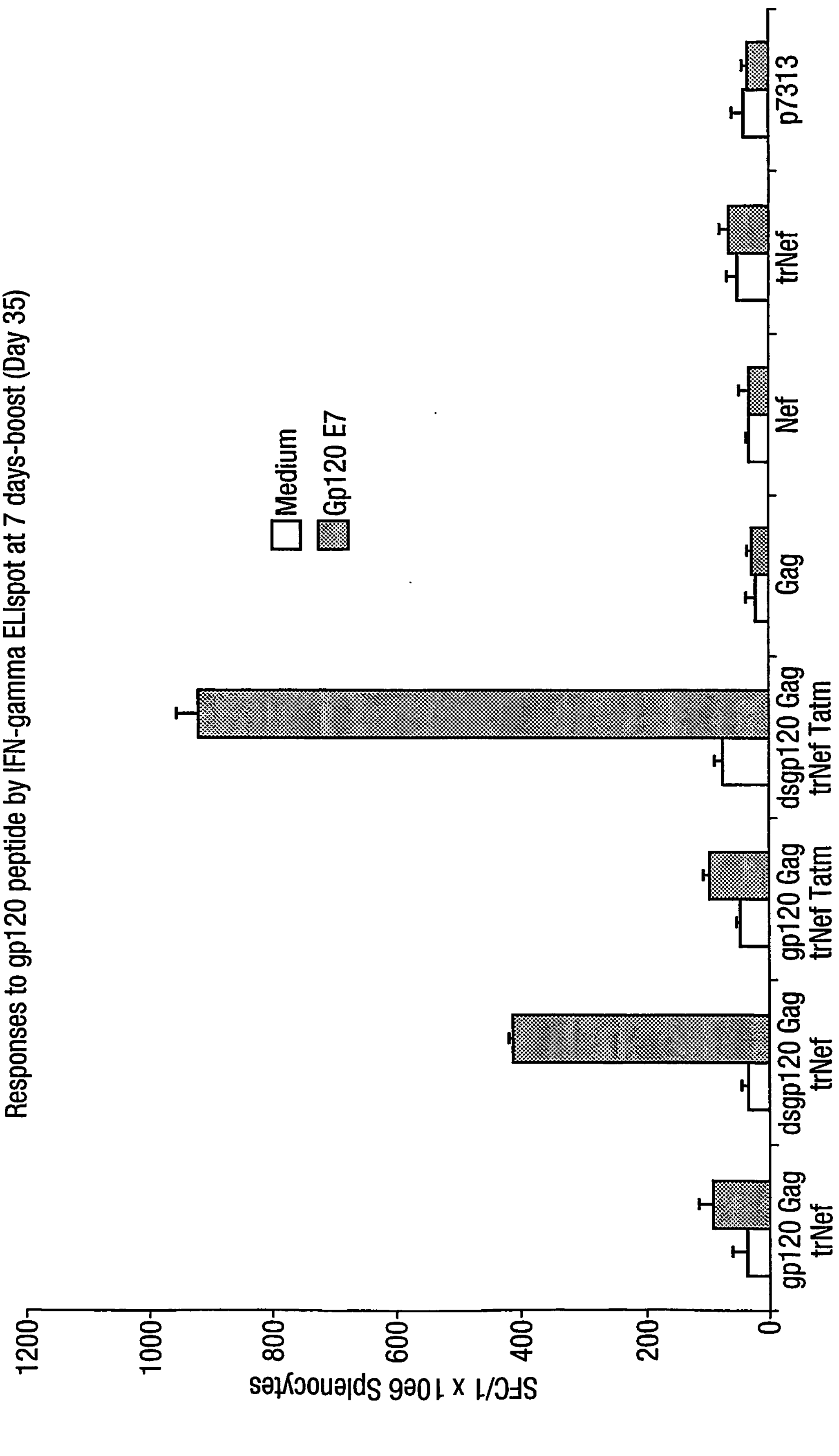

Fig.38.

Response to in vitro restimulation with Gp120, Gag and RT peptides at 7 days post-boost (Day 35) using IFN-gamma ELIspot

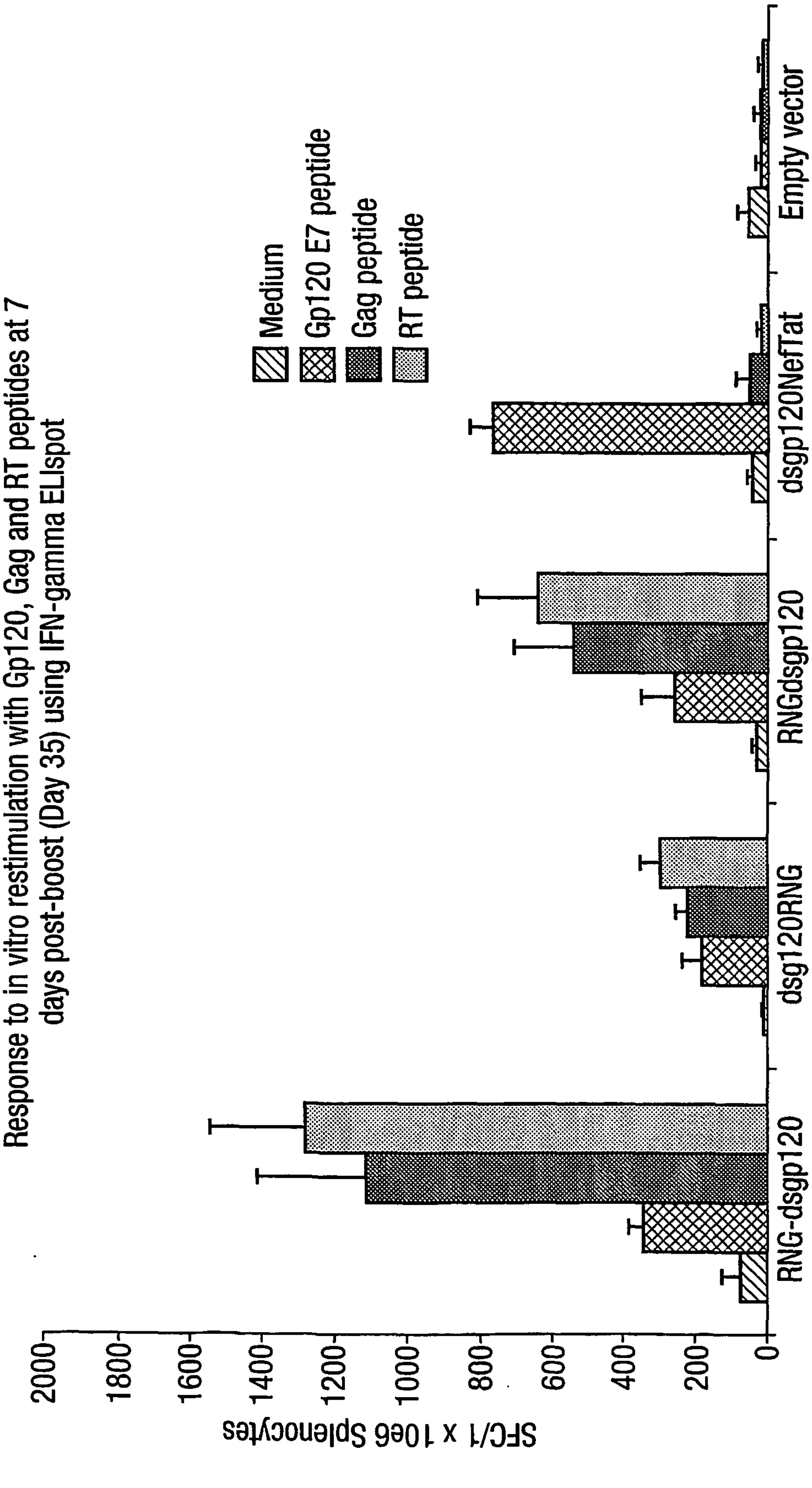

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03025003 A
- WO 0236792 A
- US 4436727 A
- US 4877611 A
- US 4866034 A
- US 4912094 A
- WO 9602555 A
- WO 9933488 A
- US 6008200 A
- US 5856462 A
- US 6083716 A
- WO 03046124 A
- WO 03046142 A
- WO 9107487 A
- US 5846796 A
- US 6010478 A
- US 5865796 A
- US 5584807 A
- EP 0500799 A
- US 4790824 A
- US 5064413 A
- US 5312335 A
- US 5383851 A
- US 5399163 A
- US 5520639 A
- US 5993412 A
- US 5697901 A
- WO 0023592 A


### Non-patent literature cited in the description


- **Green et al.** *New England J. Med,* 1991, vol. 324 (5), 308
- **Bryant et al.** Pediatr. Infect. Dis. J. 1992, vol. 11, 390
- **C. David Pauza.** Immunization with Tat toxoid attenuates SHIV89.6PD infection in rhesus macaques. *12th Cent Gardes meeting,* 26 October 1999
- **Fields BN ; Knipe DM ; Howley M.** *Fundamental Virology,* 1996
- *Fields Virology,* 1996, vol. 2
- **Skalka AM.** *99 Adv in Virus Res,* vol. 52, 271-273
- **Donnelly et al.** DNA vaccines. *Ann. Rev Immunol.,* 1997, vol. 15, 617-648
- **Doe et al.** *Eur J immunol,* 1994, vol. 24, 2369-2376
- **Bresnahan P.A. et al.** *Current Biology,* 1998, vol. 8 (22), 1235-8
- **Harrison et al.** Reduction of recurrent HSV disease using imiquimod alone or combined with a glycoprotein vaccine. *Vaccine,* 2001, vol. 19, 1820-1826
- **Vasilakos et al.** Adjuvant activites of immune response modifier R-848: Comparison with CpG ODN. *Cellular immunology,* 2000, vol. 204, 64-74
- **Rhodes, J. et al.** Therapeutic potentiation of the immune system by costimulatory Schiff-base-forming drugs. *Nature,* 1995, vol. 377, 71-75
- **Reyes et al.** Vaxfectin enhances antigen specific antibody titres and maintains Th1 type immune responses to plasmid DNA immunization. *Vaccine,* vol. 19, 3778-3786
- **Beutler, B.** Endotoxin, 'Toll-like receptor 4, and the afferent limb of innate immunity. *Current Opinion in Microbiology,* 2000, vol. 3, 23-30
- **Sato, Y. et al.** Immunostimulatory DNA sequences necessary for effective intradermal gene immunization. *Science,* 1996, vol. 273 (5273), 352-354
- **Hemmi, H. et al.** A Toll-like receptor recognizes bacterial DNA. *Nature,* 2000, vol. 408, 740-745
- **Sato et al.** *Science,* 1996, vol. 273, 352
- **Nakamura.** *Nucleic Acids Research,* 1996, vol. 24, 214-215
- **Sambrook et al.** Molecular Cloning: a Laboratory Manual. CSH Laboratory Press, 1989
- **Verme et al.** *Nature,* 1997, vol. 389, 239-242
- **Haynes et al.** *J Biotechnology,* 1996, vol. 44, 37-42
- Remington's Pharmaceutical Sciences. Mack Publishing Company, 1985
- **Ertl, PF. ; Thomsen, LL.** Technical issues in construction of nucleic acid vaccines. *Methods,* 2003, vol. 31 (3), 199-206
- *Cell,* 1985, vol. 40, 9-17
- *Science,* 1985, vol. 229, 69-73
- *Virology,* 1997, vol. 235, 48-64
- **Moriarty et al.** *Proc.Natl.Acad.Sci. USA,* 1981, vol. 78, 2606-10

- **B.Maschera ; E Furfine ; E.D. Blair.** *J.Virol,* 1995, vol. 69, 5431-5436
- **Andre S. ; Seed B. ; Eberle J. ; Schraut W. ; Bultmann A. ; Haas J.** Increased immune response elicited by DNA vaccination with a synthetic gp120 sequence with optimized codon usage. *Journal of Virology,* 1998, vol. 72 (2), 1497-503
- **Vinner L. ; Nielsen HV ; Bryder K. ; Corbet S. ; Nielsen C. ; Fomsgaard A.** Gene gun DNA vaccination with Rev-independent synthetic HIV-1 gp160 envelope gene using mammalian codons. *Vaccine,* 1999, vol. 17 (17), 2166-75
- **Collins KL ; Baltimore D.** HIV's evasion of the cellular immune response. *Immunological Reviews,* 1999, vol. 168, 65-74
- **Eisenbraun et al.** *DNA and Cell Biology,* 1993, vol. 12 (9), 791-797